# EUROPEAN PATENT APPLICATION

(11) **EP 1 876 186 A1**
(43) Date of publication of application: **09.01.2008**
(21) Application number: 06715709.9
(22) Date of filing: 14.03.2006
(51) Int. Cl.: C07K 16/32, A61K 39/395, A61P 35/00, A61P 43/00, C12N 15/09, C12P 21/08

(54) **PROPHYLACTIC/THERAPEUTIC AGENT FOR CANCER**

(30) Priority: 15.03.2005 JP 2005074065
(71) Applicant: Takeda Pharmaceutical Company Limited, Osaka-shi, Osaka 541-0045 (JP)
(72) Inventor: SATOU, Shuji c/o Takeda Pharmaceutical Co. Ltd., Osaka-shi, Osaka 5328686 (JP); ISHII, Takafumi c/o Takeda Pharmaceutical Co. Ltd., Osaka-shi, Osaka 5328686 (JP)
(74) Representative: Helbing, Jörg
(86) International application number: PCT/JP2006/305481
(87) International publication number: WO 2006/098465

(57) **Abstract**

The present invention provides a safe drug that targets a molecule specifically expressed in cancer cells, and that induces cancer cell growth inhibition. More specifically, the present invention provides a neutralizing antibody against a protein comprising the same or substantially the same amino acid sequence as that shown by SEQ ID NO:1, SEQ ID NO:3, SEQ ID NO:5, SEQ ID NO:7 or SEQ ID NO:9, or a partial peptide thereof or a salt thereof, and a prophylactic/therapeutic agent for cancers (for example, breast cancer, ovarian cancer, colorectal cancer, lung cancer, pancreatic cancer) and the like, apoptosis promoter, cancer cell growth suppressant, DDR1 antagonist and the like, comprising the neutralizing antibody.

## Description

### Field of the Invention

The present invention relates to an antibody that neutralizes an activity of DDR1 (Discoidin Domain Receptor 1), an apoptosis inducer or cancer prophylactic/therapeutic agent comprising the antibody, screening for an apoptosis inducer or prophylactic/therapeutic agent for cancer and the like.

### Background of Invention

In cancer, it is anticipated that the pathologic condition can be evaluated by gene microarray profiling data; in fact, it has been reported that leukemia can be classified by gene expression profile. It is considered that by clarifying the gene expression profiles of individual cancer tissues, and compiling data on the classification thereof, it is possible to predict their responsiveness to a particular cancer therapeutic method, and to discover a novel drug discovery target protein for a particular cancer. Specifically, if an upregulated expression of a certain protein is observed in a certain cancer, it is possible to induce anti-tumor activity in a patient newly diagnosed as being antigen-positive by a method such as (i) reducing the expression level thereof, (ii) suppressing the function of the protein, or (iii) causing the host's immune responses to the protein. At the same time, it is expected that for a patient diagnosed as being antigen-negative, there will be no concern of posing an unwanted burden on the patient because of quick switchability to another therapy and the like. Hence, expression profile analysis is expected to possibly make major contributions to the molecular diagnosis of cancer and the development of molecule-targeted therapeutic drugs for cancer.

The DDR1a gene (RefSeq Accession No. NM_001954) is a gene cloned from a human keratinocyte-derived cDNA and a human fetal brain-derived cDNA, encoding a protein consisting of 876 amino acids (RefSeq Accession No. NP_001945). The DDR1b gene (GenBank Accession No. L11315) is a gene cloned from a human placenta-derived cDNA, encoding a protein consisting of 913 amino acids (GenBank Accession No. AAA02866). The DDR1c gene (RefSeq Accession No. NM_013994) is a gene cloned from a human fetal brain-derived cDNA, encoding a protein consisting of 919 amino acids (RefSeq Accession No. NP_054700). The DDR1d gene and the DDR1e gene are known to be expressed in cancer cell lines (FASEB J. (2001) 15(7), p1321-p1323) (hereinafter DDR1a, DDR1b, DDR1c, DDR1d, and DDR1e are also generically referred to as DDR1). Furthermore, a mouse gene homologous to the DDR1b gene (RefSeq Accession No. NM_007584) has been cloned, which encodes a protein consisting of 911 amino acids (RefSeq Accession No. NP_031610). This mouse gene has a homology of about 86% in terms of base sequence and a homology of about 93% in terms of amino acid sequence to the DDR1b gene. A rat gene homologous to the DDR1b gene (RefSeq Accession No. NM_013137) has also been cloned, which encodes a protein consisting of 910 amino acids (RefSeq Accession No. NP_037269). This rat gene has a homology of about 86% in terms of base sequence and a homology of about 93% in terms of amino acid sequence to the DDR1b gene. The DDR1 gene is a gene having synonyms such as MCK10, Cak, NEP, trkE, PTK3, RTK6, and CD167, belonging to the DDR family; the DDR family consists of DDR1 and DDR2 (hereinafter these are also generically referred to as DDR). The DDR1b gene is a splicing variant of the DDR1a gene; the protein encoded by the DDR1b gene has 37 amino acids added between the 505th and 506th amino acids of the protein encoded by the DDR1a gene. LLSNPAY, a sequence included in the 37 amino acids, is known to be a sequence that binds to the PTB domain of Shc (Official Gazette for US2003070184). The protein encoded by the DDR1c gene has a sequence wherein 37 amino acids and 6 amino acids are added between the 505th and 506th amino acids and between the 665th and 666th amino acids of the protein encoded by the DDR1a gene, respectively. The DDR1d gene encodes a protein consisting of 508 amino acids, wherein the sequence from the 505th to C-terminal amino acids of the protein encoded by the DDR1a gene is substituted by another amino acid sequence consisting of 4 amino acids. The DDR1e gene encodes a protein consisting of 767 amino acids, wherein the sequence consisting of 137 amino acids corresponding to the 450th to 586th amino acids in the protein encoded by the DDR1a gene is substituted by another amino acid sequence consisting of 28 amino acids.

DDR1 is a receptor whose ligand is collagen; it is known that upon binding of collagen to the extracellular region of DDR1, DDR1 is activated; increase in the kinase activity catalyzed by the intracellular region induces auto-phosphorylation (Mol. Cell (1997) 1(1), p13-p23). The DDR1b gene is a gene whose expression is induced by the cancer suppressor gene p53; it is considered that cancer cells receive growth promotion signals from DDR1b, such as enhancement of the phosphorylation of MAPK (ERK1/2) and enhancement of the phosphorylation of Akt by the ligand collagen (EMBO J. (2003) 22(6), p1289-p1301). Furthermore, it is also known that apoptosis of cancer cells is induced by inactivated type DDR1b deprived of the kinase domain present in DDR1b (EMBO J. (2003) 22(6), p1289-p1301). The mRNA of the DDR1 gene is known to be expressed in ovarian cancer cells (Cell Growth Differ. (1994) 5(11), p1173-p1183), and is also known to be expressed in various other cancer cells (Official Gazette for US5677144 and Official Gazette for WO 03/085125); it has been reported that the DDR1 gene is one of the genes useful for the diagnosis and treatment of breast cancer and lung cancer (Official Gazette for US2003124133), one of the genes useful for the diagnosis and treatment of colorectal cancer (Official Gazette for WO 01/22920), one of the genes useful for the diagnosis and treatment of pancreatic cancer (Official Gazette for WO 00/55320), and one of the genes useful for the diagnosis of ovarian cancer (Official Gazette for WO 04/22778).

### Disclosure of the Invention

There is a strong demand for a safe drug that targets a molecule specifically expressed in cancer cells, and that induces cancer cell growth inhibition.

The present inventors conducted diligent investigations to solve the above-described problems and, as a result, took note of the fact that the DDR1 gene exhibited remarkably upregulated expression in cancer tissues such as breast cancer, ovarian cancer, colorectal cancer, lung cancer and pancreatic cancer. The present inventors also found that because the DDR1 gene product is a cytoplasmic membrane protein and is suitable as an antibody target, a DDR1-neutralizing antibody having an activity to suppress the DDR1 protein function was useful as therapeutic agent for cancers such as breast cancer, ovarian cancer, colorectal cancer, lung cancer and pancreatic cancer and the like. The present inventors conducted further investigations based on this finding, succeeded in preparing a DDR1-neutralizing antibody, and thus developed the present invention.

Accordingly, the present invention provides:
(1) A neutralizing antibody against a protein comprising the same or substantially the same amino acid sequence as that shown by SEQ ID NO:1, SEQ ID NO:3, SEQ ID NO:5, SEQ ID NO:7 or SEQ ID NO:9, or a partial peptide thereof or a salt thereof,
(2) the neutralizing antibody described in (1) above, which neutralizes the apoptosis-inhibitory activity resulting from the collagen binding to a protein comprising the same or substantially the same amino acid sequence as that shown by SEQ ID NO:1, SEQ ID NO:3, SEQ ID NO:5, SEQ ID NO:7 or SEQ ID NO:9, or a partial peptide thereof or a salt thereof,
(3) the neutralizing antibody described in (1) above, which neutralizes the cancer cell growth stimulation resulting from the collagen binding to a protein comprising the same or substantially the same amino acid sequence as that shown by SEQ ID NO:1, SEQ ID NO:3, SEQ ID NO:5, SEQ ID NO:7 or SEQ ID NO:9, or a partial peptide thereof or a salt thereof,
(4) the neutralizing antibody described in (2) or (3) above, wherein the collagen is type IV collagen,
(5) the neutralizing antibody described in (1) above, wherein the neutralizing antibody is an antibody against the polypeptide which amino acid sequence is from the 22nd to the 416th of that shown by SEQ ID NO:3 or a partial peptide thereof or a salt thereof,
(6) the neutralizing antibody described in (1), prepared by the DNA immunization method,
(7) the neutralizing antibody described in (1), wherein the neutralizing antibody is a polyclonal antibody,
(8) the neutralizing antibody described in (1), wherein the neutralizing antibody is a monoclonal antibody,
(9) the neutralizing antibody described in (1), wherein the neutralizing antibody is a humanized antibody,
(10) the neutralizing antibody described in (1), wherein the neutralizing antibody is a human antibody,
(11) a medicament comprising a neutralizing antibody against a protein comprising the same or substantially the same amino acid sequence as that shown by SEQ ID NO:1, SEQ ID NO:3, SEQ ID NO:5, SEQ ID NO:7 or SEQ ID NO:9, or a partial peptide thereof or a salt thereof,
(12) an antagonist for a protein comprising the same or substantially the same amino acid sequence as that shown by SEQ ID NO:1, SEQ ID NO:3, SEQ ID NO:5, SEQ ID NO:7 or SEQ ID NO:9, or a partial peptide thereof or a salt thereof, which comprises the neutralizing antibody against a protein comprising the same or substantially the same amino acid sequence as that shown by SEQ ID NO:1, SEQ ID NO:3, SEQ ID NO:5, SEQ ID NO:7 or SEQ ID NO:9, or a partial peptide thereof or a salt thereof,
(13) an apoptosis inducer comprising a neutralizing antibody against a protein comprising the same or substantially the same amino acid sequence as that shown by SEQ ID NO:1, SEQ ID NO:3, SEQ ID NO:5, SEQ ID NO:7 or SEQ ID NO:9, or a partial peptide thereof or a salt thereof,
(14) a cancer cell growth suppressant comprising a neutralizing antibody against a protein comprising the same or substantially the same amino acid sequence as that shown by SEQ ID NO:1, SEQ ID NO:3, SEQ ID NO:5, SEQ ID NO:7 or SEQ ID NO:9, or a partial peptide thereof or a salt thereof,
(15) a cancer prophylactic/therapeutic agent comprising a neutralizing antibody against a protein comprising the same or substantially the same amino acid sequence as that shown by SEQ ID NO:1, SEQ ID NO:3, SEQ ID NO:5, SEQ ID NO:7 or SEQ ID NO:9, or a partial peptide thereof or a salt thereof,
(16) the agent described in (15), wherein the cancer is breast cancer, ovarian cancer, colorectal cancer, lung cancer or pancreatic cancer,
(17) a cancer prophylactic/therapeutic method comprising administering, to a mammal, an effective amount of a neutralizing antibody against a protein comprising the same or substantially the same amino acid sequence as that shown by SEQ ID NO:1, SEQ ID NO:3, SEQ ID NO:5, SEQ ID NO:7 or SEQ ID NO:9, or a partial peptide thereof or a salt thereof,
(18) a use of a neutralizing antibody against a protein comprising the same or substantially the same amino acid sequence as that shown by SEQ ID NO:1, SEQ ID NO:3, SEQ ID NO:5, SEQ ID NO:7 or SEQ ID NO:9, or a partial peptide thereof or a salt thereof, for producing a cancer prophylactic/therapeutic agent.

The neutralizing antibody of the present invention is useful for the induction of apoptosis, the prevention/treatment of a specified cancer (for example, breast cancer, ovarian cancer, colorectal cancer, lung cancer and pancreatic cancer) and the like.

### Brief Description of the Drawings

Figure 1 shows the effect of type IV collagen on apoptosis induced by serum removal.
Figure 2 shows the inhibition of collagen-induced cell protecting action by the αDDR1b rabbit polyclonal antibody on DDR1FL-#117 and DDR1bDN-#206, which are cell lines wherein DDR1b is forcibly expressed.
Figure 3 shows the inhibition of collagen-induced cell protecting action by the αDDR1b rabbit polyclonal antibody on the cancer cell line HCT116.

### Best Mode for Embodying the Invention

A protein comprising the same or substantially the same amino acid sequence as that shown by SEQ ID NO:1, SEQ ID NO:3, SEQ ID NO:5, SEQ ID NO:7 or SEQ ID NO:9 (hereinafter also referred to as the receptor used in the present invention) may be a protein derived from a cell (e.g., hepatocyte, splenocyte, nerve cell, glial cell, pancreatic β cell, myelocyte, mesangial cell, Langerhans' cell, epidermal cell, epithelial cell, goblet cell, endothelial cell, smooth muscle cell, fibroblast, fibrocyte, myocyte, adipocyte, immune cell (e.g., macrophage, T cell, B cell, natural killer cell, mast cell, neutrophil, basophil, eosinophil, monocyte), megakaryocyte, synovial cell, chondrocyte, bone cell, osteoblast, osteoclast, mammary gland cell, hepatocyte or interstitial cell, or a corresponding precursor cell, stem cell or cancer cell thereof, and the like) of a human or warm-blooded animal (for example, guinea pigs, rats, mice, chicken, rabbits, pigs, sheep, cattle, monkeys and the like) or any tissue in which these cells are present, for example, brain or any portion of brain (e.g., olfactory bulb, amygdaloid nucleus, basal ganglia, hippocampus, thalamus, hypothalamus, cerebral cortex, medulla oblongata, cerebellum), spinal cord, hypophysis, stomach, pancreas, kidney, liver, gonad, thyroid, gallbladder, bone marrow, adrenal gland, skin, muscle, lung, gastrointestinal tract (e.g., large intestine and small intestine), blood vessel, heart, thymus, spleen, submandibular gland, peripheral blood, prostate, testicle, ovary, placenta, uterus, bone, joint, skeletal muscle, and the like, and may be a synthetic protein.

As substantially the same amino acid sequence as that shown by SEQ ID NO:1, an amino acid sequence having a homology of about 50% or more, preferably about 60% or more, preferably about 70% or more, preferably about 80% or more, preferably about 90% or more, preferably about 95% or more, to the amino acid sequence shown by SEQ ID NO:1 and the like can be mentioned.

As the protein comprising substantially the same amino acid sequence as that shown by SEQ ID NO:1, for example, a protein comprising the above-mentioned substantially the same amino acid sequence as that shown by SEQ ID NO:1, and having substantially the same quality of activity as a protein comprising the amino acid sequence shown by SEQ ID NO:1 and the like are preferable.

As substantially the same amino acid sequence as that shown by SEQ ID NO:3, an amino acid sequence having a homology of about 50% or more, preferably about 60% or more, preferably about 70% or more, preferably about 80% or more, preferably about 90% or more, preferably about 95% or more, to the amino acid sequence shown by SEQ ID NO:3 and the like can be mentioned.

As the protein comprising substantially the same amino acid sequence as that shown by SEQ ID NO:3, for example, a protein comprising the above-mentioned substantially the same amino acid sequence as that shown by SEQ ID NO: 3, and having substantially the same quality of activity as a protein comprising the amino acid sequence shown by SEQ ID NO:3 and the like are preferable.

As substantially the same amino acid sequence as that shown by SEQ ID NO:5, an amino acid sequence having a homology of about 50% or more, preferably about 60% or more, preferably about 70% or more, preferably about 80% or more, preferably about 90% or more, preferably about 95% or more, to the amino acid sequence shown by SEQ ID NO:5 and the like can be mentioned.

As the protein comprising substantially the same amino acid sequence as that shown by SEQ ID NO:5, for example, a protein comprising the above-mentioned substantially the same amino acid sequence as that shown by SEQ ID NO: 5, and having substantially the same quality of activity as a protein comprising the amino acid sequence shown by SEQ ID NO:5 and the like are preferable.

As substantially the same amino acid sequence as that shown by SEQ ID NO:7, an amino acid sequence having a homology of about 50% or more, preferably about 60% or more, preferably about 70% or more, preferably about 80% or more, preferably about 90% or more, preferably about 95% or more, to the amino acid sequence shown by SEQ ID NO:7 and the like can be mentioned.

As the protein comprising substantially the same amino acid sequence as that shown by SEQ ID NO:7, for example, a protein comprising the above-mentioned substantially the same amino acid sequence as that shown by SEQ ID NO: 7, and having substantially the same quality of activity as a protein comprising the amino acid sequence shown by SEQ ID NO:7 and the like are preferable.

As substantially the same amino acid sequence as that shown by SEQ ID NO:9, an amino acid sequence having a homology of about 50% or more, preferably about 60% or more, preferably about 70% or more, preferably about 80% or more, preferably about 90% or more, preferably about 95% or more, to the amino acid sequence shown by SEQ ID NO:9 and the like can be mentioned.

As the protein comprising substantially the same amino acid sequence as that shown by SEQ ID NO:9, for example, a protein comprising the above-mentioned substantially the same amino acid sequence as that shown by SEQ ID NO: 9, and having substantially the same quality of activity as a protein comprising the amino acid sequence shown by SEQ ID NO:9 and the like are preferable.

The homology of amino acid sequence can be calculated using the homology calculation algorithm NCBI BLAST (National Center for Biotechnology Information Basic Local Alignment Search Tool) under the following conditions (expectancy = 10; allowing gap; matrix=BLOSUM62;filtering=OFF).

As examples of substantially the same quality of activity described above, ligand-binding activities such as for collagens (for example, type I collagen, type II collagen, type III collagen, type IV collagen, type V collagen, type VI collagen, type VIII collagen), activities to undergo phosphorylation (e.g., activity to undergo phosphorylation by ligand stimulation and the like) and the like can be mentioned. Substantially the same quality means that the activities are qualitatively (e.g., physiologically or pharmacologically) equivalent to each other. Therefore, it is preferable that the above-described activities be equivalent to each other (e.g., about 0.01 to 100 times, preferably about 0.1 to 10 times, more preferably 0.5 to 2 times), but the quantitative factors of these activities, such as the extent of activity and the molecular weight of the protein, may be different.

A measurement of the above-described binding activity can be performed by a method known per se, for example, EIA, immunoprecipitation or a method based thereon. Specifically, for example, each of a ligand such as a collagen and the receptor used in the present invention is expressed as a tagged recombinant type protein in animal cells. As the tag, FLAG, His, V5, myc, HA and the like are used; the tag added to the ligand (tag A) and the tag added to the receptor used in the present invention (tag B) should be different. With an antibody against tag B, a mixture of the above-described ligand with tag A and the above-described receptor with tag B is immunoprecipitated, and the precipitate obtained is subjected to a Western blotting procedure using an antibody against tag A, whereby the amount of ligand bound to the receptor used in the present invention can be measured.

An activity to undergo phosphorylation is measured in accordance with a method known per se, for example, the method described in Methods in Enzymology Vol.200, pages 98 to 107, 1991, or a method based thereon. Specifically, for example, the receptor used in the present invention, having a tag (e.g., FLAG, His, V5, myc, HA and the like) added to the C terminus thereof, is expressed as a recombinant type protein in animal cells and reacted with a ligand such as a collagen, after which the cells are disrupted to prepare a cell-free extract, and the extract is immunoprecipitated using an anti-tag antibody. The amount of phosphorylated receptor used in the present invention produced can be quantified by a commonly known method (e.g., Western blot method and the like) using an anti-phosphorylated tyrosine antibody and the like.

Examples of the receptor used in the present invention also include what are called muteins of proteins comprising (i) an amino acid sequence having 1 or 2 or more (for example, about 1 to 50, preferably about 1 to 30, more preferably about 1 to 10, still more preferably several (1 to 5)) amino acids deleted from the amino acid sequence shown by SEQ ID NO:1, SEQ ID NO:3, SEQ ID NO:5, SEQ ID NO:7 or SEQ ID NO:9, (ii) an amino acid sequence having 1 or 2 or more (for example, about 1 to 50, preferably about 1 to 30, more preferably about 1 to 10, still more preferably several (1 to 5)) amino acids added to the amino acid sequence shown by SEQ ID NO:1, SEQ ID NO:3, SEQ ID NO:5, SEQ ID NO:7 or SEQ ID NO:9, (iii) an amino acid sequence having 1 or 2 or more (for example, about 1 to 50, preferably about 1 to 30, more preferably about 1 to 10, still more preferably several (1 to 5) amino acids)) inserted in the amino acid sequence shown by SEQ ID NO:1, SEQ ID NO:3, SEQ ID NO:5, SEQ ID NO:7 or SEQ ID NO:9, (iv) an amino acid sequence having 1 or 2 or more (for example, about 1 to 50, preferably about 1 to 30, more preferably about 1 to 10, still more preferably several (1 to 5)) amino acids substituted by other amino acids in the amino acid sequence shown by SEQ ID NO:1, SEQ ID NO:3, SEQ ID NO:5, SEQ ID NO:7 or SEQ ID NO:9, or (v) an amino acid sequence comprising a combination thereof.

When an amino acid sequence is inserted, deleted or substituted as described above, the position of the insertion, deletion or substitution is not subject to limitation.

As specific examples of the receptor used in the present invention, for example, a protein comprising the amino acid sequence shown by SEQ ID NO:1, a protein comprising the amino acid sequence shown by SEQ ID NO:3, a protein comprising the amino acid sequence shown by SEQ ID NO:5, a protein comprising the amino acid sequence shown by SEQ ID NO:7, a protein comprising the amino acid sequence shown by SEQ ID NO:9 and the like can be mentioned.

For the proteins mentioned herein, the left end indicates the N-terminus (amino terminus) and the right end indicates the C-terminus (carboxyl terminus), according to the common practice of peptide designation. For the receptor used in the present invention, the C-terminus may be any of a carboxyl group (-COOH), a carboxylate (-COO⁻), an amide (-CONH₂) or an ester (-COOR).

Here, as R in the ester, a C₁₋₆ alkyl group such as methyl, ethyl, n-propyl, isopropyl and n-butyl, a C₃₋₈ cycloalkyl group such as cyclopentyl and cyclohexyl, a C₆₋₁₂ aryl group such as phenyl and α-naphthyl, a phenyl-C₁₋₂ alkyl group such as benzyl and phenethyl, a C₇₋₁₄ aralkyl group such as an α-naphthyl-C₁₋₂ alkyl group such as α-naphthylmethyl, a pivaloyloxymethyl group; and the like can be used.

When the receptor used in the present invention has a carboxyl group (or a carboxylate) in addition to that on the C-terminal, one in which the carboxyl group is amidated or esterified is also included in the receptor used in the present invention. In this case, as the ester, the above-described C-terminal ester and the like, for example, can be used.

Furthermore, the receptor used in the present invention also includes a protein wherein the amino group of the N-terminal amino acid residue thereof (e.g., methionine residue) is protected by a protecting group (for example, a C₁₋₆ acyl group such as C₁₋₆ alkanoyl such as a formyl group or an acetyl group, and the like), a protein wherein the N-terminal glutamine residue, which is produced by cleavage in vivo, has been converted to pyroglutamic acid, a protein wherein a substituent (for example, -OH, -SH, an amino group, an imidazole group, an indole group, a guanidino group and the like) on an amino acid side chain in the molecule is protected by an appropriate protecting group (for example, a C₁₋₆ acyl group such as a C₁₋₆ alkanoyl group such as a formyl group or an acetyl group, and the like), a conjugated protein such as what is called a glycoprotein, which has a sugar chain bound thereto, and the like.

As the partial peptide of the receptor used in the present invention (the partial peptide used in the present invention), any partial peptide of the foregoing receptor used in the present invention, preferably having the same property as that of the foregoing receptor used in the present invention, can be used.

For example, a peptide having at least 20 or more, preferably 50 or more, more preferably 70 or more, still more preferably 100 or more, most preferably 200 or more, amino acids of the constituent amino acids of the sequence of the receptor used in the present invention and the like are used.

As specific examples of the partial peptide used in the present invention, the polypeptide consisting of the 22nd to 416th amino acids of the amino acid sequence shown by SEQ ID NO:1, SEQ ID NO:3, SEQ ID NO:5, SEQ ID NO:7 or SEQ ID NO:9 or a partial peptide thereof can be mentioned; in particular, the polypeptide consisting of the 22nd to 416th amino acids of the amino acid sequence shown by SEQ ID NO:3 or a partial peptide thereof is preferably used.

The above-described partial peptide may have 1 or 2 or more (preferably about 1 to 20, more preferably about 1 to 10, still more preferably several (1 to 5)) amino acids deleted from the amino acid sequence thereof, or 1 or 2 or more (preferably about 1 to 20, more preferably about 1 to 10, still more preferably several (1 to 5)) amino acids added to the amino acid sequence thereof, or 1 or 2 or more (preferably about 1 to 20, more preferably about 1 to 10, still more preferably several (1 to 5)) amino acids inserted in the amino acid sequence thereof, or 1 or 2 or more (preferably about 1 to 20, more preferably about 1 to 10, still more preferably several, still yet more preferably about 1 to 5) amino acids substituted by other amino acids in the amino acid sequence thereof.

For the partial peptide used in the present invention, the C-terminus may be any of a carboxyl group (-COOH), a carboxylate (-COO⁻), an amide (-CONH₂) or an ester (-COOR).

Furthermore, the partial peptide used in the present invention, like the foregoing receptor used in the present invention, also includes a partial peptide wherein a carboxyl group (or carboxylate) is present at a position other than the C-terminus, a partial peptide wherein the amino group of the N terminal amino acid residue (e.g., methionine residue) is protected by a protecting group, a partial peptide wherein glutamine residue, which is produced upon cleavage at the N terminal in vivo, has been converted to pyroglutamic acid, a partial peptide wherein a substituent on a side chain of an amino acid in the molecule is protected by an appropriate protecting group, a conjugated peptide such as what is called a glycopeptide having a sugar chain bound thereto, and the like.

The partial peptide used in the present invention can also be used as an antigen for generating an antibody.

As salts of the receptor or partial peptide used in the present invention, physiologically acceptable salts with acids (e.g., inorganic acid, organic acid) or bases (e.g., alkali metal salts) and the like can be used, and physiologically acceptable acid addition salts are preferred. Useful salts include, for example, salts with inorganic acids (e.g., hydrochloric acid, phosphoric acid, hydrobromic acid, sulfuric acid) or salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid) and the like.

The receptor used in the present invention or a partial peptide thereof or a salt thereof can be produced from the above-described cells or tissues of humans or other warm-blooded animals by a method of protein purification known per se, and can also be produced by culturing a transformant comprising a DNA that encodes the protein. The receptor used in the present invention or a partial peptide thereof or a salt thereof can also be produced in accordance with the method of peptide synthesis described below.

When the receptor used in the present invention or a partial peptide thereof or a salt thereof is produced from a tissue or cells of a human or another mammal, it can be purified and isolated by homogenizing the tissue or cells of the human or mammal, then performing extraction with acid and the like, and subjecting the extract to a combination of chromatographies such as reversed phase chromatography and ion exchange chromatography.

For the synthesis of the receptor used in the present invention or a partial peptide or a salt thereof, or an amide thereof, an ordinary commercially available resin for protein synthesis can be used. As examples of such resins, chloromethyl resin, hydroxymethyl resin, benzhydrylamine resin, aminomethyl resin, 4-benzyloxybenzyl alcohol resin, 4-methylbenzhydrylamine resin, PAM resin, 4-hydroxymethylmethylphenylacetamidomethyl resin, polyacrylamide resin, 4-(2',4'-dimethoxyphenyl-hydroxymethyl) phenoxy resin, 4-(2',4'-dimethoxyphenyl-Fmoc-aminoethyl)phenoxy resin and the like can be mentioned. Using such a resin, an amino acid having an appropriately protected α-amino group and side chain functional group is condensed on the resin in accordance with the sequence of the desired protein according to one of various methods of condensation known per se. At the end of the reaction, the protein or partial peptide is cleaved from the resin, at the same time various protecting groups are removed, and a reaction to form an intramolecular disulfide bond is carried out in a highly diluted solution to obtain the desired protein or partial peptide or an amide thereof.

For the above-described condensation of protected amino acids, various activation reagents useful for protein synthesis can be used, with preference given to a carbodiimide. As the carbodiimide, DCC, N,N'-diisopropylcarbodiimide, N-ethyl-N'-(3-dimethylaminoprolyl)carbodiimide and the like can be used. For the activation using these carbodiimides, the protected amino acid, along with a racemization-suppressing additive (for example, HOBt, HOOBt), may be added directly to the resin, or the protected amino acid may be activated in advance as a symmetric acid anhydride, or HOBt ester or HOOBt ester and then added to the resin.

A solvent used for activation of protected amino acids and condensation of protected amino acids with a resin can be appropriately selected from among solvents that are known to be usable for protein condensation reactions. Examples of such useful solvents include acid amides such as N,N-dimethylformamide, N,N-dimethylacetamide, and N-methylpyrrolidone; halogenated hydrocarbons such as methylene chloride and chloroform; alcohols such as trifluoroethanol; sulfoxides such as dimethyl sulfoxide; pyridine; ethers such as dioxane and tetrahydrofuran; nitriles such as acetonitrile and propionitrile; esters such as methyl acetate and ethyl acetate; suitable mixtures thereof; and the like. Reaction temperature is appropriately selected from the range that is known to be usable in protein binding reactions, and is normally from the range of about -20°C to about 50°C. An activated amino acid derivative is normally used from 1.5 to 4 times in excess. When the condensation is insufficient as the result of the test using a ninhydrin reaction, sufficient condensation can be carried out by repeating the condensation reaction without elimination of the protecting group. If the condensation is insufficient even though the reaction is repeated, unreacted amino acids may be acetylated using acetic anhydride or acetylimidazole to prevent the subsequent reaction from being influenced.

As the protecting group for the amino group of the starting material, Z, Boc, t-pentyloxycarbonyl, isobornyloxycarbonyl, 4-methoxybenzyloxycarbonyl, C1-Z, Br-Z, adamantyloxycarbonyl, trifluoroacetyl, phthaloyl, formyl, 2-nitrophenylsulfenyl, diphenylphosphinothioyl, Fmoc and the like, for example, can be used.

The carboxyl group can be protected by, for example, alkyl esterification (for example, linear, branched or cyclic alkyl esterification with methyl, ethyl, propyl, butyl, t-butyl, cyclopentyl, cyclohexyl, cycloheptyl, cyclooctyl, 2-adamantyl, and the like), aralkyl esterification (for example, benzyl esterification, 4-nitrobenzyl esterification, 4-methoxybenzyl esterification, 4-chlorobenzyl esterification, benzhydryl esterification), phenacyl esterification, benzyloxycarbonyl hydrazidation, t-butoxycarbonyl hydrazidation, trityl hydrazidation, and the like.

The hydroxyl group of serine can be protected by, for example, esterification or etherification. As the group suitable for this esterification, lower (C₁₋₆) alkanoyl groups such as an acetyl group, aroyl groups such as a benzoyl group, and groups derived from carbonic acid such as benzyloxycarbonyl group, ethoxycarbonyl group and the like, for example, can be used. In addition, as examples of the group suitable for etherification, a benzyl group, a tetrahydropyranyl group, a t-butyl group and the like can be mentioned.

As the protecting group for the phenolic hydroxyl group of tyrosine, Bzl, Cl₂-Bzl, 2-nitrobenzyl, Br-Z, t-butyl and the like, for example, can be used.

As the protecting group for the imidazole of histidine, Tos, 4-methoxy-2,3,6-trimethylbenzenesulfonyl, DNP, benzyloxymethyl, Bum, Boc, Trt, Fmoc and the like, for example, can be used.

As examples of the activated carboxyl group in the starting material, corresponding acid anhydrides, azides, activated esters [esters with alcohols (for example, pentachlorophenol, 2,4,5-trichlorophenol, 2,4-dinitrophenol, cyanomethyl alcohol, para-nitrophenol, HONB, N-hydroxysuccimide, N-hydroxyphthalimide, HOBt)] and the like are used. As examples of the activated amino group in the starting material, corresponding phosphoric amides are used.

As the method of removing (eliminating) a protecting group, catalytic reduction in a hydrogen stream in the presence of a catalyst such as Pd-black or Pd-carbon; acid treatment by means of anhydrous hydrogen fluoride, methanesulfonic acid, trifluoromethane-sulfonic acid, trifluoroacetic acid, or a mixture solution thereof; base treatment by means of diisopropylethylamine, triethylamine, piperidine, piperazine or the like; and reduction with sodium in liquid ammonia, and the like, for example, can be used. The elimination reaction by the above-described acid treatment is generally carried out at a temperature of about -20°C to about 40°C; the acid treatment is efficiently conducted by adding a cation scavenger such as anisole, phenol, thioanisole, m-cresol, p-cresol, dimethyl sulfide, 1,4-butanedithiol or 1,2-ethanedithiol, for example. Also, a 2,4-dinitrophenyl group used as a protecting group for the imidazole of histidine is removed by thiophenol treatment; a formyl group used as a protecting group for the indole of tryptophan is removed by acid treatment in the presence of 1,2-ethanedithiol, 1,4-butanedithiol, or the like, as well as by alkali treatment with a dilute sodium hydroxide solution, dilute ammonia, or the like.

Protection and protecting groups for the functional groups that should not involve the reaction of the starting materials, eliminating the protecting groups, activation of the functional groups involved in the reaction, and the like can be selected as appropriate from among commonly known groups or commonly known means.

In another method of preparing an amide of the protein or partial peptide, for example, the α-carboxyl group of the carboxy-terminal amino acid is first amidated and hence protected, and a peptide (protein) chain is elongated to a desired chain length toward the amino group side, thereafter the protein or partial peptide having the protecting group for the N-terminal α-amino group of the peptide chain only removed and the protein or partial peptide having the protecting group for the C-terminal carboxyl group only removed are prepared, and these proteins or peptides are condensed in a mixed solvent as described above. For details about the condensation reaction, the same as those described above applies. After the protected protein or peptide obtained by the condensation is purified, all protecting groups can be removed by the above-described method to yield a desired crude protein or peptide. By purifying this crude protein or peptide using various publicly known means of purification, and freeze-drying the main fraction, a desired amide of the protein or peptide can be prepared.

In order to obtain an ester of the protein or peptide, a desired ester of the protein or peptide can be prepared by, for example, condensing the α-carboxyl group of the carboxy-terminal amino acid with a desired alcohol to yield an amino acid ester, and then treating the ester in the same manner as with an amide of the protein or peptide.

The partial peptide used in the present invention or a salt thereof can be produced according to a method of peptide synthesis known per se, or by cleaving the protein used in the present invention with an appropriate peptidase. The method of peptide synthesis may be any of, for example, a solid phase synthesis process and a liquid phase synthesis process. That is, a desired peptide can be produced by condensing a partial peptide or amino acid capable of constituting the partial peptide used in the present invention and the remaining portion, and eliminating any protecting group the resultant product may have. As examples of the commonly known method of condensation and elimination of the protecting group, methods described in
(i) to (v) below can be mentioned.

(i) M. Bodanszky and M.A. Ondetti: Peptide Synthesis, Interscience Publishers, New York (1966)
(ii) Schroeder and Luebke: The Peptide, Academic Press, New York (1965)
(iii) Nobuo Izumiya, et al.: Peptide Gosei-no-Kiso to Jikken, published by Maruzen Co. (1975)
(iv) Haruaki Yajima and Shunpei Sakakibara: Seikagaku Jikken Koza 1, Tanpakushitsu no Kagaku IV, 205 (1977)
(v) Haruaki Yajima, ed.: Zoku Iyakuhin no Kaihatsu, Vol. 14, Peptide Synthesis, published by Hirokawa Shoten.

After the reaction, the partial peptide used in the present invention can be purified and isolated by a combination of ordinary methods of purification, for example, solvent extraction, distillation, column chromatography, liquid chromatography, recrystallization and the like. When the partial peptide obtained by the above-described method is a free form, the free form can be converted to an appropriate salt by a commonly known method or a method based thereon; conversely, when the partial peptide is obtained in the form of a salt, the salt can be converted to a free form or another salt by a commonly known method or a method based thereon.

The polynucleotide that encodes the receptor used in the present invention may be any one comprising the above-described base sequence that encodes the receptor used in the present invention. The polynucleotide is preferably a DNA. The DNA may be any of a genomic DNA, a genomic DNA library, a cDNA derived from the above-described cell or tissue, a cDNA library derived from the above-described cell or tissue, and a synthetic DNA.

The vector used for the library may be any of bacteriophage, plasmid, cosmid, phagemid and the like. The vector can also be directly amplified by Reverse Transcriptase Polymerase Chain Reaction (hereinafter abbreviated as the RT-PCR method) using a total RNA or mRNA fraction prepared from the above-described cell/tissue.

As examples of the DNA that encodes the receptor used in the present invention,
(i) a DNA comprising the base sequence shown by SEQ ID NO:2, or a DNA comprising a base sequence that hybridizes with the base sequence shown by SEQ ID NO:2 under high stringent conditions, and encoding a protein having substantially the same quality of property as a protein comprising the amino acid sequence shown by SEQ ID NO:1,
(ii) a DNA comprising the base sequence shown by SEQ ID NO:4, or a DNA comprising a base sequence that hybridizes with the base sequence shown by SEQ ID NO:4 under high stringent conditions, and encoding a protein having substantially the same quality of property as a protein comprising the amino acid sequence shown by SEQ ID NO:3,
(iii) a DNA comprising the base sequence shown by SEQ ID NO:6, or a DNA comprising a base sequence that hybridizes with the base sequence shown by SEQ ID NO:6 under high stringent conditions, and encoding a protein having substantially the same quality of property as a protein comprising the amino acid sequence shown by SEQ ID NO:5,
(iv) a DNA comprising the base sequence shown by SEQ ID NO:8, or a DNA comprising a base sequence that hybridizes with the base sequence shown by SEQ ID NO:8 under high stringent conditions, and encoding a protein having substantially the same quality of property as a protein comprising the amino acid sequence shown by SEQ ID NO:7,
(v) a DNA comprising the base sequence shown by SEQ ID NO:10, or a DNA comprising a base sequence that hybridizes with the base sequence shown by SEQ ID NO:10 under high stringent conditions, and encoding a protein having substantially the same quality of property as a protein comprising the amino acid sequence shown by SEQ ID NO:9 and the like can be mentioned.

As the DNA capable of hybridizing with the base sequence shown by SEQ ID NO:2 under high stringent conditions, for example, a DNA comprising a base sequence having a homology of about 50% or more, preferably about 60% or more, preferably about 70% or more, preferably about 80% or more, preferably about 90% or more, preferably about 95% or more, to the base sequence shown by SEQ ID NO:2, and the like are used.

As the DNA capable of hybridizing with the base sequence shown by SEQ ID NO:4 under high stringent conditions, for example, a DNA comprising a base sequence having a homology of about 50% or more, preferably about 60% or more, preferably about 70% or more, preferably about 80% or more, preferably about 90% or more, preferably about 95% or more, to the base sequence shown by SEQ ID NO:4, and the like are used.

As the DNA capable of hybridizing with the base sequence shown by SEQ ID NO:6 under high stringent conditions, for example, a DNA comprising a base sequence having a homology of about 50% or more, preferably about 60% or more, preferably about 70% or more, preferably about 80% or more, preferably about 90% or more, preferably about 95% or more, to the base sequence shown by SEQ ID NO:6, and the like are used.

As the DNA capable of hybridizing with the base sequence shown by SEQ ID NO:8 under high stringent conditions, for example, a DNA comprising a base sequence having a homology of about 50% or more, preferably about 60% or more, preferably about 70% or more, preferably about 80% or more, preferably about 90% or more, preferably about 95% or more, to the base sequence shown by SEQ ID NO:8, and the like are used.

As the DNA capable of hybridizing with the base sequence shown by SEQ ID NO:10 under high stringent conditions, for example, a DNA comprising a base sequence having a homology of about 50% or more, preferably about 60% or more, preferably about 70% or more, preferably about 80% or more, preferably about 90% or more, preferably about 95% or more, to the base sequence shown by SEQ ID NO:10, and the like are used.

Hybridization can be conducted according to a method known per se or a method based thereon, for example, a method described in Molecular Cloning, 2nd edition (J. Sambrook et al., Cold Spring Harbor Lab. Press, 1989) and the like. When a commercially available library is used, hybridization can be conducted according to the method described in the attached instruction manual. Hybridization can more preferably be conducted under high stringent conditions.

High-stringent conditions refer to, for example, conditions involving a sodium concentration of about 19 to 40mM, preferably about 19 to 20mM, and a temperature of about 50 to 70°C, preferably about 60 to 65°C. In particular, a case wherein the sodium concentration is about 19mM and the temperature is about 65°C is most preferred.

More specifically, (i) as the DNA that encodes a protein comprising the amino acid sequence shown by SEQ ID NO:1, a DNA comprising the base sequence shown by SEQ ID NO:2 and the like can be used; (ii) as the DNA that encodes a protein comprising the amino acid sequence shown by SEQ ID NO:3, a DNA comprising the base sequence shown by SEQ ID NO:4 and the like can be used; (iii) as the DNA that encodes a protein comprising the amino acid sequence shown by SEQ ID NO:5, a DNA comprising the base sequence shown by SEQ ID NO:6 and the like can be used; (iv) as the DNA that encodes a protein comprising the amino acid sequence shown by SEQ ID NO:7, a DNA comprising the base sequence shown by SEQ ID NO:8 and the like can be used; v) as the DNA that encodes a protein comprising the amino acid sequence shown by SEQ ID NO:9, a DNA that encodes the base sequence shown by SEQ ID NO:10 and the like can be used.

The polynucleotide (e.g., DNA) that encodes the partial peptide used in the present invention may be any one comprising the above-described base sequence that encodes the partial peptide used in the present invention. The DNA may be any of a genomic DNA, a genomic DNA library, a cDNA derived from the above-described cell or tissue, a cDNA library derived from the above-described cell or tissue, and a synthetic DNA.

As the DNA that encodes the partial peptide used in the present invention, for example, a DNA having a portion of a DNA comprising the base sequence shown by SEQ ID NO:2, SEQ ID NO:4, SEQ ID NO:6, SEQ ID NO:8 or SEQ ID NO:10, or a DNA comprising a portion of a DNA comprising a base sequence that hybridizes with the base sequence shown by SEQ ID NO:2, SEQ ID NO:4, SEQ ID NO:6, SEQ ID NO:8 or SEQ ID NO:10 under high stringent conditions, and encoding a protein having substantially the same quality of activity as the protein of the present invention and the like are used.

As specific examples of the DNA that encodes the partial peptide used in the present invention, a DNA that encodes the polypeptide consisting of the 22nd to 416th amino acids of the amino acid sequence shown by SEQ ID NO:1, SEQ ID NO:3, SEQ ID NO:5, SEQ ID NO:7 or SEQ ID NO:9 or a partial peptide thereof; particularly a DNA that encodes the polypeptide consisting of the 22nd to 416th amino acids of the amino acid sequence shown by SEQ ID NO:3 or a partial peptide thereof is preferably used.

As the DNA that encodes the polypeptide consisting of the 22nd to 416th amino acids of the amino acid sequence shown by SEQ ID NO:1, SEQ ID NO:3, SEQ ID NO:5, SEQ ID NO:7 or SEQ ID NO:9 or a partial peptide thereof, for example, a DNA comprising the 64th to 1248th bases encoding the amino acid sequence shown by SEQ ID NO:2, SEQ ID NO:4, SEQ ID NO:6, SEQ ID NO:8 or SEQ ID NO:10, respectively, is used.

The DNA capable of hybridizing with the base sequence shown by SEQ ID NO:2, SEQ ID NO:4, SEQ ID NO:6, SEQ ID NO:8 or SEQ ID NO:10 has the same definition as that described above.

The method and high stringent conditions used for the hybridization are the same as those described above.

As a means of cloning a DNA that completely encodes the receptor or partial peptide used in the present invention (in the explanation of the cloning and expression of DNAs that encode them, these are sometimes simply abbreviated as the protein of the present invention), the DNA can be amplified by a PCR method using synthetic DNA primers having a portion of a base sequence that encodes the protein of the present invention, or selected by hybridization with a DNA incorporated in an appropriate vector or with a labeled DNA fragment or a labeled synthetic DNA that encodes a portion or the entire region of the protein of the present invention. The hybridization can be performed according to, for example, a method described in Molecular Cloning, 2nd Edition (J. Sambrook et al., Cold Spring Harbor Lab. Press, 1989) and the like. When a commercially available library is used, the hybridization can be performed according to the method described in the instruction manual attached thereto.

The base sequence of the DNA can be converted according to a method known per se, such as the ODA-LA PCR method, the Gapped duplex method, or the Kunkel method, or a method based thereon, using PCR, a commonly known kit, for example, Mutant^{™}-super Express Km (Takara Bio Inc.), Mutan^{™}-K (Takara Bio Inc.) and the like.

The cloned DNA that encodes the protein can be used as is, or after digestion with a restriction endonuclease or addition of a linker as desired, depending on the purpose of its use. The DNA may have the translation initiation codon ATG at the 5' end thereof, and the translation stop codon TAA, TGA or TAG at the 3' end thereof. These translation initiation codon and translation stop codons can be added using an appropriate synthetic DNA adapter.

An expression vector for the protein of the present invention can be produced by, for example, (i) cutting out a desired DNA fragment from a DNA that encodes the protein of the present invention, and (ii) joining the DNA fragment downstream of a promoter in an appropriate expression vector.

Useful vectors include plasmids derived from E. coli (e.g., pBR322, pBR325, pUC12, pUC13); plasmids derived from *Bacillus* subtilis (e.g., pUB110, pTP5, pC194); plasmids derived from yeast (e.g., pSH19, pSH15); bacteriophages such as λ phage; animal viruses such as retrovirus, vaccinia virus and baculovirus; pA1-11, pXT1, pRc/CMV, pRc/RSV, pcDNAI/Neo and the like.

The promoter used in the present invention may be any promoter appropriate for the host used to express the gene. For example, when an animal cell is used as the host, the SR_{α} promoter, the SV40 promoter, the LTR promoter, the CMV promoter, the HSV-TK promoter and the like can be mentioned. Of these promoters, the CMV (cytomegalovirus) promoter, the SR_{α} promoter and the like are preferably used.

When the host is a bacterium of the genus Escherichia, the trp promoter, the lac promoter, the recA promoter, the λP_{L} promoter, the lpp promoter, the T7 promoter and the like are preferred. When the host is a bacterium of the genus Bacillus, the SPO1 promoter, the SP02 promoter, the penP promoter and the like are preferred. When the host is yeast, the PHO5 promoter, the PGK promoter, the GAP promoter, the ADH promoter and the like are preferred. When the host is an insect cell, the polyhedrin promoter, the P10 promoter and the like are preferred.

Useful expression vectors include, in addition to the above, expression vectors that optionally comprises an enhancer, a splicing signal, a polyA addition signal, a selection marker, an SV40 replication origin (hereinafter also abbreviated as SV40ori), and the like. As examples of the selection markers, the dihydrofolate reductase (hereinafter also abbreviated as dhfr) gene [methotrexate (MTX) resistance], the ampicillin resistance gene (hereinafter also abbreviated as Amp^{r}), the neomycin resistance gene (hereinafter also abbreviated as NeO^{r}, G418 resistance), and the like can be mentioned. In particular, when a dhfr gene-defective Chinese hamster cell is used and the dhfr gene is used as the selection marker, a target gene can also be selected using a thymidine-free medium.

In addition, as required, a signal sequence that matches with the host may be added to the N-terminal of the protein of the present invention. Useful signal sequences include a PhoA signal sequence, an OmpA signal sequence and the like when the host is a bacterium of the genus Escherichia; an α-amylase signal sequence, a subtilisin signal sequence and the like when the host is a bacterium of the genus Bacillus; an MFα signal sequence, an SUC2 signal sequence and the like when the host is yeast; and an insulin signal sequence, an α-interferon signal sequence, an antibody molecule signal sequence and the like when the host is an animal cell.

Using the thus-constructed vector comprising a DNA that encodes the protein of the present invention, a transformant can be produced.

As useful examples of the host, a bacterium of the genus Escherichia, a bacterium of the genus Bacillus, yeast, an insect cell, an insect, an animal cell, and the like can be mentioned.

As specific examples of the bacterium of the genus *Escherichia, Escherichia* coli K12 DH1 *(*Proc. Natl. Acad. Sci. U.S.A., Vol. 60, 160 (1968)), JM103 *(*Nucleic Acids Research, Vol. 9, 309 (1981)), JA221 (Journal of Molecular Biology, Vol. 120, 517 (1978)), HB101 (Journal of Molecular Biology, Vol. 41, 459 (1969)), C600 (Genetics, Vol. 39, 440 (1954)), and the like can be mentioned.

As useful examples of the bacterium of the genus Bacillus, Bacillus subtilis MI114 (Gene, Vol. 24, 255 (1983)), 207-21 (Journal of Biochemistry, Vol. 95, 87 (1984)) and the like can be mentioned.

As useful examples of the yeast, Saccharomyces cerevisiae AH22, AH22R⁻, NA87-11A, DKD-5D and 20B-12, Schizosaccharomyces pombe NCYC1913 and NCYC2036, Pichia pastoris KM71 and the like can be mentioned.

As useful examples of the insect cell, Spodoptera frugiperda cell (Sf cell), MG1 cell derived from the mid-intestine of Trichoplusia ni, High Five^{™} cell derived from an egg of Trichoplusia ni, cell derived from Mamestra brassicae, cell derived from Estigmena acrea, and the like can be mentioned when the virus is AcNPV. When the virus is BmNPV, Bombyx mori N cell (BmN cell) and the like can be used. As useful examples of the Sf cell, Sf9 cell (ATCC CRL1711), Sf21 cell (both in Vaughn, J. L. et al., In Vivo, 13, 213-217 (1977), and the like can be mentioned.

As useful examples of the insect, a larva of Bombyx mori (Maeda et al., Nature, Vol. 315, 592 (1985)), and the like can be mentioned.

As useful examples of the animal cell, monkey cell COS-7, Vero, Chinese hamster cell CHO (hereinafter abbreviated as CHO cell), Chinese hamster cell (CHO) lacking the dhfr gene (hereinafter abbreviated as CHO (dhfr⁻) cell), mouse L cell, mouse AtT-20, mouse myeloma cell, mouse ATDC5 cell, rat GH3, human FL cell and the like can be mentioned.

A bacterium of the genus *Escherichia* can be transformed, for example, in accordance with a method described in Proc. Natl. Acad. Sci. USA, Vol.69, 2110 (1972), Gene, Vol.17, 107 (1982) and the like.

A bacterium of the genus *Bacillus* can be transformed, for example, according to a method described in Molecular & General Genetics, Vol.168, 111 (1979) and the like.

Yeast can be transformed, for example, in accordance with a method described in Methods in Enzymology, Vol.194, 182-187 (1991), Proc. Natl. Acad. Sci. USA, Vol.75, 1929 (1978) and the like.

An insect cell or insect can be transformed, for example, according to a method described in Bio/Technology, 6, 47-55 (1988) and the like.

An animal cell can be transformed, for example, in accordance with a method described in Saibo Kogaku (Cell Engineering), extra issue 8, Shin Saibo Kogaku Jikken Protocol (New Cell Engineering Experimental Protocol), 263-267 (1995) (published by Shujunsha), or Virology, Vol.52, 456 (1973).

Thus, a transformant transformed with an expression vector comprising a DNA that encodes the protein can be obtained.

When a transformant whose host is a bacterium of the genus *Escherichia* or a bacterium of the genus *Bacillus* is cultured, the culture medium used is preferably a liquid medium, in which a carbon source, a nitrogen source, an inorganic substance and others necessary for the growth of the transformant are contained. As examples of the carbon source, glucose, dextrin, soluble starch, sucrose and the like can be mentioned; as examples of the nitrogen source, inorganic or organic substances such as an ammonium salt, a nitrate salt, corn steep liquor, peptone, casein, meat extract, soybean cake, and potato extract can be mentioned; as examples of the inorganic substance, calcium chloride, sodium dihydrogen phosphate, magnesium chloride and the like can be mentioned. In addition, yeast extract, vitamins, a growth promoting factor and the like may be added. The pH of the medium is desirably about 5 to 8.

As an example of the medium used to culture a bacterium of the genus *Escherichia,* an M9 medium comprising glucose and casamino acid [Miller, Journal of Experiments in Molecular Genetics, 431-433, Cold Spring Harbor Laboratory, New York, 1972] is preferable. As required, in order to increase promoter efficiency, a chemical agent, for example, 3β-indolylacrylic acid, may be added to the medium.

When the host is a bacterium of the genus *Escherichia,* cultivation is normally performed at about 15 to 43°C for about 3 to 24 hours, and the culture may be aerated or agitated as necessary.

When the host is a bacterium of the genus *Bacillus,* cultivation is normally performed at about 30 to 40°C for about 6 to 24 hours, and the culture may be aerated or agitated as necessary.

When a transformant whose host is yeast is cultured, as examples of the medium, Burkholder's minimal medium [Proc. Natl. Acad. Sci. USA, Vol.77, 4505(1980)] and an SD medium supplemented with 0.5% casamino acid [Proc. Natl. Acad. Sci. USA, Vol.81, 5330(1984)] can be mentioned. The pH of the medium is preferably adjusted to about 5 to 8. Cultivation is normally performed at about 20°C to 35°C for about 24 to 72 hours, and the culture may be aerated or agitated as necessary.

When a transformant whose host is an insect cell or insect is cultured, as the medium, Grace's Insect Medium (Nature, 195, 788(1962)) supplemented with inactivated 10% bovine serum and other additives as appropriate and the like are used. The pH of the medium is preferably adjusted to about 6.2 to 6.4. Cultivation is normally performed at about 27°C for about 3~5 days, and the culture may be aerated or agitated as necessary.

Useful medium for cultivating a transformant whose host is an animal cell include, for example, MEM medium supplemented with about 5 to 20% fetal bovine serum [Science, Vol. 122, 501(1952)], DMEM medium [Virology, Vol. 8, 396(1959)], RPMI 1640 medium [The Journal of the American Medical Association, Vol. 199, 519(1967)], 199 medium [Proceeding of the Society for the Biological Medicine, Vol. 73, 1(1950)] and the like. The medium's pH is preferably about 6 to 8. Cultivation is normally performed at about 30 to 40°C for about 15 to 60 hours, and the culture may be aerated or agitated as necessary.

Thus, the protein of the present invention can be produced in the cells, on the cell membrane or out of the cells of the transformant.

Separation and purification of the protein of the present invention from the above-described culture can be performed by, for example, the method described below.

When the protein of the present invention is extracted from a cultured bacterium or cells, a method is used as appropriate wherein the bacterium or cells are collected by a commonly known method after cultivation, suspended in an appropriate buffer solution, and disrupted by means of sonication, lysozyme and/or freeze-thawing and the like, after which a crude extract of the protein is obtained by centrifugation or filtration. The buffer solution may contain a protein denaturant such as urea or guanidine hydrochloride and a surfactant such as Triton X-100^{™}. When the protein is secreted in the culture broth, the bacterium or cells are separated from the supernatant by a method known per se, and the supernatant is collected, after completion of the cultivation.

Purification of the protein contained in the thus-obtained culture supernatant or extract can be performed by an appropriate combination of methods of separation/purification known per se. These commonly known methods of separation/purification include methods based on solubility, such as salting-out and solvent precipitation; methods based mainly on differences in molecular weight, such as dialysis, ultrafiltration, gel filtration, and SDS-polyacrylamide gel electrophoresis; methods based on differences in electric charge, such as ion exchange chromatography; methods based on specific affinity, such as affinity chromatography; methods based on differences in hydrophobicity, such as reverse phase high performance liquid chromatography; methods based on differences in isoelectric point, such as isoelectric focusing; and the like.

When the protein thus obtained is a free form, the free form can be converted to a salt by a method known per se or a method based thereon; conversely, when the protein is obtained in the form of a salt, the salt can be converted to a free form or another salt by a method known per se or a method based thereon.

The protein produced by the transformant can be optionally modified or partially deprived of a polypeptide by allowing an appropriate protein-modifying enzyme to act thereon before the purification or after the purification. As the protein-modifying enzyme used, for example, trypsin, chymotrypsin, arginyl endopeptidase, protein kinase, glycosidase and the like are used.

The presence of the protein of the present invention thus produced can be measured by an enzyme immunoassay, Western blotting and the like using a specific antibody.

The "neutralizing antibody against a protein comprising the same or substantially the same amino acid sequence as that shown by SEQ ID NO:1, SEQ ID NO:3, SEQ ID NO:5, SEQ ID NO:7 or SEQ ID NO:9, or a partial peptide thereof or a salt thereof (the receptor used in the present invention)" may be any antibody capable of inhibiting the binding of the receptor used in the present invention and a ligand thereof; for example, an antibody that reacts specifically to the receptor used in the present invention, a bispecific antibody that reacts specifically to the receptor used in the present invention, an antibody that inhibits an activity (e.g., binding activity for ligand such as collagen, activity to undergo phosphorylation and the like) of the receptor used in the present invention (hereinafter these are also generically referred to as the antibody of the present invention) and the like can be mentioned.

The antibody of the present invention may be any of a polyclonal antibody and a monoclonal antibody.

The antibody of the present invention may also be a chimeric antibody, humanized or human antibody, considering the therapeutic effect and safety in humans.

The antibody of the present invention is preferably a neutralizing antibody having an activity to neutralize the apoptosis-inducing stimulation or cancer cell growth stimulation resulting from the binding of a ligand such as a collagen (for example, type I collagen, type II collagen, type III collagen, type IV collagen, type V collagen, type VI collagen, type VIII collagen) and the receptor used in the present invention.

As the antibody of the present invention, an antibody against the polypeptide consisting of the 22nd to 416th amino acids of the amino acid sequence shown by SEQ ID NO:1, SEQ ID NO:3, SEQ ID NO:5, SEQ ID NO:7 or SEQ ID NO:9 or a partial peptide thereof or a salt thereof is preferable; particularly, an antibody against the polypeptide which amino acid sequence is from the 22nd to the 416th of that shown by SEQ ID NO:3 or a partial peptide thereof or a salt thereof is preferably used.

Described below are the method of preparing an antigen of the antibody of the present invention, and the method of producing the antibody.

### (1) Preparation of antigen

As examples of the antigen used to prepare the antibody of the present invention, any of a peptide (e.g., a synthetic peptide) having 1 kind or 2 kinds or more of the same antigen determinant as that of a protein comprising the amino acid sequence shown by SEQ ID NO:1, SEQ ID NO:3, SEQ ID NO:5, SEQ ID NO:7 or SEQ ID NO:9 or a partial peptide thereof or a salt thereof and the like can be used (hereinafter these are also simply referred to as the antigen of the present invention). The partial peptide used as the antigen can be the entire extracellular region of the above-described protein, or an immunogenic peptide (epitope) contained in the region. The length of the immunogenic peptide is not subject to limitation, as long as it is a length having immunogenicity, and the length can be, for example, one having 8, preferably 10, more preferably 12, continuous amino acid residues.

The above-described protein or a partial peptide thereof or a salt thereof can be produced in accordance with Reference Examples described below or a commonly known method, and can also be produced by (a) preparing from, for example, a tissue or cells of a mammal such as a human, monkey, rat, or mouse, using a commonly known method or a method based thereon, (b) chemically synthesizing by a commonly known method of peptide synthesis using a peptide synthesizer and the like, or (c) culturing a transformant comprising a DNA that encodes a polypeptide comprising the amino acid sequence shown by SEQ ID NO:1, SEQ ID NO:3, SEQ ID NO:5, SEQ ID NO:7 or SEQ ID NO:9 or a salt thereof.
(a) When the antigen of the present invention is prepared from a tissue or cells of the mammal, the tissue or cells may be homogenized, and then the crude fraction (e.g., membrane fraction, soluble fraction) can also be used as is as the antigen. Alternatively, the antigen of the present invention can also be purified and isolated by performing extraction with an acid, surfactant or alcohol and the like, and applying the extract to a combination of salting-out, dialysis, gel filtration, and chromatographies such as reversed-phase chromatography, ion exchange chromatography, and affinity chromatography.
(b) When the antigen of the present invention is chemically prepared, examples of the synthetic peptide used include one having the same structure as that of the antigen of the present invention purified from a natural material using the above described method (a), a peptide comprising 1 kind or 2 kinds or more of the same amino acid sequence as the amino acid sequence consisting of 3 or more, preferably 6 or more amino acids in an optionally chosen portion of the amino acid sequence shown by SEQ ID NO:1, SEQ ID NO:3, SEQ ID NO:5, SEQ ID NO:7 or SEQ ID NO:9 and the like.
(c) When a protein comprising the amino acid sequence shown by SEQ ID NO:1, SEQ ID NO:3, SEQ ID NO:5, SEQ ID NO:7 or SEQ ID NO:9 or a salt thereof is produced using a transformant comprising a DNA, the DNA can be prepared according to a commonly known method of cloning [for example, the method described in Molecular Cloning (2nd ed.; J. Sambrook et al., Cold Spring Harbor Lab. Press, 1989) and the like]. As the method of cloning, (1) a method comprising obtaining a transformant comprising a DNA that encodes a protein comprising the amino acid sequence shown by SEQ ID NO:1, SEQ ID NO:3, SEQ ID NO:5, SEQ ID NO:7 or SEQ ID NO:9 or a salt thereof from a cDNA library by a hybridization method using a DNA probe or DNA primers designed on the basis of the amino acid sequence of a protein comprising the amino acid sequence shown by SEQ ID NO:1, SEQ ID NO:3, SEQ ID NO:5, SEQ ID NO:7 or SEQ ID NO:9 or a salt thereof, or (2) a method comprising obtaining a transformant comprising a DNA that encodes a protein comprising the amino acid sequence shown by SEQ ID NO:1, SEQ ID NO:3, SEQ ID NO:5, SEQ ID NO:7 or SEQ ID NO:9 or a salt thereof by a PCR method using DNA primers designed on the basis of the amino acid sequence of a polypeptide comprising the amino acid sequence shown by SEQ ID NO:1, SEQ ID NO:3, SEQ ID NO:5, SEQ ID NO:7 or SEQ ID NO:9 or a salt thereof and the like can be mentioned.

The mammalian cell that expresses the receptor used in the present invention per se can also be used directly as the antigen of the present invention. As the mammalian cell, natural cells as described in term (a) above, cells transformed by a method as described in term (c) above and the like can be used. The host used for the transformation may be any cell collected from a human, a monkey, a rat, a mouse, a hamster and the like; HEK293, COS7, CHO-K1, NIH3T3, Balb3T3, FM3A, L929, SP2/0, P3U1, B16, or P388 and the like are preferably used. Natural mammalian cells or transformed mammalian cells that express the receptor used in the present invention can be injected to an immunized animal in suspension in a medium used for tissue culture (e.g., RPMI1640) or a buffer solution (e.g., Hanks' Balanced Salt Solution). The method of immunization may be any method that enables promotion of antibody production; intravenous injection, intraperitoneal injection, intramuscular injection or subcutaneous injection and the like are preferably used.

A peptide as the antigen of the present invention can also be produced (1) according to a commonly known method of peptide synthesis, or (2) by cleaving a protein comprising the amino acid sequence shown by SEQ ID NO:1, SEQ ID NO:3, SEQ ID NO:5, SEQ ID NO:7 or SEQ ID NO:9 with an appropriate peptidase.

The method of peptide synthesis may be any of, for example, a solid phase synthesis process and a liquid phase synthesis process. That is, a desired peptide can be produced by condensing a partial peptide or amino acids capable of constituting the peptide and the remaining portion, and eliminating any protecting group the resultant product may have. As examples of the commonly known methods of condensation and elimination of the protecting group, the methods described below and the like can be mentioned.
(i) M. Bodanszky and M.A. Ondetti, Peptide Synthesis, Interscience Publishers, New York (1966)
(ii) Schroeder and Luebke, The Peptide, Academic Press, New York (1965)

After the reaction, the peptide can be purified and isolated by a combination of ordinary methods of purification, for example, solvent extraction, distillation, column chromatography, liquid chromatography, recrystallization and the like. When the peptide obtained by the above-described method is a free form, the free form can be converted to an appropriate salt by a commonly known method; conversely, when the peptide is obtained in the form of a salt, the salt can be converted to a free form by a commonly known method.

To prepare an amide of the peptide, a commercially available resin for peptide synthesis suitable for amide formation can be used. As examples of such resins, chloromethyl resin, hydroxymethyl resin, benzhydrylamine resin, aminomethyl resin, 4-benzyloxybenzyl alcohol resin, 4-methylbenzhydrylamine resin, PAM resin, 4-hydroxymethylmethylphenylacetamidomethyl resin, polyacrylamide resin, 4-(2',4'-dimethoxyphenyl-hydroxymethyl)phenoxy resin, 4-(2',4'-dimethoxyphenyl-Fmoc-aminoethyl)phenoxy resin and the like can be mentioned. Using such a resin, an amino acid having an appropriately protected α-amino group and side chain functional group is condensed on the resin in accordance with the sequence of the desired protein according to various methods of condensation known *per se.* At the end of the reaction, the peptide is cleaved from the resin, and at the same time various protecting groups are removed, to obtain the desired peptide. Alternatively, the desired peptide can also be obtained by taking out a partially protected peptide using chlorotrityl resin, oxime resin, 4-hydroxybenzoate resin and the like, and removing protecting groups by a conventional means.

For the above-described condensation of protected amino acids, various activation reagents which can be used for peptide synthesis can be used, and a carbodiimide is preferably used. As the carbodiimide, DCC, N,N'-diisopropylcarbodiimide, N-ethyl-N'-(3-dimethylaminopropyl)carbodiimide and the like can be mentioned. For the activation using them, the protected amino acid, along with a racemization-suppressing additive (for example, HOBt, HOOBt and the like), may be added directly to the resin, or the protected amino acid may be activated in advance as a symmetric acid anhydride or an HOBt ester or an HOOBt ester, and then added to the resin. Solvents used for the activation of protected amino acids and condensation thereof with a resin can be appropriately selected from among solvents known to be usable for peptide condensation reactions. As examples of useful solvents, acid amides such as N,N-dimethylformamide, N,N-dimethylacetamide and N-methylpyrrolidone; halogenated hydrocarbons such as methylene chloride and chloroform; alcohols such as trifluoroethanol; sulfoxides such as dimethyl sulfoxide; tertiary amines such as pyridine; ethers such as dioxane and tetrahydrofuran; nitriles such as acetonitrile and propionitrile; esters such as methyl acetate and ethyl acetate; suitable mixtures thereof; and the like can be mentioned. Reaction temperature is appropriately selected from the range known to be usable for peptide bond formation reactions, and is normally selected from the range of about -20°C to about 50°C. An activated amino acid derivative is normally used from about 1.5 to about 4 times in excess. If a test using the ninhydrin reaction reveals that the condensation is insufficient, sufficient condensation can be performed by repeating the condensation reaction without elimination of protecting groups. If the condensation is insufficient even though the reaction is repeated, unreacted amino acids may be acetylated using acetic anhydride or acetylimidazole to prevent the subsequent reaction from being influenced.

As examples of the protecting group for the amino group of the amino acid to be the starting material, Z, Boc, tertiary pentyloxycarbonyl, isobornyloxycarbonyl, 4-methoxybenzyloxycarbonyl, Cl-Z, Br-Z, adamantyloxycarbonyl, trifluoroacetyl, phthaloyl, formyl, 2-nitrophenylsulphenyl, diphenylphosphinothioyl, Fmoc and the like can be mentioned. As examples of the protecting group for the carboxyl group, C₁-6 alkyl groups, C₃₋₈ cycloalkyl groups, C₇₋₁₄ aralkyl groups, 2-adamantyl, 4-nitrobenzyl, 4-methoxybenzyl, 4-chlorobenzyl, phenacyl and benzyloxycarbonylhydrazide, tertiary butoxycarbonylhydrazide, tritylhydrazide and the like can be mentioned.

The hydroxyl groups of serine and threonine can be protected by, for example, esterification or etherification. As examples of a group suitable for this esterification, lower (C₁₋₆) alkanoyl groups such as an acetyl group; aroyl groups such as a benzoyl group; and groups derived from carbonic acid such as a benzyloxycarbonyl group and an ethoxycarbonyl group, and the like can be mentioned. As examples of a group suitable for etherification, a benzyl group, a tetrahydropyranyl group, a t-butyl group and the like can be mentioned.

As examples of the protecting group for the phenolic hydroxyl group of tyrosine, Bzl, Cl-Bzl, 2-nitrobenzyl, Br-Z, t-butyl and the like can be mentioned.

As the protecting group for the imidazole of histidine, Tos, 4-methoxy-2,3,6-trimethylbenzenesulfonyl, DNP, Bom, Bum, Boc, Trt, Fmoc and the like can be mentioned.

As examples of the carboxyl group of the starting material in an activated form, a corresponding acid anhydride, an azide, an activated ester [an ester with an alcohol (for example, pentachlorophenol, 2,4,5-trichlorophenol, 2,4-dinitrophenol, cyanomethyl alcohol, p-nitrophenol, HONB, N-hydroxysuccimide, N-hydroxyphthalimide, or HOBt)] and the like can be mentioned. As examples of the amino group of the starting material in an activated form, a corresponding phosphoric amide can be mentioned.

As examples of the method of removing (eliminating) a protecting group, catalytic reduction in a hydrogen stream in the presence of a catalyst such as Pd-black or Pd-carbon; acid treatment by means of anhydrous hydrogen fluoride, methanesulfonic acid, trifluoromethanesulfonic acid, trifluoroacetic acid or a mixed solution thereof; base treatment by means of diisopropylethylamine, triethylamine, piperidine, piperazine or the like; and reduction with sodium in liquid ammonia, and the like can also be mentioned. The elimination reaction by the above-described acid treatment is generally performed at a temperature of -20°C to 40°C; the acid treatment is efficiently performed by adding a cation scavenger like anisole, phenol, thioanisole, m-cresol, p-cresol, dimethylsulfide, 1,4-butanedithiol and 1,2-ethanedithiol. Also, the 2,4-dinitrophenyl group used as a protecting group for the imidazole of histidine is removed by thiophenol treatment; the formyl group used as a protecting group for the indole of tryptophan is removed by the above-described acid treatment in the presence of 1,2-ethanedithiol, 1,4-butanedithiol or the like, as well as by alkali treatment with a dilute sodium hydroxide, dilute ammonia or the like.

Protection and protecting groups for the functional groups that should not involve the reaction of the starting materials, eliminating the protecting groups, activation of the functional groups involved in the reaction, and the like can be selected as appropriate from among commonly known groups or commonly known means.

In another method of preparing an amide of the peptide, for example, the α-carboxyl group of the carboxy-terminal amino acid is first amidated, and a peptide chain is elongated to a desired chain length toward the amino group side, thereafter the peptide having the protecting group for the N-terminal α-amino group of the peptide chain only removed and the peptide (or amino acid) having the protecting group for the C-terminal carboxyl group only removed are prepared, and these peptides are condensed in a mixed solvent described above. For details about the condensation reaction, the same as those described above applies. After the protected peptide obtained by the condensation is purified, all protecting groups can be removed by the above-described method to yield a desired crude peptide. By purifying this crude peptide using various publicly known means of purification, and freeze-drying the main fraction, a desired amide of the peptide can be prepared.

In order to obtain an ester of the peptide, a desired ester of the peptide can be prepared by, for example, condensing the α-carboxyl group of the carboxy-terminal amino acid with a desired alcohol to yield an amino acid ester, and then treating the ester in the same manner as with an amide of the peptide.

The antigen of the present invention permits direct use for immunization in an insolubilized form. The antigen of the present invention may be used for immunization in the form of a conjugate thereof bound or adsorbed to a suitable carrier. Regarding the mixing ratio of the carrier and the antigen of the present invention (hapten), any carrier can be bound or adsorbed in any ratio, as long as an antibody against the antigen of the present invention bound or adsorbed to the carrier is efficiently produced; usually, a natural or synthetic polymeric carrier in common use for preparation of an antibody against a hapten antigen, bound or adsorbed in a ratio of 0.1 to 100 parts by weight to 1 part by weight of the hapten, can be used. As examples of the natural polymeric carrier, the serum albumin of a mammal such as cattle, rabbit, or human, the thyroglobulin of a mammal such as cattle or rabbit, the hemoglobin of a mammal such as cattle, rabbit, human, or sheep, keyhole limpet hemocyanin and the like are used. As examples of the synthetic polymeric carrier, various latexes of polymers or copolymers of polyamino acids, polystyrenes, polyacryls, polyvinyls, polypropylenes and the like, and the like can be used.

Various condensing agents can be used for crosslinking the hapten and carrier. For example, diazonium compounds such as bisdiazotized benzidine, which crosslink tyrosine, histidine, and tryptophan; dialdehyde compounds such as glutaraldehyde, which crosslink amino groups together; diisocyanate compounds such as toluene-2,4-diisocyanate; dimaleimide compounds such as N,N'-o-phenylenedimaleimide, which crosslink thiol groups together; maleimide activated ester compounds, which crosslink amino groups and thiol groups; carbodiimide compounds, which crosslink amino groups and carboxyl groups; and the like are conveniently used. When amino groups are crosslinked together, it is also possible to react one amino group with an activated ester reagent having a dithiopyridyl group (for example, 3-(2-pyridyldithio)propionic acid N-succinimidyl (SPDP) and the like), followed by reduction, to introduce the thiol group, and to introduce a maleimide group into the other amino group using a maleimide activated ester reagent, followed by a reaction of both.

### (2) Preparation of monoclonal antibody

The antigen of the present invention is administered to a warm-blooded animal by, for example, a method of administration such as intraperitoneal injection, intravenous injection, or subcutaneous injection, alone per se or along with a carrier or a diluent, to a site permitting antibody production. In order to increase antibody productivity during the administration, complete Freund's adjuvant or incomplete Freund's adjuvant may be administered. The administration is usually made every 2 to 6 weeks about 2 to 10 times in total. In preparing the monoclonal antibody of the present invention, the DNA immunization method may be utilized (see, for example, Nature, Vol.356, term 152 to term 154). As the warm-blooded animal, for example, monkeys, rabbits, dogs, guinea pigs, mice, rats, sheep, goat, chicken and the like can be mentioned; for preparing the monoclonal antibody, a mouse or a rabbit is preferably used.

In preparing the monoclonal antibody, an individual found to have an antibody titer is selected from among warm-blooded animals, for example, mice, immunized with the antigen of the present invention, its spleen or lymph node are collected at 2 to 5 days after final immunization, and the antibody-producing cells contained therein are fused with myeloma cells, whereby a hybridoma that produces the antibody of the present invention can be prepared. A measurement of the antibody titer of the antibody of the present invention in serum is performed by, for example, labeling the receptor used in the present invention with a radioactive substance or an enzyme and the like, and reacting it with an antiserum, and then measuring the activity of the labeling agent bound to the antibody. The fusion operation can be performed according to a publicly known method, for example, the Koehler and Milstein method [Nature, Vol.256, page 495 (1975)]. As the fusion promoter, polyethylene glycol (PEG), Sendai virus and the like can be mentioned; preferably, PEG and the like are used. As examples of the myeloma cell, NS-1, P3U1, SP2/0, AP-1 and the like can be mentioned, and P3U1 and the like are preferably used. A preferable ratio of the number of antibody-producing cells (splenocytes) and number of myeloma cells used is generally about 1:1 to 20:1; cell fusion can be efficiently performed by adding a PEG (preferably PEG1000 to PEG6000) at concentrations of about 10 to 80%, and conducting incubation generally at 20 to 40°C, preferably at 30 to 37°C, generally for 1 to 10 minutes.

For screening for the hybridoma that produces the antibody of the present invention, various methods can be used; for example, a method comprising adding the hybridoma culture supernatant to a solid phase (e.g., microplate) adsorbed with a protein comprising the amino acid sequence shown by SEQ ID NO:1, SEQ ID NO:3, SEQ ID NO:5, SEQ ID NO:7 or SEQ ID NO:9 or a salt thereof or a partial peptide thereof directly or via a carrier, then adding an anti-immunoglobulin antibody (when mouse cells are used for the cell fusion, an anti-mouse immunoglobulin antibody is used) labeled with a radioactive substance, an enzyme or the like, or Protein A, and detecting the antibody of the present invention bound to the solid phase; a method comprising adding the hybridoma culture supernatant to a solid phase adsorbed with an anti-immunoglobulin antibody or protein A, adding a polypeptide comprising the amino acid sequence shown by SEQ ID NO:1, SEQ ID NO:3, SEQ ID NO:5, SEQ ID NO:7 or SEQ ID NO:9, labeled with a radioactive substance, an enzyme or the like, and detecting the antibody of the present invention bound to the solid phase; and the like can be mentioned. Screening for the antibody of the present invention and its breeding can be performed usually in a medium for animal cells supplemented with HAT (hypoxanthine, aminopterin, thymidine). Any medium for the selection and breeding can be used as far as the hybridoma can grow therein. For example, an RPMI 1640 medium comprising 1 to 20%, preferably 10 to 20%, fetal calf serum, a GIT medium (Wako Pure Chemical Industries, Ltd.) comprising 1 to 10% fetal calf serum, a serum free medium for hybridoma culture (SFM-101, Nissui Seiyaku Co., Ltd.) and the like can be used. Cultivation temperature is normally 20 to 40°C, preferably about 37°C. Cultivation time is normally 5 days to 3 weeks, preferably 1 week to 2 weeks. The cultivation can be performed normally in the presence of 5% gaseous carbon dioxide.

Separation and purification of the antibody of the present invention, like the separation and purification of an ordinary polyclonal antibody, is performed by a method of immunoglobulin separation and purification [e.g., salting-out, alcohol precipitation, isoelectric point precipitation, electrophoresis, adsorption and desorption with ion exchanger (e.g., DEAE), ultracentrifugation, gel filtration, or specific purification comprising collecting the antibody only with an antigen-bound solid phase or an activated adsorbent such as Protein A or Protein G, and dissociating the bond to give the antibody, and the like].

Thus, hybridoma cells are cultured in the living body of a warm-blooded animal or in vitro, and the antibody is collected from a body fluid or culture thereof, whereby the antibody of the present invention can be produced.

Screening for (a) a hybridoma that produces the antibody of the present invention that reacts with a partial region of a protein comprising the amino acid sequence shown by SEQ ID NO:1, SEQ ID NO:3, SEQ ID NO:5, SEQ ID NO:7 or SEQ ID NO:9, and (b) a hybridoma that produces the antibody of the present invention that reacts with the above-described protein but does not react with a partial region thereof can be performed by, for example, measuring the binding capacity to a peptide corresponding to the partial region and the antibody produced by the hybridoma.

A bispecific monoclonal antibody that specifically reacts with the receptor used in the present invention can be produced in accordance with a commonly known method.

Furthermore, a chimeric antibody, a humanized antibody, or a human antibody can be produced in accordance with a commonly known method; for example, a chimeric antibody can be prepared with reference to, for example, "Jikken Igaku (extra issue), Vol.6, No. 10, 1988", Japanese Patent Kokoku Publication No. HEI-3-73280 and the like; a humanized antibody can be prepared with reference to, for example, Japanese Patent Kohyo Publication No. HEI-4-506458, Japanese Patent Kokai Publication No. SHO-62-296890 and the like; and a human antibody can be prepared with reference to, for example, "Nature Genetics, Vol. 15, p.146-156, 1997", "Nature Genetics, Vol.7, p.13-21, 1994", Japanese Patent Kohyo Publication No. HEI-4-504365, Official Gazette for International Patent Application Publication No. WO94/25585, "Nikkei Science, June issue, p.40 to 50, 1995", "Nature, Vol.368, p.856-859, 1994", Japanese Patent Kohyo Publication No. HEI-6-500233 and the like.

### [Preparation of polyclonal antibody]

The polyclonal antibody of the present invention can be produced according to a method know per se or a method based thereon. For example, the polyclonal antibody can be produced by immunizing an immune antigen per se or a complex of the antigen and a carrier protein to a warm-blooded animal in the same manner as the above-described method of producing a monoclonal antibody, collecting a product containing the antibody of the present invention from the immunized animal, and separating and purifying the antibody.

Regarding the complex of the immune antigen and carrier protein used to immunize a warm-blooded animal, any type of carrier protein and any mixing ratio of the carrier protein and hapten can be used, as long as an antibody against the hapten used for immunization as crosslinked to the carrier protein is efficiently produced; for example, a method wherein bovine serum albumin, bovine thyroglobulin, hemocyanin or the like is crosslinked in a ratio of about 0.1 to 20, preferably about 1 to 5, parts by weight to 1 part by weight of the hapten, is used.

Various condensing agents can be used for crosslinking the hapten and carrier protein; glutaraldehyde, carbodiimide, maleimide activated ester, activated ester reagents containing a thiol group or dithiopyridyl group, and the like can be used.

The condensation product is administered to a warm-blooded animal as is or along with a carrier or a diluent to a site permitting antibody production. In order to increase antibody productivity during the administration, complete Freund's adjuvant or incomplete Freund's adjuvant may be administered. The administration is usually made about every 2 to 6 weeks about 3 to 10 times in total.

The polyclonal antibody of the present invention can be prepared by the DNA immunization method (see, for example, Nature, Vol.356, term 152 to term 154). According to the DNA immunization method, an antibody having an excellent titer is obtained.

The polyclonal antibody can be collected from blood, ascites fluid and the like, preferably blood, of a warm-blooded animal immunized by the above-described method.

The polyclonal antibody titer in antiserum can be measured in the same manner as, for example, the measurement of the antibody titer of the hybridoma culture supernatant, described in (2) above. Separation and purification of the polyclonal antibody can be performed according to the same method of immunoglobulin separation and purification as the above-described separation and purification of a monoclonal antibody.

Described below are intended uses of the antibody of the present invention.

### [1] Cancer prophylactic/therapeutic agent, cancer cell apoptosis inducer, and cancer cell growth suppressant

The receptor used in the present invention exhibits upregulated expression in cancer cells such as breast cancer, ovarian cancer, colorectal cancer, lung cancer, and pancreatic cancer, and suppresses cancer cell apoptosis by being bound by a ligand such as a collagen. This cancer cell apoptosis suppression phenomenon is neutralized and apoptosis is induced by inhibiting, for example (i) the binding of a ligand such as a collagen and the receptor used in the present invention, (ii) induction of the activation of the receptor used in the present invention (e.g., induction/promotion of activity to undergo phosphorylation and the like) and the like.

Therefore, a medicament comprising the antibody of the present invention (including a salt thereof) can be used as a safe medicament of low toxicity, for example, a prophylactic/therapeutic agent for cancers (e.g., breast cancer, ovarian cancer, colorectal cancer, lung cancer, pancreatic cancer and the like), a cancer cell apoptosis inducer, a cancer cell growth suppressant and the like.

The above-described agent comprising the antibody of the present invention or the above-described substance is of low toxicity, and can be orally or parenterally (e.g., intravascular administration, intraperitoneal administration, subcutaneous administration and the like) administered to a human or a mammal (e.g., rat, rabbit, sheep, pig, cattle, cat, dog, monkey and the like) as a liquid as is or as a pharmaceutical composition in an appropriate dosage form.

The antibody of the present invention may be administered as is, or may be administered as an appropriate pharmaceutical composition. The pharmaceutical composition used for the administration may comprise the antibody of the present invention or the above-described substance and a pharmacologically acceptable carrier, diluent or filler. Such a pharmaceutical composition is provided as a dosage form suitable for oral or parenteral administration.

As examples of the composition for parenteral administration, injections, suppositories, vaccines and the like are used; the injections may include dosage forms such as intravenous injections, subcutaneous injections, intracutaneous injections, intramuscular injections, and drip infusion injections. Such an injection can be prepared according to a commonly known method. The injection can be prepared by, for example, dissolving, suspending or emulsifying the antibody of the present invention or the above-described substance in a sterile aqueous or oily solution normally used for injections. As examples of aqueous solutions for injection, physiological saline, an isotonic solution containing glucose or other auxiliary agent and the like can be used, which may be used in combination with an appropriate solubilizer, for example, an alcohol (e.g., ethanol), a polyalcohol (e.g., propylene glycol, polyethylene glycol), a non-ionic surfactant [e.g., polysorbate 80, HCO-50 (polyoxyethylene (50mol) adduct of hydrogenated castor oil)] and the like. As examples of oily solutions, sesame oil, soybean oil and the like can be used, which may be used in combination with solubilizers benzyl benzoate, benzyl alcohol and the like. The injectable preparation prepared is preferably filled in an appropriate ampoule. A suppository used for rectal administration may also be prepared by mixing the antibody of the present invention or the above-described substance in an ordinary suppository base.

As the composition for oral administration, solid or liquid dosage forms, specifically tablets (including sugar-coated tables and film-coated tablets), pills, granules, powders, capsules (including soft capsules), syrups, emulsions, suspensions and the like can be mentioned. Such a composition is produced by a commonly known method, and may contain a carrier, diluent or filler normally used in the field of pharmaceutical making. As the carrier or filler for tablets, for example, lactose, starch, sucrose, and magnesium stearate are used.

Each of the foregoing compositions may contain another active ingredient, as long as no undesirable interaction is produced when blended with the above-described antibody or substance.

The above-described pharmaceutical composition for parenteral or oral administration is conveniently prepared in a medication unit dosage form suitable for the dosage of the active ingredient. As examples of such a medication unit dosage form, tablets, pills, capsules, injections (ampoules), and suppositories can be mentioned. As the content amount of the antibody or substance, it is preferable that normally 5 to 500 mg, particularly 5 to 100 mg for injections or 10 to 250 mg for other dosage forms, per medication unit dosage form, of the above-described antibody or substance be contained.

The dosage of the above-described agent varies also depending on the subject of administration, target disease, symptoms, route of administration and the like; for example, when the agent is used for the treatment/prevention of breast cancer in an adult, the antibody or substance of the present invention is conveniently administered by venous injection at a dose of normally about 0.01 to 20 mg/kg body weight, preferably about 0.1 to 10 mg/kg body weight, more preferably about 0.1 to 5 mg/kg body weight, about 1 to 5 times a day, preferably about 1 to 3 times a day. In the case of other parenteral administrations and oral administration, a dose based thereon can be administered. If the symptom is particularly severe, the dosage may be increased depending on the symptom.

Furthermore, the antibody of the present invention may be used in combination with other drugs, for example, alkylating agents (e.g., cyclophosphamide, ifosfamide and the like), metabolic antagonists (e.g., methotrexate, 5-fluorouracil and the like), anticancer antibiotics (e.g., mitomycin, adriamycin and the like), plant-derived anticancer agents (e.g., vincristine, vindesine, Taxol and the like), cisplatin, carboplatin, ethopoxide and the like. The antibody of the present invention or the above-described substance and the above-described drug may be administered to a patient simultaneously or at different times.

### [2] Quantitation of the receptor used in the present invention

By using the antibody of the present invention, a measurement or detection by tissue staining and the like of the receptor used in the present invention can be performed. For these purposes, the antibody molecule itself may be used, and the F(ab')2, Fab' or Fab fraction of the antibody molecule and the like may also be used.

The method for measurement using the antibody of the present invention is not to be limited particularly; any method of measurement can be used, so long as the amount of antibody, antigen or antibody-antigen complex corresponding to the amount of antigen (for example, amount of the receptor used in the present invention) in a test liquid can be detected by a chemical or physical means and can be calculated from a standard curve generated using standard solutions containing known amounts of the antigen. For example, the sandwich method, competitive method, immunometric method, nephelometry, and the like are used, and the sandwich method and competitive method described below are preferable in terms of sensitivity and specificity, and the sandwich method is particularly preferable.

### (1) Sandwich method

In the sandwich method, the antibody of the present invention insolubilized is reacted with a test liquid (primary reaction), then reacted with the antibody of the present invention labeled (secondary reaction), after which the activity of the labeling agent on the insolubilizing carrier is measured, whereby the amount of the receptor used in the present invention in the test liquid can be quantified. The primary and secondary reactions may be performed simultaneously or with a time lag. The labeling agent and the method for insolubilization can be the same as those described above. In the immunoassay by the sandwich method, the antibody used for the solid phase or the antibody for labeling is not necessarily from one kind, but a mixture of two or more kinds of antibodies may be used for increasing the measurement sensitivity and other purposes. For the antibodies used in the primary and secondary reactions, for example, when the antibody used in the secondary reaction recognizes the C-terminus of the receptor used in the present invention, the antibody used in the primary reaction is an antibody that preferably recognizes a portion other than the C-terminus, for example, the N-terminus.

### (2) Competitive method

The antibody of the present invention, a test liquid and the receptor used in the present invention labeled are competitively reacted, and the ratio of the receptor used in the present invention labeled bound to the antibody is measured, whereby the receptor used in the present invention in the test liquid is quantified.

This reaction method is performed using, for example, the solid phase immobilization method.

As a specific example, (i) the antibody of the present invention, (ii) the receptor used in the present invention labeled with HRP, and (iii) a test liquid are added to a plate wherein an anti-mouse IgG antibody (manufactured by ICN/CAPPEL Company) is present as an immobilized antibody, and they are reacted, after which the HRP activity adsorbed to the solid phase is measured, and the receptor used in the present invention is quantified.

### (3) Immunometric method

In the immunometric method, the antigen in a test liquid and a solid-phase-immobilized antigen are competitively reacted with a given amount of the antibody of the present invention labeled, after which the solid phase and the liquid phase are separated, or the antigen in the test liquid and an excess amount of the antibody of the present invention labeled are reacted, and then a solid-phase-immobilized antigen is added to bind the unreacted portion of the antibody of the present invention labeled to the solid phase, after which the solid phase and the liquid phase are separated. Next, the amount of labeling agent in either phase is measured to quantify the amount of antigen in the test liquid.

### (4) Nephelometry

Also, in nephelometry, the amount of insoluble precipitate resulting from an antigen-antibody reaction in the gel or in the solution is measured. Even when the amount of antigen in the test solution is small and only a small amount of precipitate is obtained, laser nephelometry, which utilizes laser scattering, and the like are preferably used.

As the labeling agent used for the assay methods using a labeled substance in (1) to (4) above, a radioisotope, an enzyme, a fluorescent substance, a luminescent substance, a lanthanide element and the like are used. As the radioisotope, for example, [¹²⁵I] , [¹³¹I], [³H], [¹⁴C] and the like are preferable; as the enzyme, stable enzymes with a high specific activity are preferable; for example, β-galactosidase, β-glucosidase, alkaline phosphatase, peroxidase, malate dehydrogenase and the like can be mentioned; as examples of the fluorescent substance, cyanine fluorescent dyes (e.g., Cy2, Cy3, Cy5, Cy5.5, Cy7 (manufactured by Amersham Biosciences K.K.) and the like), fluorescamine, fluorescein isothiocyanate and the like can be mentioned; as examples of the luminescent substance, luminol, luminol derivatives, luciferin, lucigenin and the like can be mentioned. Furthermore, a biotin-avidin system can also be used for the binding of the antibody and the labeling agent.

For insolubilization of the antigen or antibody, physical adsorption may be used, and chemical binding methods conventionally used to insolubilize or immobilize proteins, enzymes and the like may be used as well. As examples of the carrier, insoluble polysaccharides such as agarose, dextran, and cellulose; synthetic resins, for example, polystyrene, polyacrylamide, silicon and the like, or glass and the like can be mentioned.

In applying these individual immunological measurement methods to the method of the present invention, it is unnecessary to set special conditions, procedures and the like. Making ordinary technical considerations for those skilled in the art to the ordinary conditions and procedures in each method, a measurement system of the present invention can be constructed. For details of these general technical means, compendia, books and the like can be referred to. [For example, see edited by Hiroshi Irie, "Rajioimunoassei" (Kodansha, published in 1974), edited by Hiroshi Irie, "Zoku Rajioimunoassei" (Kodansha, published in 1979), edited by Eiji Ishikawa et al., "Kouso Meneki Sokuteihou" (Igaku-Shoin, published in 1978), edited by Eiji Ishikawa et al., "Kouso Meneki Sokuteihou" (2nd edition) (Igaku-Shoin, published in 1982), edited by Eiji Ishikawa, "Kouso Meneki Sokuteihou" (3rd edition) (Igaku-Shoin, published in 1987), "Methods in ENZYMOLOGY", Vol. 70 (Immunochemical Techniques (Part A)), ibidem, Vol. 73 (Immunochemical Techniques (Part B)), ibidem, Vol. 74 (Immunochemical Techniques (Part C)), ibidem, Vol. 84 (Immunochemical Techniques (Part D: Selected Immunoassays)), ibidem, Vol. 92 (Immunochemical Techniques (Part E: Monoclonal Antibodies and General Immunoassay Methods)), ibidem, Vol. 121 (Immunochemical Techniques (Part I: Hybridoma Technology and Monoclonal Antibodies)) (all published by Academic Press) and the like.] Therefore, when an assay system based on the sandwich immunoassay method of the present invention and the like is constructed, the method is not limited by the Examples described below.

As described above, the antibody of the present invention is capable of sensitively quantifying the receptor used in the present invention, and is therefore useful for further elucidation of the physiological functions of the receptor used in the present invention, and diagnosis of diseases involved by the receptor used in the present invention. Specifically, by measuring the amount of the receptor used in the present invention contained in a tissue or body fluid (blood, plasma, serum, urine and the like) using the antibody of the present invention, for example, cancers (e.g., breast cancer, ovarian cancer, colorectal cancer, lung cancer, pancreatic cancer and the like) and the like can be diagnosed.

### [3] Screening for medicament candidate for disease

The receptor used in the present invention exhibits upregulated expression in cancer cells such as breast cancer, ovarian cancer, colorectal cancer, lung cancer, and pancreatic cancer, and suppresses cancer cell apoptosis by being bound by a ligand such as a collagen. This cancer cell apoptosis suppression phenomenon is neutralized and apoptosis is induced by inhibiting, for example (i) the binding of a ligand such as a collagen and the receptor used in the present invention, (ii) induction of the activation of the receptor used in the present invention (e.g., induction/promotion of activity to undergo phosphorylation and the like) and the like.

Therefore, a compound that inhibits an activity of the receptor used in the present invention or a salt thereof can be used as, for example, a prophylactic/therapeutic agent for cancers (e.g., breast cancer, ovarian cancer, colorectal cancer, lung cancer, pancreatic cancer and the like), a cancer cell apoptosis inducer, a cancer cell growth suppressant and the like.

Therefore, the receptor used in the present invention is useful as a reagent for screening for a substance that inhibits an activity of the receptor of the present invention.

Accordingly, the present invention provides a screening method for a substance that inhibits an activity of the receptor used in the present invention, comprising using the receptor used in the present invention.

As specific examples of the screening method for a substance that inhibits an activity (e.g., activity to undergo phosphorylation and the like) of the receptor used in the present invention, for example, the receptor used in the present invention with a tag (e.g., FLAG, His, V5, myc, HA and the like) added to the C-terminus thereof is expressed as a recombinant type protein in animal cells, and is reacted with (i) a ligand such as a collagen (for example, type I collagen, type II collagen, type III collagen, type IV collagen, type V collagen, type VI collagen, type VIII collagen) or (ii) a ligand and a test compound, after which the cells are disrupted to prepare a cell-free extract, the extract is immunoprecipitated using an antibody against the tag, the amount of the phosphorylated receptor used in the present invention produced is quantified by a commonly known method (e.g., Western blot method and the like) using an anti-phosphorylated tyrosine antibody and the like, and is compared between the above-described cases (i) and (ii).

For example, a test compound that inhibits an activity of the receptor used in the present invention in the case (ii) above about 20% or more, preferably 30% or more, more preferably about 50% or more, compared to the case (i) above, can be selected as a compound that inhibits an activity of the receptor used in the present invention.

As the above-described cells having the capability of producing the receptor used in the present invention, for example, a host transformed with a vector comprising a DNA that encodes the receptor used in the present invention (transformant) is used. As the host, for example, animal cells such as COS7 cells, CHO cells, and HEK293 cells are preferably used. For the screening, for example, a transformant cultured by the above-described method to express the protein of the present invention on the cell membrane thereof is preferably used. The cultivation method for cells capable of expressing the protein of the present invention is the same as the foregoing cultivation method for the transformant of the present invention. As the cells having the capability of producing the receptor used in the present invention, cells of cancers highly expressing the receptor (for example, breast cancer, ovarian cancer, colorectal cancer, lung cancer, pancreatic cancer and the like) can also be used.

As examples of the test compound, peptides, proteins, antibodies, non-peptide compounds, synthetic compounds, fermentation products, cell extract, plant extract, animal tissue extract, plasma and the like can be mentioned.

Furthermore, because the gene for the receptor used in the present invention also exhibits upregulated expression in cancer tissue, a substance that inhibits the expression of the gene for the receptor used in the present invention can also be used as, for example, a prophylactic/therapeutic agent for cancers (e.g., breast cancer, ovarian cancer, colorectal cancer, lung cancer, pancreatic cancer and the like), a cancer cell apoptosis inducer, a cancer cell growth suppressant and the like.

Therefore, a polynucleotide (e.g., DNA) that encodes the receptor used in the present invention is useful as a reagent for screening for a compound that inhibits the expression of the gene for the receptor used in the present invention or a salt thereof.

As the screening method, a screening method comprising comparing (iii) a case where cells having the capability of producing the receptor used in the present invention are cultured and (iv) a case where cells having the capability of producing the receptor used in the present invention are cultured in the presence of a test compound can be mentioned.

In the above-described method, the expression level of the above-described gene (specifically, the amount of the receptor used in the present invention or the amount of mRNA that encodes the receptor used in the present invention) is measured and compared between the cases (iii) and (iv).

As the test compound and the cells having the capability of producing the protein of the present invention, the same as those described above can be mentioned.

Protein contents can be measured by a commonly known method, for example, by measuring the above-described protein in cell extract and the like according to a method such as Western blot analysis or ELISA or a method based thereon using the antibody of the present invention.

The amount of mRNA can be measured according to a commonly known method, for example, a Northern hybridization using as the probe a nucleic acid comprising the base sequence shown by SEQ ID NO:2, SEQ ID NO:4, SEQ ID NO:6, SEQ ID NO:8 or SEQ ID NO:10 or a portion thereof, or a PCR method using as the primer a nucleic acid comprising the base sequence shown by SEQ ID NO:2, SEQ ID NO:4, SEQ ID NO:6, SEQ ID NO:8 or SEQ ID NO:10 or a portion thereof, or a method based thereon.

For example, a test compound that inhibits gene expression in the case (iv) above about 20% or more, preferably 30% or more, more preferably about 50% or more, compared to the case (iii) above can be selected as a compound that inhibits the expression of the gene for the receptor used in the present invention.

The screening kit of the present invention comprises the receptor used in the present invention, or cells having the capability of producing the receptor used in the present invention and the like.

The substance obtained using the screening method or screening kit of the present invention is selected from among the above-described test compounds, for example, peptides, proteins, antibodies, non-peptide compounds, synthetic compounds, fermentation products, cell extract, plant extract, animal tissue extract, plasma and the like.

As the salt, the same as the foregoing salt of the protein of the present invention is used.

When the substance obtained using the screening method or screening kit of the present invention is used as the above-described agent, it can be prepared as a pharmaceutical preparation according to a conventional method.

As examples of the composition for oral administration or parenteral administration, the same as the composition described in [1] above can be mentioned, and the composition can be produced in the same manner and can be used in the same manner.

The sequence identification numbers in the sequence listing of the present description show the following sequences.
[SEQ ID NO:1]
   Shows the amino acid sequence of DDR1a.
[SEQ ID NO:2]
   Shows the base sequence of a DNA that encodes DDR1a having the amino acid sequence shown by SEQ ID NO:1.
[SEQ ID NO:3]
   Shows the amino acid sequence of DDR1b.
[SEQ ID NO:4]
   Shows the base sequence of a DNA that encodes DDR1b having the amino acid sequence shown by SEQ ID NO:3.
[SEQ ID NO:5]
   Shows the amino acid sequence of DDR1c
[SEQ ID NO:6]
   Shows the base sequence of a DNA that encodes DDR1c having the amino acid sequence shown by SEQ ID NO:5.
[SEQ ID NO:7]
   Shows the amino acid sequence of DDR1d.
[SEQ ID NO:8]
   Shows the base sequence of a DNA that encodes DDR1d having the amino acid sequence shown by SEQ ID NO:7.
[SEQ ID NO:9]
   Shows the amino acid sequence of DDR1e.
[SEQ ID NO:10]
   Shows the base sequence of a DNA that encodes DDR1e having the amino acid sequence shown by SEQ ID NO:9.
[SEQ ID NO:11]
   Shows the base sequence of the primer 1 used in Reference Example 4 and Reference Example 8.
[SEQ ID NO:12]
   Shows the base sequence of the primer 2 used in Reference Example 4.
[SEQ ID NO:13]
   Shows the base sequence of the primer 3 used in Reference Example 5.
[SEQ ID NO:14]
   Shows the base sequence of the primer 4 used in Reference Example 5.
[SEQ ID NO:15]
   Shows the amino acid sequence of DDR1bDN.
[SEQ ID NO:16]
   Shows the base sequence of a DNA that encodes DDR1bDN having the amino acid sequence shown by SEQ ID NO:15.
[SEQ ID NO:17]
   Shows the base sequence of the primer 5 used in Reference Example 1.
[SEQ ID NO:18]
   Shows the base sequence of the primer 6 used in Reference Example 1.
[SEQ ID NO:19]
   Shows the base sequence of the TaqMan probe 1 used in Reference Example 1.
[SEQ ID NO:20]
   Shows the base sequence of the primer 7 used in Reference Example 2 and Reference Example 3.
[SEQ ID NO:21]
   Shows the base sequence of the primer 8 used in Reference Example 2 and Reference Example 3.
[SEQ ID NO:22]
   Shows the base sequence of the TaqMan probe 2 used in Reference Example 2 and Reference Example 3.
[SEQ ID NO:23]
   Shows the base sequence of the primer 9 used in Reference Example 8.
[SEQ ID NO:24]
   Shows the amino acid sequence of DDR1bED-Fc.
[SEQ ID NO:25]
   Shows the base sequence of a DNA that encodes DDR1bED-Fc having the amino acid sequence shown by SEQ ID NO:24.
[SEQ ID NO:26]
   Shows the amino acid sequence of DDR1bED-Flag.
[SEQ ID NO:27]
   Shows the base sequence of a DNA that encodes DDR1bED-Flag having the amino acid sequence shown by SEQ ID NO:26.
[SEQ ID NO:28]
   Shows the base sequence of the primer 10 used in Reference Example 8.
[SEQ ID NO:29]
   Shows the base sequence of the primer 11 used in Reference Example 8.

Abbreviations for bases, amino acids and the like used in the present description are based on abbreviations specified by the IUPAC-IUB Commission on Biochemical Nomenclature or abbreviations in common use in relevant fields, some examples of which are given below. When an optical isomer may be present in amino acid, it is of the L-configuration, unless otherwise stated.
DNA: Deoxyribonucleic acid
cDNA: Complementary deoxyribonucleic acid
A: Adenine
T: Thymine
G: Guanine
C: Cytosine
RNA: Ribonucleic acid
mRNA: Messenger ribonucleic acid
dATP: Deoxyadenosine triphosphate
dTTP: Deoxythymidine triphosphate
dGTP: Deoxyguanosine triphosphate
dCTP: Deoxycytidine triphosphate
ATP: Adenosine triphosphate
EDTA: Ethylenediaminetetraacetic acid
SDS: Sodium dodecyl sulfate
Gly: Glycine
Ala: Alanine
Val: Valine
Leu: Leucine
Ile: Isoleucine
Ser: Serine
Thr: Threonine
Cys: Cysteine
Met: Methionine
Glu: Glutamic acid
Asp: Aspartic acid
Lys: Lysine
Arg: Arginine
His: Histidine
Phe: Phenylalanine
Tyr: Tyrosine
Trp: Tryptophan
Pro: Proline
Asn: Asparagine
Gln: Glutamine
pGlu: Pyroglutamic acid
Sec: Selenocysteine

Substituents, protecting groups and reagents frequently mentioned herein are represented by the symbols shown below. Me: Methyl group
Et: Ethyl group
Bu: Butyl group
Ph: Phenyl group
TC: Thiazolidine-4(R)-carboxamide group
Tos: p-Toluenesulfonyl
CHO: Formyl
Bzl: Benzyl
Cl₂-Bzl: 2, 6-Dichlorobenzyl
Bom: Benzyloxymethyl
Z: Benzyloxycarbonyl
Cl-Z: 2-Chlorobenzyloxycarbonyl
Br-Z: 2-Bromobenzyloxycarbonyl
Boc: t-Butoxycarbonyl
DNP: Dinitrophenyl
Trt: Trityl
Bum: t-Butoxymethyl
Fmoc: N-9-Fluorenylmethoxycarbonyl
HOBt: 1-Hydroxybenztriazole
HOOBt: 3,4-Dihydro-3-hydroxy-4-oxo-1,2,3-benzotriazine
HONB:1-Hydroxy-5-norbornene-2,3-dicarboximide
DCC: N, N'-Dicyclohexylcarbodiimide

### Examples

The present invention is hereinafter described in more detail by means of the following Examples, by which, however, the invention is not limited by any means.

### [Reference Example 1] Investigation of upregulation of mRNA expression of the DDR1 gene in human cancer tissues

In this Reference Example, the DDR1a gene, the DDR1b gene, and the DDR1c gene are generically defined as the DDR1 gene. Whether or not the mRNA expression of the DDR1 gene is upregulated in cancer tissues was investigated by a quantitative PCR method. For measuring expression levels, cDNA CeHAT-SD Breast Tumor 1 (Cosmo Bio Co., Ltd.), cDNA CeHAT-SD Breast Tumor 2 (Cosmo Bio Co., Ltd.), Human Colon Matched cDNA Pair Panel (CLONTECH Company), Human Lung Matched cDNA Pair Panel (CLONTECH Company), and Human Ovary Matched cDNA Pair Panel (CLONTECH Company) were used. With 1 µL of cDNA as the template, 7.5 µL of TaqMan Universal PCR Master Mix (Applied Biosystems Company), 500 nM of each of primer 5 (SEQ ID NO:17) and primer 6 (SEQ ID NO:18), and 100 nM of FAM-labeled TaqMan probe 1 (SEQ ID NO:19) were added to make a reaction liquid volume of 15 µL. Note that for cDNA CeHAT-SD Breast Tumor 1 (Cosmo Bio Co., Ltd.) and cDNA CeHAT-SD Breast Tumor 2 (Cosmo Bio Co., Ltd.), the amount of template was 0.2 µL. The PCR reaction was performed by a treatment at 50°C for 2 minutes and at 95°C for 10 minutes, followed by a cycle of a treatment at 95°C for 15 seconds and at 60°C for 1 minute repeated 40 times. On the other hand, the β-actin gene expression level contained in the same amount of the template cDNA was measured and this was used as the internal standard. As a result, the DDR1 gene expression level in cancer tissues compared to normal tissue increased 2 fold, 5.1 fold, and 2.5 fold, respectively, in 3 donors in the cDNA CeHAT-SD Breast Tumor 1 (Cosmo Bio Co., Ltd.), and 6.9 fold, 1.1 fold, and 4.3 fold, respectively, in 3 other donors in the cDNA CeHAT-SD Breast Tumor 2 (Cosmo Bio Co., Ltd.). Likewise, the expression level increased 6.9 fold, 2.2 fold, 1.3 fold, and 1.7 fold, respectively, in 4 of the 5 donors in the Human Colon Matched cDNA Pair Panel (CLONTECH Company), 6.5 fold, 4.9 fold, 4.6 fold, and 10.3 fold, respectively, in 4 of the 5 donors in the Human Lung Matched cDNA Pair Panel (CLONTECH Company), and 5.4 fold, 1.8 fold, and 1.4 fold, respectively, in 3 of the 5 donors in the Human Ovary Matched cDNA Pair Panel (CLONTECH Company). Confirmed from these results was upregulation of the expression of the DDR1 gene in cancer tissues.

### [Reference Example 2] Investigation of upregulation of mRNA expression of the DDR1b gene in cancer tissues

With Matched Tumor/Normal cDNA Pair (CLONTECH Company) derived from human cancer tissues (breast cancer, lung cancer, rectal cancer, ovarian cancer) as the template, a quantitative PCR reaction using the FAM-labeled TaqMan probe was performed, whereby expression levels of the DDR1b gene in cancer tissues and normal tissue were measured.

For the composition of the reaction liquid in the reaction, 1 µL of the above-described cDNA, 10 µL of TaqMan Universal PCR Master Mix (Applied Biosystems Company), 200 nM of each of primer 7 (SEQ ID NO:20) and primer 8 (SEQ ID NO:21), and 200 nM of TaqMan probe 2 (SEQ ID NO:22) were added to make a liquid volume of 20 µl. The PCR reaction was performed by a treatment at 50°C for 2 minutes and at 95°C for 10 minutes, followed by a cycle of a treatment at 95°C for 15 seconds and at 60°C for 1 minute repeated 40 cycles.

As a result, the DDR1b gene expression level in cancer tissues compared to surrounding normal tissues increased about 3 fold and about 5 fold, respectively, in 2 of 6 cases of human breast cancer, about 7 fold, about 5 fold, about 6 fold and about 16 fold, respectively, in 4 of 5 cases of human lung cancer, about 5 fold in 1 of 5 cases of human rectal cancer, and about 5 fold and about 2 fold, respectively, in 2 of 5 cases of human ovarian cancer.

### [Reference Example 3] Quantitation of mRNA of the DDR1b gene in human cancer cell lines

For use in the following, the osteosarcoma cell line Saos-2, the brain tumor cell lines SK-N-MC, SK-N-AS, SK-N-BE, SK-N-DZ, SK-N-FI, SK-N-SH, D341 Med, Daoy, DBTRG-05MG, U-118 MG, U-87 MG, CCF-STTG1, and SW 1088, the breast cancer cell lines HCC1937, ZR-75-1, AU565, MCF-7, MDA-MB-231, SKBR-3, BT474, and MDA-MB-435s, the colorectal cancer cell lines Caco-2, COLO 201, COLO 205, COLO 320DM, DLD-1, HCT-15, HCT-8, HT-29, LoVo, LS180, LS123, LS174T, NCI-H548, SNU-C1, SK-CO-1, SW 403, SW 48, SW 480, SW 620, SW 837, SW 948, and HCT 116, the small-cell lung cancer cell lines NCI-H187, NCI-H378, NCI-H526, NCI-H889, NCI-H1672, NCI-H1836, NCI-H2227, NCI-N417, and SHP-77, the non-small-cell lung cancer cell lines A549, NCI-H23, NCI-H226, NCI-H358, NCI-H460, NCI-H522, NCI-H661, NCI-H810, NCI-H1155, NCI-H1299, NCI-H1395, NCI-H1417, NCI-H1435, NCI-H1581, NCI-H1651, NCI-H1703, NCI-H1793, NCI-H1963, NCI-H2073, NCI-H2085, NCI-H2106, NCI-H2228, NCI-H2342, and NCI-H2347, the ovarian cancer cell lines ES-2, Caov-3, MDAH2774, NIH:OVCAR3, OV-90, SK-OV-3, TOV-112D, and TOV-21G, the prostatic cancer cell lines DU 145 and LNCaP, the retinoblastoma cell lines WERI-Rb-1 and Y79, and the testicular cancer cell line Cates-1B were purchased from the American Type Culture Collection (ATCC). The colorectal cancer cell line COCM1, the non-small-cell lung cancer cell line VMRC-LCD and the prostatic cancer cell line PC3 were purchased from the Japanese Collection of Research Bioresources (JCRB). Each of the above-described cancer cell lines was cultured according to the cultivation method recommended by ATCC or JCRB, and a total RNA was prepared using RNeasy Mini Total RNA Kit (QIAGEN Company). With this total RNA as the template, a cDNA was prepared by a reverse transcription reaction using a random primer, and a quantitative PCR reaction was performed, whereby the DDR1b gene expression level was quantified.

The reaction was performed with the cDNA from 5 ng of the above-described total RNA as the template according to the method described in Reference Example 2. Concurrently, the copy number of the β-actin gene contained in 1 ng of the above-described total RNA was calculated, and this was used as the internal standard.

Relative expression levels obtained by standardizing the above-described expression levels for all of the above-described genes by the β-actin gene expression level are shown in [Table 1]. 16 cancer cell lines exhibited an expression level of the β-actin gene increased by 10% or more, demonstrating the presence of cell lines highly expressing the DDR1b gene.

**Table 1**

| cell line | Expression (% of beta-actin) | cell line | Expression (% of beta-actin) | cell line | Expression (% of beta-actin) |
|---|---|---|---|---|---|
| Saos-2 | 1.2 | LS 180 | 3.9 | NCI-H1155 | 0.4 |
| CCF-STTG1 | 4.8 | LSI23 | 3.6 | NCI-H1299 | 1.2 |
| SW1088 | 1.1 | LSI74T | 4.9 | NCI-H1581 | 1.0 |
| DBTRG-05MG | 1.8 | NCI-H548 | 3.0 | NCI-H2106 | 3.4 |
| U-118 MG | 1.5 | NCI-SNU-C1 | 11.6 | NCI-H187 | 7.0 |
| U-87 MG | 0.6 | SK-CO-1 | 20.5 | NCI-H378 | 1.0 |
| D341 Med | 0.2 | SW 403 | 16.2 | NCI-H526 | 3.1 |
| Daoy | 0.5 | SW 48 | 10.8 | NCI-H889 | 1.0 |
| SK-N-AS | 0.7 | SW 480 | 5.0 | NCI-H1417 | 2.8 |
| SK-N-BE | 0.7 | SW 620 | 2.6 | NCI-H1672 | 4.3 |
| SK-N-DZ | 0.6 | SW 837 | 15.2 | NCI-H1836 | 2.0 |
| SK-N-FI | 2.2 | SW 948 | 10.3 | NCI-H1963 | 6.5 |
| SK-N-SH | 1.0 | COCM1 | 12.1 | NCI-H2227 | 2.8 |
| SK-N-MC | 1.5 | HCT 116 | 11.2 | NCI-H417 | 3.1 |
| AU565 | 10.3 | A549 | 0.7 | SHP-77 | 2.7 |
| MCF-7 | 3.2 | NCI-H23 | 0.5 | NCI-H226 | 1.5 |
| MDA-MB-231 | 2.9 | NCI-H358 | 2.4 | NCI-H1703 | 1.4 |
| SKBR-3 | 10.5 | NCI-H522 | 1.6 | Caov-3 | 2.9 |
| BT474 | 4.1 | NCI-H1395 | 20.9 | MDAH2774 | 4.6 |
| HCC1937 | 6.1 | NCI-H1435 | 8.4 | OVCAR3 | 6.5 |
| MDA-MB-435s | 1.4 | NCI-H1651 | 0.4 | OV-90 | 1.8 |
| ZR-75-1 | 28.1 | NCl-H1793 | 1.6 | SK-OV-3 | 2.1 |
| Caco-2 | 2.7 | NCI-H2073 | 1.2 | TOV-112D | 0.9 |
| COLO 201 | 12.3 | NCI-H2085 | 4.1 | TOV-21 G | 2.0 |
| COLO 205 | 5.4 | NCI-H2228 | 2.2 | ES-2 | 0.2 |
| COLO 320DM | 0.8 | NCI-H2342 | 9.4 | DU 145 | 5.6 |
| DLD-1 | 11.8 | NCI-H2347 | 3.0 | LNCaP | 4.9 |
| HCT-15 | 5.3 | VMRC-LCD | 16.5 | PC3 | 1.9 |
| HCT-8 | 8.5 | NCI-H460 | 1.6 | Y79 | 0.5 |
| HT-29 | 4.8 | NCI-H661 | 1.0 | WERI-Rb-1 | 3.7 |
| LoVo | 10.3 | NCI-H810 | 3.4 | Cates-1 B | 0.6 |

### [Reference Example 4] Construction of animal cell expression vectors for recombinant type full-length DDR1a and DDR1b proteins

A PCR reaction was performed with Marathon-Ready cDNA derived from human breast cancer cell GI-101 (CLONTECH Company) as the template, using primer 1 with a sequence recognized by the restriction endonuclease BamHI added thereto (SEQ ID NO:11), and primer 2 with a sequence recognized by the restriction endonuclease EcoRI added thereto (SEQ ID NO:12). For the composition of the reaction liquid in the reaction, using 2 µL of the above-described cDNA as the template, 2.5 U of PfuUltra Hotstart DNA Polymerase (STRATAGENE Company), 0.2 µM of each of primer 1 (SEQ ID NO:11) and primer 2 (SEQ ID NO:12), 200 µM of dNTPs, and 5 µL of 10x Pfu Ultra Buffer (STRATAGENE Company) were added to make a liquid volume of 50 µL. The PCR reaction was performed by a treatment at 95°C for 2 minutes, followed by a cycle of a treatment at 95°C for 30 seconds, at 55°C for 30 seconds, and at 72°C for 3 minutes repeated 40 times, followed by a reaction at 72°C for 10 minutes. Next, after purification using PCR Purification Kit (QIAGEN Company), the PCR reaction product was treated with the restriction endonucleases BamHI and EcoRI. pcDNA3.1(+) (Invitrogen Company) was also treated with the restriction endonucleases BamHI and EcoRI. These were purified using PCR Purification Kit (QIAGEN Company), and each DNA fragment was subjected to a ligation reaction using Ligation High (TOYOBO Company), after which it was introduced to Escherichia coli TOP10 (Invitrogen Company), and cells were selected in an LB agar medium comprising ampicillin. As a result of an analysis of the sequence of each clone, the animal cell expression vector pcDNA3.1(+)-DDR1a, having a cDNA sequence that encodes the DDR1a protein (SEQ ID NO:1) (SEQ ID NO:2), and the animal cell expression vector pcDNA3.1(+)-DDR1b, having a cDNA sequence that encodes the DDR1b protein (SEQ ID NO:3) (SEQ ID NO:4), were obtained.

### [Reference Example 5] Construction of animal cell expression vector for recombinant type dominant negative type DDR1b protein

An animal cell expression vector for a dominant negative type DDR1b protein not having kinase activity (hereinafter referred to as DDR1bDN) was constructed. A DNA fragment comprising the sequence of the 2710th to 2739th amino acids of the DDR1b full-length cDNA sequence (SEQ ID NO:4) and a sequence derived from pcDNA3.1(+) was amplified by a PCR reaction with pcDNA3.1(+)-DDR1b as the template using primer 3 with a sequence recognized by the restriction endonuclease XhoI added thereto (SEQ ID NO:13) and primer 4 that hybridizes to pcDNA3.1(+) (SEQ ID NO:14). For the composition of the reaction liquid in the reaction, 10 ng of the above-described pcDNA3.1(+)-DDR1b, 2.5 U of PfuUltra Hotstart DNA Polymerase (STRATAGENE Company), 0.2 µM of each of primer 3 (SEQ ID NO:13) and primer 4 (SEQ ID NO:14), 200 µM of dNTPs, and 5 µL of 10x Pfu Ultra Buffer (STRATAGENE Company) were added to make a liquid volume of 50 µL. The PCR reaction was performed by a treatment at 95°C for 2 minutes, followed by a cycle of a treatment at 95°C for 30 seconds, at 60°C for 30 seconds, and at 72°C for 30 seconds repeated 30 times, followed by a reaction at 72°C for 10 minutes. Next, after purification using PCR Purification Kit (QIAGEN Company), the PCR reaction product was treated with the restriction endonuclease XhoI. pcDNA3.1(+)-DDR1b was also treated with the restriction endonuclease XhoI, and about 7 kb DNA fragment was recovered. These were purified using Wizard SV Gel and PCR Clean-Up System (Promega Company). After a ligation reaction using Ligation High (TOYOBO Company), each DNA fragment was introduced to Escherichia coli TOP10 (Invitrogen Company), and cells were selected in an LB agar medium comprising ampicillin. As a result of an analysis of the sequence of each clone, the animal cell expression vector pcDNA3.1(+)-DDR1bDN, having a cDNA sequence that encodes the DDR1bDN protein (SEQ ID NO:15) (SEQ ID NO:16) was obtained.

### [Reference Example 6] Establishment of cell line that stably expresses recombinant type full-length DDR1b protein

A cell line that constitutively expresses the DDR1b protein (SEQ ID NO:3) was established using the human colorectal cancer cell line HCT116 described in Reference Example 3. 1.2x10⁶ cells of HCT116 were suspended in 10 ml of Dulbecco's modified Eagle' s Minimal Essential Medium (Sigma Company) comprising 10% fetal bovine serum (JRH Company) and 50 µg/ml penicillin-streptomycin (Invitrogen Company), and sown to a 10 cm Petri dish, after which they were cultured in a 5% gaseous carbon dioxide stream at 37°C overnight. A mixture of 18 µl of the FuGENE6 transfection reagent (Roche Diagnostics Company), 6 µg of plasmid pcDNA3.1(+)-DDR1b and 600 µl of OPTI-MEM I (Invitrogen Company), previously allowed to stand at room temperature for 20 minutes, was added, and the cultivation was continued. Two days later, the medium was exchanged with the above-described medium comprising 1 mg/ml G418 (Promega Company) (G418 selection medium). The cultivation was continued with the G418 selection medium, and the cells were twice subcultured using trypsin-EDTA (Invitrogen Company), after which they were sown to a 96-well plate at 1 cell per well, and the cultivation was continued with the G418 selection medium. 14 days later, the cells were recovered from the wells showing colonization, and sown to a 24-well plate. Still 5 days later, the cells were sown to a 6-well plate, and the cultivation was continued with the G418 selection medium, after which the cells were suspended in 200 µL of a sample buffer for SDS-PAGE (Bio-Rad Company) comprising 1% 2-mercaptoethanol. After heat treatment at 100°C for 3 minutes, 20 µl was subjected to SDS-PAGE on 10% acrylamide gel. Western blotting was performed using an anti-DDR1 antibody (Santa Cruz Company), whereby the cell line DDR1bFL-#117 that expresses the DDR1b protein was obtained.

### [Reference Example 7] Establishment of cell lines that stably express recombinant type DDR1bDN protein

A cell line that constitutively expresses the DDR1bDN protein (SEQ ID NO:15) was established according to the method described in Reference Example 6 with the plasmid used for transfection replaced with pcDNA3.1(+) -DDR1bDN. As a result, the cell lines DDR1bDN-#206 and DDR1bDN-#218 that express the DDR1bDN protein were obtained.

### [Reference Example 8] Construction of animal cell expression vector for recombinant type DDR1b extracellular region protein - 1

### (1) Cloning of human IgG1-Fc fragment

A PCR reaction was performed with human spleen-derived Marathon-Ready cDNA (CLONTECH Company) as the template, using primer 10 with a sequence recognized by the restriction endonuclease EcoRI added thereto (SEQ ID NO:28) and primer 11 with a sequence recognized by the restriction endonuclease XhoI added thereto (SEQ ID NO:29). For the composition of the reaction liquid in the reaction, 1 µL of the above-described cDNA, 1 U of PfuTurbo Hotstart DNA Polymerase (STRATAGENE Company), 1 µM of each of primer 10 (SEQ ID NO:28) and primer 11 (SEQ ID NO:29), 200 µM of dNTPs, and 10 µL of 2xGC Buffer I (TaKaRa Bio Company) were added to make a liquid volume of 20 µL. The PCR reaction was performed by a treatment at 95°C for 1 minute, followed by a cycle of a treatment at 95°C for 20 seconds, at 60°C for 15 seconds, and at 72°C for 2 minutes repeated 30 times. Next, the PCR reaction product was purified using PCR Purification Kit (QIAGEN Company) and treated with the restriction endonucleases EcoRI and XhoI. pcDNA3.1(+) (Invitrogen Company) was also treated with the restriction endonucleases EcoRI and XhoI. These were purified using PCR Purification Kit (QIAGEN Company), and each DNA fragment was subjected to a ligation reaction using DNA Ligation Kit ver.2 (TaKaRa Bio Company), after which the ligation product was introduced to Escherichia coli TOP10 (Invitrogen Company), and cells were selected in an LB agar medium comprising ampicillin. As a result of an analysis of the sequence of each clone, the animal cell expression vector pcDNA3.1(+)-IgG1Fc having a cDNA sequence that encodes the Fc region of human IgG1 was obtained.

### (2) Construction of DDR1b extracellular region - Fc chimeric protein expression vector

with pcDNA3.1 (+)-DDR1b as the template, using primer 1 with a sequence recognized by the restriction endonuclease BamHI added thereto (SEQ ID NO:11) and primer 9 with a sequence recognized by the restriction endonuclease EcoRI added thereto (SEQ ID NO:23), a cDNA that encodes the extracellular region of DDR1b was amplified. For the composition of the reaction liquid in the reaction, 10 ng of the above-described pcDNA3.1(+)-DDR1b, 2.5 U of PfuUltra Hotstart DNA Polymerase (STRATAGENE Company), 0.2 µM of each of primer 1 (SEQ ID NO:11) and primer 9 (SEQ ID NO:23), 200 µM of dNTPs, and 5 µL of 10x Pfu Ultra Buffer (STRATAGENE Company) were added to make a liquid volume of 50 µL. The PCR reaction was performed by a treatment at 95°C for 2 minutes, followed by a cycle of a treatment at 95°C for 30 seconds, at 60°C for 30 seconds, and at 72°C for 1 minute 15 seconds repeated 30 times, followed by a reaction at 72°C for 10 minutes. Next, after purification using PCR Purification Kit (QIAGEN Company), the PCR reaction product was treated with the restriction endonucleases BamHI and EcoRI. The pcDNA3.1(+)-IgG1Fc acquired in the previous term (1) was treated in the same manner with the restriction endonucleases BamHI and EcoRI. Each DNA fragment was purified using Wizard SV Gel and PCR Clean-Up System (Promega Company), and a ligation reaction was performed using Ligation High (TOYOBO Company), after which the ligation product was introduced to Escherichia coli TOP10 (Invitrogen Company), and cells were selected in an LB agar medium comprising ampicillin. As a result of an analysis of the sequence of each clone, the animal cell expression vector pcDNA3.1(+)-DDR1bED-Fc having a cDNA sequence that encodes the protein as the fusion of the DDR1b extracellular region and the Fc region of IgG1 (SEQ ID NO:24) (SEQ ID NO:25) was obtained.

### [Reference Example 9] Construction of animal cell expression vector for recombinant type DDR1b extracellular region protein - 2

The pcDNA3.1(+)-DDR1bED-Fc prepared in Reference Example 8-(2) and pCMV-Tag4 (STRATAGENE Company) were treated with the restriction endonucleases EcoRI and BamHI, and each DNA fragment was purified using Wizard SV Gel and PCR Clean-Up System (Promega Company), after which a ligation reaction was performed using Ligation High (TOYOBO Company). The plasmid obtained was introduced to Escherichia coli TOP10 (Invitrogen Company), and cells were selected in an LB agar medium comprising kanamycin. As a result of an analysis of the sequence of each clone, the animal cell expression vector pCMV-Tag4-DDR1bED-Flag having a cDNA sequence that encodes a protein fused with a Flag tag at the C terminus of the extracellular region of DDR1b (SEQ ID NO:26) (SEQ ID NO:27) was obtained. [Reference Example 10] Investigation of apoptosis susceptibilities of DDR1bFL-#117 and DDR1bDN-#206 and #218 - 1

The apoptosis susceptibilities of DDR1bFL-#117 prepared in Reference Example 6 and DDR1bDN-#206 and DDR1bDN-#218 prepared in Reference Example 7 were compared with the susceptibility of the parent strain HCT116 cells.

Each of HCT116 cells, DDR1bFL-#117, and DDR1bDN-#206, DDR1bDN-#218 was suspended in 1.5 ml of Dulbecco's modified Eagle' s Minimal Essential Medium (Sigma Company) comprising 10% fetal bovine serum (JRH Company) and 50 µg/ml penicillin-streptomycin (Invitrogen Company) to obtain a cell density of 5x10⁵ cells, and each suspension was sown to a 6-well plate, after which the cells were cultured in a 5% gaseous carbon dioxide stream at 37°C. On the following day, the medium was exchanged with the above-described medium comprising 0.5 µg/ml of doxorubicin hydrochloride (Wako Company). After 36 hours, each cell type was recovered using trypsin-EDTA (Invitrogen Company), and twice washed with PBS (Invitrogen Company). Furthermore, Annexin V-FITC (BECKMAN COULTER Company) was added, and the mixture was allowed to stand at 4°C for 15 minutes, after which the fluorescence intensity of Annexin V-FITC bound to the cells was analyzed using FACScan (BD Bioscience Company). In the HCT116 cells, Annexin V-FITC-bound cells accounted for about 36%, whereas in DDR1bFL-#117, the same cells accounted for about 27%, and in DDR1bDN-#206 and DDR1bDN-#218, the same cells accounted for about 70% and about 66%, respectively. From this, it was found that with the increase in the expression or activity of DDR1b, the cells became resistant to doxorubicin-induced apoptosis.

### [Reference Example 11] Apoptosis susceptibilities of DDR1bDN expression cells #206 and #218 - 2

Each of HCT116 cells and DDR1bDN-#206 and #218 was suspended in 1.5 ml of Dulbecco's modified Eagle' s Minimal Essential Medium (Sigma Company) comprising 50 µg/ml penicillin-streptomycin (Invitrogen Company) to obtain a cell density of 1.5x10⁶ cells, and sown to a 6-well plate. After cultivation in a 5% gaseous carbon dioxide stream at 37°C for 48 hours, each cell type was recovered using trypsin-EDTA (Invitrogen Company), and twice washed with PBS (Invitrogen Company). Furthermore, Annexin V-FITC (BECKMAN COULTER Company) was added, and the plate was allowed to stand at 4°C for 15 minutes, after which the fluorescence intensity of Annexin V-FITC bound to the cells was analyzed using FACScan (BD Bioscience Company). In the parent strain HCT116 cells, Annexin V-FITC-bound cells accounted for about 52%, whereas in DDR1bDN-#206 and DDR1bDN-#218, the same cells accounted for about 77% and about 73%, respectively. From this, it was found that apoptosis induction was promoted by suppressing the DDR1b function.

### [Example 1] Preparation and purification of anti-human DDR1b rabbit polyclonal antibody using DNA immunization method

Preparation of a rabbit polyclonal antibody against human DDR1b was outsourced to Genovac Company, which has a technology for antibody preparation by the DNA immunization method. For the immunization, a cDNA that encodes the 22nd to 416th amino acid sequence of human DDR1b (SEQ ID NO:3) was used; according to the method described in a patent document for an application by Genovac Company (WO 00/29442), two rabbits were immunized with the antigen. About 150 mL of antiserum (rAS1) was obtained from the rabbit of ID No.1 and 120 mL of antiserum (rAS2) was obtained from the rabbit of ID No.2.

Next, from each antiserum, an IgG fraction was prepared. First, 1.25 mL of 0.2 M sodium phosphate buffer solution (pH 7.0) was added to 12.5 mL of the above-described rAS1 or rAS2, and while gently stirring, 13.75 mL of ammonium sulfate saturated aqueous solution was added dropwise. After stirring in ice for 1 hour, the mixture was centrifuged at 10,600 x g for 30 minutes, and the precipitated fraction was recovered. The fraction was suspended in 10 mL of 50% ammonium sulfate aqueous solution, the suspension was again centrifuged at 10,600 x g for 20 minutes, and the precipitated fraction was recovered. After 5 mL of 20 mM sodium phosphate buffer solution (pH 7.0) was added to dissolve the precipitate, the solution was dialyzed against 5 L of sodium phosphate buffer solution (pH 7.0) at 4°C overnight to perform desalination operation. After the dialysate was filtered through Millex-HV filter having a pore size of 0.45 µm (Millipore Company), IgG was adsorbed to HiTrap rProtein A FF (column volume: 5 mL, Amersham Bioscience Company), previously equilibrated with 25 mL of 20 mM sodium phosphate buffer solution (pH 7.0). After the unadsorbed fraction was washed off with 25 mL of 20 mM sodium phosphate buffer solution (pH 7.0), elution was performed with 25 mL of 0.1 M citrate buffer solution (pH 3.0). After the elution, the eluate was immediately neutralized with a 1/10 volume of 1 M Tris-HCl buffer solution (pH 9.0), and a 0.5 µL aliquot was subjected to SDS-PAGE using 5-20% acrylamide-gradient gel. The protein was stained using BioSafe Coomassie (Bio-Rad Company), and an IgG-containing fraction was harvested, after which dialysis against 2 L of 20 mM sodium phosphate buffer solution (pH 7.0) was twice performed at 4°C. The dialysate was concentrated using VIVA SPIN 6 having 30,000 molecular weight cutoff (SARTORIUS K.K.), and adjusted to a final volume of 1 mL. Absorbance at 280 nm was measured, the concentration was calculated on assumption of a molecular absorption coefficient of 2.24 x 10⁵ M⁻¹ for a light path length of 1 cm, and the amount of IgG recovered was calculated; it was found that 30 mg of IgG was recovered from rAS1, and 72 mg from rAS2.

### [Example 2] Apoptosis induction activity of anti-human DDR1b rabbit polyclonal antibody (1)

### (1) Apoptosis induction by serum removal and cell protecting action of type IV collagen

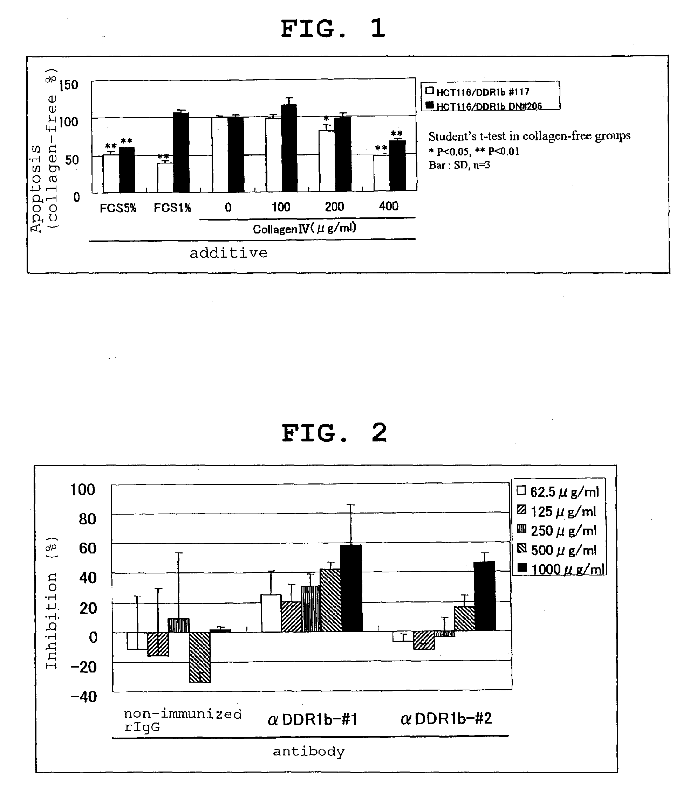

DDR1FL-#117 described in Reference Example 6 and DDR1bDN-#206 described in Reference Example 7 were dispersed by trypsin-EDTA (Invitrogen Company) treatment, and once washed with 10 mL of PBS. Furthermore, using Dulbecco's modified Eagle's Minimal Essential Medium (Invitrogen Company) comprising 50 µg/ml gentamycin (Invitrogen Company) (hereinafter referred to as serum-free DMEM), cells were suspended to obtain a cell density of 1x10⁴ cells/50 µL, and aliquots(50 µL) were dispensed to each well of a flat-based 96-well plate in which 50 µL of a serum-free DMEM comprising 800 µg/mL of type IV collagen (Sigma Company) was placed in advance. For negative control, cells were also dispensed to wells containing a serum-free DMEM not containing type IV collagen. After the cells were cultured in a 5% gaseous carbon dioxide stream at 37°C for 3 days, the apoptosis induced in each cell type was detected using Cell Death Detection ELISA^{PLUS} (Roche Diagnostics Company); in DDR1FL-#117, apoptosis was suppressed by about 53% by the addition of type IV collagen, whereas in DDR1bDN-#206, apoptosis was suppressed only by about 34% (Figure 1). That is, it was found that the apoptosis induced by serum removal was partially rescued by the addition of type IV collagen, and that the rescue was mediated by DDR1b.

### (2) Apoptosis induction activity of anti-human DDR1b rabbit polyclonal antibody

Whether or not the rabbit polyclonal antibody described in Example 1 suppresses the cell protecting action of type IV collagen was determined. DDR1FL-#117 described in the previous term (1) was dispersed in the Cell dissociation buffer (Invitrogen Company) and once washed with 10 mL of PBS, after which it was suspended in a serum-free DMEM comprising 800 µg/mL of type IV collagen (Sigma Company) to obtain a cell density of 2x10⁴ cells/50 µL. 50 µL of the above-described cell suspension was dispensed to a flat-based 96-well plate, to which non-immunized rabbit IgG (Jackson Immunoresearch Company) or the rabbit polyclonal antibody described in Example 1, diluted to various concentrations with serum-free DMEM, was added at 50 µL per well in advance. For comparison, cells were also dispensed to wells containing the serum-free DMEM alone in the same manner. After the cells were cultured in a 5% gaseous carbon dioxide stream at 37°C for 3 days, the apoptosis induced in each cell type was detected using Cell Death Detection ELISA^{PLUS} (Roche Diagnostics Company). The cell protection inhibitory activity with the addition of each polyclonal antibody at 1 mg/mL was calculated relative to the absorbance change induced by the addition of type IV collagen (apoptosis index) as 100%; because inhibitory activity values of 1.7% for non-immunized rabbit IgG, 58.6% for IgG purified from rAS1, and 46.3% for IgG purified from rAS2 were obtained (Figure 2), it was shown that the anti-DDR1b rabbit polyclonal antibody inhibited the binding of type IV collagen and DDR1b, and nullified the cell protecting action of type IV collagen.

### [Example 3] Apoptosis induction activity of anti-human DDR1b rabbit polyclonal antibody (2)

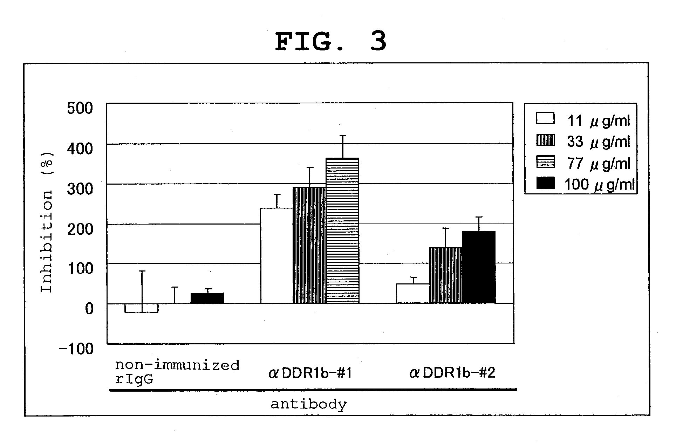

Whether or not the rabbit polyclonal antibody described in Example 1 suppresses the cell protecting action of type IV collagen was determined. The cancer cell line HCT116, wherein DDR1b was not forcibly expressed, was dispersed in the Cell dissociation buffer (Invitrogen Company) and once washed with 10 mL of PBS, after which it was suspended in a serum-free DMEM comprising 100 µg/mL of type IV collagen (Sigma Company) to obtain a cell density of 2x10⁴ cells/50 µL. 50 µL of the above-described cell suspension was dispensed to a flat-based 96-well plate, to which non-immunized rabbit IgG (Jackson Immunoresearch Company) or the rabbit polyclonal antibody described in Example 1, diluted to various concentrations with serum-free DMEM, was added at 50 µL per well in advance. For comparison, cells were also dispensed to wells containing the serum-free DMEM alone in the same manner. After the cells were cultured in a 5% gaseous carbon dioxide stream at 37°C for 3 days, the apoptosis induced in each cell type was detected using Caspase-Glo^{™} 3/7 Assay (Promega Company). The inhibitory activity with the addition of each polyclonal antibody at 33 µg/mL was calculated relative to the absorbance change induced by the addition of type IV collagen (apoptosis index) as 100%; because cell protection inhibitory activity values of 0% for non-immunized rabbit IgG, 290% for IgG purified from rAS1, and 139% for IgG purified from rAS2 were obtained (Figure 3), it was shown that the anti-DDR1b rabbit polyclonal antibody inhibited the binding of type IV collagen and DDR1b, nullified the cell protecting action of type IV collagen, and induced apoptosis in the cancer cell line HCT116.

### Industrial Applicability

The neutralizing antibody of the present invention is useful as, for example, a prophylactic/therapeutic agent for cancers (e.g., breast cancer, ovarian cancer, colorectal cancer, lung cancer, pancreatic cancer and the like), a cancer cell apoptosis inducer, a cancer cell growth suppressant, a DDR1 antagonist and the like.

This application is based on a patent application No. 2005-74065 filed in Japan on March 15, 2005, the contents of which are incorporated in full herein by this reference.

## Claims

1. A neutralizing antibody against a protein comprising the same or substantially the same amino acid sequence as that shown by SEQ ID NO:1, SEQ ID NO:3, SEQ ID NO:5, SEQ ID NO:7 or SEQ ID NO:9, or a partial peptide thereof or a salt thereof.

2. The neutralizing antibody of claim 1, which neutralizes the apoptosis-inhibitory activity resulting from the collagen binding to a protein comprising the same or substantially the same amino acid sequence as that shown by SEQ ID NO:1, SEQ ID NO:3, SEQ ID NO:5, SEQ ID NO:7 or SEQ ID NO:9, or a partial peptide thereof or a salt thereof.

3. The neutralizing antibody of claim 1, which neutralizes the cancer cell growth stimulation resulting from the collagen binding to a protein comprising the same or substantially the same amino acid sequence as that shown by SEQ ID NO:1, SEQ ID NO:3, SEQ ID NO:5, SEQ ID NO:7 or SEQ ID NO:9, or a partial peptide thereof or a salt thereof.

4. The neutralizing antibody of claim 2 or 3, wherein the collagen is type IV collagen.

5. The neutralizing antibody of claim 1, wherein the neutralizing antibody is an antibody against the polypeptide which amino acid sequence is from the 22nd to the 416th of that shown by SEQ ID NO:3 or a partial peptide thereof or a salt thereof.

6. The neutralizing antibody of claim 1, prepared by the DNA immunization method.

7. The neutralizing antibody of claim 1, wherein the neutralizing antibody is a polyclonal antibody.

8. The neutralizing antibody of claim 1, wherein the neutralizing antibody is a monoclonal antibody.

9. The neutralizing antibody of claim 1, wherein the neutralizing antibody is a humanized antibody.

10. The neutralizing antibody of claim 1, wherein the neutralizing antibody is a human antibody.

11. A medicament comprising a neutralizing antibody against a protein comprising the same or substantially the same amino acid sequence as that shown by SEQ ID NO:1, SEQ ID NO:3, SEQ ID NO:5, SEQ ID NO:7 or SEQ ID NO:9, or a partial peptide thereof or a salt thereof.

12. An antagonist for a protein comprising the same or substantially the same amino acid sequence as that shown by SEQ ID NO:1, SEQ ID NO:3, SEQ ID NO:5, SEQ ID NO:7 or SEQ ID NO:9, or a partial peptide thereof or a salt thereof, which comprises the neutralizing antibody against a protein comprising the same or substantially the same amino acid sequence as that shown by SEQ ID NO:1, SEQ ID NO:3, SEQ ID NO:5, SEQ ID NO:7 or SEQ ID NO:9, or a partial peptide thereof or a salt thereof.

13. An apoptosis inducer comprising a neutralizing antibody against a protein comprising the same or substantially the same amino acid sequence as that shown by SEQ ID NO:1, SEQ ID NO:3, SEQ ID NO:5, SEQ ID NO:7 or SEQ ID NO:9, or a partial peptide thereof or a salt thereof.

14. A cancer cell growth suppressant comprising a neutralizing antibody against a protein comprising the same or substantially the same amino acid sequence as that shown by SEQ ID NO:1, SEQ ID NO:3, SEQ ID NO:5, SEQ ID NO:7 or SEQ ID NO:9, or a partial peptide thereof or a salt thereof.

15. A cancer prophylactic/therapeutic agent comprising a neutralizing antibody against a protein comprising the same or substantially the same amino acid sequence as that shown by SEQ ID NO:1, SEQ ID NO:3, SEQ ID NO:5, SEQ ID NO:7 or SEQ ID NO:9, or a partial peptide thereof or a salt thereof.

16. The agent of claim 15, wherein the cancer is breast cancer, ovarian cancer, colorectal cancer, lung cancer or pancreatic cancer.

17. A cancer prophylactic/therapeutic method comprising administering, to a mammal, an effective amount of a neutralizing antibody against a protein comprising the same or substantially the same amino acid sequence as that shown by SEQ ID NO:1, SEQ ID NO:3, SEQ ID NO:5, SEQ ID NO:7 or SEQ ID NO:9, or a partial peptide thereof or a salt thereof.

18. A use of a neutralizing antibody against a protein comprising the same or substantially the same amino acid sequence as that shown by SEQ ID NO:1, SEQ ID NO:3, SEQ ID NO:5, SEQ ID NO:7 or SEQ ID NO:9, or a partial peptide thereof or a salt thereof, for producing a cancer prophylactic/therapeutic agent.


SEQUENCE LISTING

<110> Takeda Pharmaceutical Company Limited

<120> PROPHYLACTIC/THERAPEUTIC AGENT FOR CANCER

<130> A6794

<150> JP 2005-74065
<151> 2005-3-15

<160> 29

<210> 1
<211> 876
<212> PRT
<213> Human

<400> 1

Met Gly Pro Glu Ala Leu Ser Ser Leu Leu Leu Leu Leu Leu Val Ala
                5                   10                  15
Ser Gly Asp Ala Asp Met Lys Gly His Phe Asp Pro Ala Lys Cys Arg
            20                  25                  30
Tyr Ala Leu Gly Met Gln Asp Arg Thr Ile Pro Asp Ser Asp Ile Ser
        35                  40                  45
Ala Ser Ser Ser Trp Ser Asp Ser Thr Ala Ala Arg His Ser Arg Leu
    50                  55                  60
Glu Ser Ser Asp Gly Asp Gly Ala Trp Cys Pro Ala Gly Ser Val Phe
65                  70                  75                  80
Pro Lys Glu Glu Glu Tyr Leu Gln Val Asp Leu Gln Arg Leu His Leu
                85                  90                  95
Val Ala Leu Val Gly Thr Gln Gly Arg His Ala Gly Gly Leu Gly Lys
            100                 105                 110
Glu Phe Ser Arg Ser Tyr Arg Leu Arg Tyr Ser Arg Asp Gly Arg Arg
        115                 120                 125
Trp Met Gly Trp Lys Asp Arg Trp Gly Gln Glu Val Ile Ser Gly Asn
    130                 135                 140
Glu Asp Pro Glu Gly Val Val Leu Lys Asp Leu Gly Pro Pro Met Val
145                 150                 155                 160
Ala Arg Leu Val Arg Phe Tyr Pro Arg Ala Asp Arg Val Met Ser Val
                165                 170                 175
Cys Leu Arg Val Glu Leu Tyr Gly Cys Leu Trp Arg Asp Gly Leu Leu
            180                 185                 190
Ser Tyr Thr Ala Pro Val Gly Gln Thr Met Tyr Leu Ser Glu Ala Val
        195                 200                 205
Tyr Leu Asn Asp Ser Thr Tyr Asp Gly His Thr Val Gly Gly Leu Gln
    210                 215                 220
Tyr Gly Gly Leu Gly Gln Leu Ala Asp Gly Val Val Gly Leu Asp Asp
225                 230                 235                 240
Phe Arg Lys Ser Gln Glu Leu Arg Val Trp Pro Gly Tyr Asp Tyr Val
                245                 250                 255
Gly Trp Ser Asn His Ser Phe Ser Ser Gly Tyr Val Glu Met Glu Phe
            260                 265                 270
Glu Phe Asp Arg Leu Arg Ala Phe Gln Ala Met Gln Val His Cys Asn
        275                 280                 285
Asn Met His Thr Leu Gly Ala Arg Leu Pro Gly Gly Val Glu Cys Arg
    290                 295                 300
Phe Arg Arg Gly Pro Ala Met Ala Trp Glu Gly Glu Pro Met Arg His
305                 310                 315                 320
Asn Leu Gly Gly Asn Leu Gly Asp Pro Arg Ala Arg Ala Val Ser Val
                325                 330                 335
Pro Leu Gly Gly Arg Val Ala Arg Phe Leu Gln Cys Arg Phe Leu Phe

            340                 345                 350
Ala Gly Pro Trp Leu Leu Phe Ser Glu Ile Ser Phe Ile Ser Asp Val
        355                 360                 365
Val Asn Asn Ser Ser Pro Ala Leu Gly Gly Thr Phe Pro Pro Ala Pro
    370                 375                 380
Trp Trp Pro Pro Gly Pro Pro Pro Thr Asn Phe Ser Ser Leu Glu Leu
385                 390                 395                 400
Glu Pro Arg Gly Gln Gln Pro Val Ala Lys Ala Glu Gly Ser Pro Thr
                405                 410                 415
Ala Ile Leu Ile Gly Cys Leu Val Ala Ile Ile Leu Leu Leu Leu Leu
            420                 425                 430
Ile Ile Ala Leu Met Leu Trp Arg Leu His Trp Arg Arg Leu Leu Ser
        435                 440                 445
Lys Ala Glu Arg Arg Val Leu Glu Glu Glu Leu Thr Val His Leu Ser
    450                 455                 460
Val Pro Gly Asp Thr Ile Leu Ile Asn Asn Arg Pro Gly Pro Arg Glu
465                 470                 475                 480
Pro Pro Pro Tyr Gln Glu Pro Arg Pro Arg Gly Asn Pro Pro His Ser
                485                 490                 495
Ala Pro Cys Val Pro Asn Gly Ser Ala Tyr Ser Gly Asp Tyr Met Glu
            500                 505                 510
Pro Glu Lys Pro Gly Ala Pro Leu Leu Pro Pro Pro Pro Gln Asn Ser
        515                 520                 525
Val Pro His Tyr Ala Glu Ala Asp Ile Val Thr Leu Gln Gly Val Thr
    530                 535                 540
Gly Gly Asn Thr Tyr Ala Val Pro Ala Leu Pro Pro Gly Ala Val Gly
545                 550                 555                 560
Asp Gly Pro Pro Arg Val Asp Phe Pro Arg Ser Arg Leu Arg Phe Lys
                565                 570                 575
Glu Lys Leu Gly Glu Gly Gln Phe Gly Glu Val His Leu Cys Glu Val
            580                 585                 590
Asp Ser Pro Gln Asp Leu Val Ser Leu Asp Phe Pro Leu Asn Val Arg
        595                 600                 605
Lys Gly His Pro Leu Leu Val Ala Val Lys Ile Leu Arg Pro Asp Ala
    610                 615                 620
Thr Lys Asn Ala Arg Asn Asp Phe Leu Lys Glu Val Lys Ile Met Ser
625                 630                 635                 640
Arg Leu Lys Asp Pro Asn Ile Ile Arg Leu Leu Gly Val Cys Val Gln
                645                 650                 655
Asp Asp Pro Leu Cys Met Ile Thr Asp Tyr Met Glu Asn Gly Asp Leu
            660                 665                 670
Asn Gln Phe Leu Ser Ala His Gln Leu Glu Asp Lys Ala Ala Glu Gly
        675                 680                 685
Ala Pro Gly Asp Gly Gln Ala Ala Gln Gly Pro Thr Ile Ser Tyr Pro
    690                 695                 700
Met Leu Leu His Val Ala Ala Gln Ile Ala Ser Gly Met Arg Tyr Leu
705                 710                 715                 720
Ala Thr Leu Asn Phe Val His Arg Asp Leu Ala Thr Arg Asn Cys Leu
                725                 730                 735
Val Gly Glu Asn Phe Thr Ile Lys Ile Ala Asp Phe Gly Met Ser Arg
            740                 745                 750
Asn Leu Tyr Ala Gly Asp Tyr Tyr Arg Val Gln Gly Arg Ala Val Leu
        755                 760                 765
Pro Ile Arg Trp Met Ala Trp Glu Cys Ile Leu Met Gly Lys Phe Thr
    770                 775                 780
Thr Ala Ser Asp Val Trp Ala Phe Gly Val Thr Leu Trp Glu Val Leu
785                 790                 795                 800
Met Leu Cys Arg Ala Gln Pro Phe Gly Gln Leu Thr Asp Glu Gln Val
                805                 810                 815
Ile Glu Asn Ala Gly Glu Phe Phe Arg Asp Gln Gly Arg Gln Val Tyr
            820                 825                 830
Leu Ser Arg Pro Pro Ala Cys Pro Gln Gly Leu Tyr Glu Leu Met Leu
        835                 840                 845

Arg Cys Trp Ser Arg Glu Ser Glu Gln Arg Pro Pro Phe Ser Gln Leu
    850                 855                 860
His Arg Phe Leu Ala Glu Asp Ala Leu Asn Thr Val
865                 870                 875

<210> 2
<211> 2628
<212> DNA
<213> Human

<400> 2

atgggaccag aggccctgtc atctttactg ctgctgctct tggtggcaag tggagatgct      60
gacatgaagg gacattttga tcctgccaag tgccgctatg ccctgggcat gcaggaccgg     120
accatcccag acagtgacat ctctgcttcc agctcctggt cagattccac tgccgcccgc     180
cacagcaggt tggagagcag tgacggggat ggggcctggt gccccgcagg gtcggtgttt     240
cccaaggagg aggagtactt gcaggtggat ctacaacgac tgcacctggt ggctctggtg     300
ggcacccagg gacggcatgc cgggggcctg ggcaaggagt tctcccggag ctaccggctg     360
cgttactccc gggatggtcg ccgctggatg ggctggaagg accgctgggg tcaggaggtg     420
atctcaggca atgaggaccc tgagggagtg gtgctgaagg accttgggcc ccccatggtt     480
gcccgactgg ttcgcttcta cccccgggct gaccgggtca tgagcgtctg tctgcgggta     540
gagctctatg gctgcctctg gagggatgga ctcctgtctt acaccgcccc tgtggggcag     600
acaatgtatt tatctgaggc cgtgtacctc aacgactcca cctatgacgg acataccgtg     660
ggcggactgc agtatggggg tctgggccag ctggcagatg gtgtggtggg gctggatgac     720
tttaggaaga gtcaggagct gcgggtctgg ccaggctatg actatgtggg atggagcaac     780
cacagcttct ccagtggcta tgtggagatg gagtttgagt ttgaccggct gagggccttc     840
caggctatgc aggtccactg taacaacatg cacacgctgg gagcccgtct gcctggcggg     900
gtggaatgtc gcttccggcg tggccctgcc atggcctggg agggggagcc catgcgccac     960
aacctagggg gcaacctggg ggaccccaga gcccgggctg tctcagtgcc ccttggcggc    1020
cgtgtggctc gctttctgca gtgccgcttc ctctttgcgg ggccctggtt actcttcagc    1080
gaaatctcct tcatctctga tgtggtgaac aattcctctc cggcactggg aggcaccttc    1140
ccgccagccc cctggtggcc gcctggccca cctcccacca acttcagcag cttggagctg    1200
gagcccagag gccagcagcc cgtggccaag gccgagggga gcccgaccgc catcctcatc    1260
ggctgcctgg tggccatcat cctgctcctg ctgctcatca ttgccctcat gctctggcgg    1320
ctgcactggc gcaggctcct cagcaaggct gaacggaggg tgttggaaga ggagctgacg    1380
gttcacctct ctgtccctgg ggacactatc ctcatcaaca accgcccagg tcctagagag    1440
ccacccccgt accaggagcc ccggcctcgt gggaatccgc cccactccgc tccctgtgtc    1500
cccaatggct ctgcctacag tggggactat atggagcctg agaagccagg cgccccgctt    1560
ctgcccccac ctccccagaa cagcgtcccc cattatgccg aggctgacat tgttaccctg    1620
cagggcgtca ccgggggcaa cacctatgct gtgcctgcac tgcccccagg ggcagtcggg    1680
gatggccccc ccagagtgga tttccctcga tctcgactcc gcttcaagga gaagcttggc    1740
gagggccagt ttggggaggt gcacctgtgt gaggtcgaca gccctcaaga tctggttagt    1800
cttgatttcc cccttaatgt gcgtaaggga caccctttgc tggtagctgt caagatctta    1860
cggccagatg ccaccaagaa tgccaggaat gatttcctga aagaggtgaa gatcatgtcg    1920
aggctcaagg acccaaacat cattcggctg ctgggcgtgt gtgtgcagga cgaccccctc    1980
tgcatgatta ctgactacat ggagaacggc gacctcaacc agttcctcag tgcccaccag    2040
ctggaggaca aggcagccga gggggcccct ggggacgggc aggctgcgca ggggcccacc    2100
atcagctacc caatgctgct gcatgtggca gcccagatcg cctccggcat gcgctatctg    2160
gccacactca actttgtaca tcgggacctg gccacgcgga actgcctagt tggggaaaat    2220
ttcaccatca aaatcgcaga ctttggcatg agccggaacc tctatgctgg ggactattac    2280
cgtgtgcagg gccgggcagt gctgcccatc cgctggatgg cctgggagtg catcctcatg    2340
gggaagttca cgactgcgag tgacgtgtgg gcctttggtg tgaccctgtg ggaggtgctg    2400
atgctctgta gggcccagcc ctttgggcag ctcaccgacg agcaggtcat cgagaacgcg    2460
ggggagttct tccgggacca gggccggcag gtgtacctgt cccggccgcc tgcctgcccg    2520
cagggcctat atgagctgat gcttcggtgc tggagccggg agtctgagca gcgaccaccc    2580
ttttcccagc tgcatcggtt cctggcagag gatgcactca acacggtg                 2628

<210> 3
<211> 913
<212> PRT
<213> Human

<400> 3

Met Gly Pro Glu Ala Leu Ser Ser Leu Leu Leu Leu Leu Leu Val Ala
                5                   10                  15
Ser Gly Asp Ala Asp Met Lys Gly His Phe Asp Pro Ala Lys Cys Arg
            20                  25                  30
Tyr Ala Leu Gly Met Gln Asp Arg Thr Ile Pro Asp Ser Asp Ile Ser
        35                  40                  45
Ala Ser Ser Ser Trp Ser Asp Ser Thr Ala Ala Arg His Ser Arg Leu
    50                  55                  60
Glu Ser Ser Asp Gly Asp Gly Ala Trp Cys Pro Ala Gly Ser Val Phe
65                  70                  75                  80
Pro Lys Glu Glu Glu Tyr Leu Gln Val Asp Leu Gln Arg Leu His Leu
                85                  90                  95
Val Ala Leu Val Gly Thr Gln Gly Arg His Ala Gly Gly Leu Gly Lys
            100                 105                 110
Glu Phe Ser Arg Ser Tyr Arg Leu Arg Tyr Ser Arg Asp Gly Arg Arg
        115                 120                 125
Trp Met Gly Trp Lys Asp Arg Trp Gly Gln Glu Val Ile Ser Gly Asn
    130                 135                 140
Glu Asp Pro Glu Gly Val Val Leu Lys Asp Leu Gly Pro Pro Met Val
145                 150                 155                 160
Ala Arg Leu Val Arg Phe Tyr Pro Arg Ala Asp Arg Val Met Ser Val
                165                 170                 175
Cys Leu Arg Val Glu Leu Tyr Gly Cys Leu Trp Arg Asp Gly Leu Leu
            180                 185                 190
Ser Tyr Thr Ala Pro Val Gly Gln Thr Met Tyr Leu Ser Glu Ala Val
        195                 200                 205
Tyr Leu Asn Asp Ser Thr Tyr Asp Gly His Thr Val Gly Gly Leu Gln
    210                 215                 220
Tyr Gly Gly Leu Gly Gln Leu Ala Asp Gly Val Val Gly Leu Asp Asp
225                 230                 235                 240
Phe Arg Lys Ser Gln Glu Leu Arg Val Trp Pro Gly Tyr Asp Tyr Val
                245                 250                 255
Gly Trp Ser Asn His Ser Phe Ser Ser Gly Tyr Val Glu Met Glu Phe
            260                 265                 270
Glu Phe Asp Arg Leu Arg Ala Phe Gln Ala Met Gln Val His Cys Asn
        275                 280                 285
Asn Met His Thr Leu Gly Ala Arg Leu Pro Gly Gly Val Glu Cys Arg
    290                 295                 300
Phe Arg Arg Gly Pro Ala Met Ala Trp Glu Gly Glu Pro Met Arg His
305                 310                 315                 320
Asn Leu Gly Gly Asn Leu Gly Asp Pro Arg Ala Arg Ala Val Ser Val
                325                 330                 335
Pro Leu Gly Gly Arg Val Ala Arg Phe Leu Gln Cys Arg Phe Leu Phe
            340                 345                 350
Ala Gly Pro Trp Leu Leu Phe Ser Glu Ile Ser Phe Ile Ser Asp Val
        355                 360                 365
Val Asn Asn Ser Ser Pro Ala Leu Gly Gly Thr Phe Pro Pro Ala Pro
    370                 375                 380
Trp Trp Pro Pro Gly Pro Pro Pro Thr Asn Phe Ser Ser Leu Glu Leu
385                 390                 395                 400
Glu Pro Arg Gly Gln Gln Pro Val Ala Lys Ala Glu Gly Ser Pro Thr
                405                 410                 415
Ala Ile Leu Ile Gly Cys Leu Val Ala Ile Ile Leu Leu Leu Leu Leu
            420                 425                 430
Ile Ile Ala Leu Met Leu Trp Arg Leu His Trp Arg Arg Leu Leu Ser
        435                 440                 445
Lys Ala Glu Arg Arg Val Leu Glu Glu Glu Leu Thr Val His Leu Ser
    450                 455                 460
Val Pro Gly Asp Thr Ile Leu Ile Asn Asn Arg Pro Gly Pro Arg Glu
465                 470                 475                 480

Pro Pro Pro Tyr Gln Glu Pro Arg Pro Arg Gly Asn Pro Pro His Ser
                485                 490                 495
Ala Pro Cys Val Pro Asn Gly Ser Ala Leu Leu Leu Ser Asn Pro Ala
            500                 505                 510
Tyr Arg Leu Leu Leu Ala Thr Tyr Ala Arg Pro Pro Arg Gly Pro Gly
        515                 520                 525
Pro Pro Thr Pro Ala Trp Ala Lys Pro Thr Asn Thr Gln Ala Tyr Ser
    530                 535                 540
Gly Asp Tyr Met Glu Pro Glu Lys Pro Gly Ala Pro Leu Leu Pro Pro
545                 550                 555                 560
Pro Pro Gln Asn Ser Val Pro His Tyr Ala Glu Ala Asp Ile Val Thr
                565                 570                 575
Leu Gln Gly Val Thr Gly Gly Asn Thr Tyr Ala Val Pro Ala Leu Pro
            580                 585                 590
Pro Gly Ala Val Gly Asp Gly Pro Pro Arg Val Asp Phe Pro Arg Ser
        595                 600                 605
Arg Leu Arg Phe Lys Glu Lys Leu Gly Glu Gly Gln Phe Gly Glu Val
    610                 615                 620
His Leu Cys Glu Val Asp Ser Pro Gln Asp Leu Val Ser Leu Asp Phe
625                 630                 635                 640
Pro Leu Asn Val Arg Lys Gly His Pro Leu Leu Val Ala Val Lys Ile
                645                 650                 655
Leu Arg Pro Asp Ala Thr Lys Asn Ala Arg Asn Asp Phe Leu Lys Glu
            660                 665                 670
Val Lys Ile Met Ser Arg Leu Lys Asp Pro Asn Ile Ile Arg Leu Leu
        675                 680                 685
Gly Val Cys Val Gln Asp Asp Pro Leu Cys Met Ile Thr Asp Tyr Met
    690                 695                 700
Glu Asn Gly Asp Leu Asn Gln Phe Leu Ser Ala His Gln Leu Glu Asp
705                 710                 715                 720
Lys Ala Ala Glu Gly Ala Pro Gly Asp Gly Gln Ala Ala Gln Gly Pro
                725                 730                 735
Thr Ile Ser Tyr Pro Met Leu Leu His Val Ala Ala Gln Ile Ala Ser
            740                 745                 750
Gly Met Arg Tyr Leu Ala Thr Leu Asn Phe Val His Arg Asp Leu Ala
        755                 760                 765
Thr Arg Asn Cys Leu Val Gly Glu Asn Phe Thr Ile Lys Ile Ala Asp
    770                 775                 780
Phe Gly Met Ser Arg Asn Leu Tyr Ala Gly Asp Tyr Tyr Arg Val Gln
785                 790                 795                 800
Gly Arg Ala Val Leu Pro Ile Arg Trp Met Ala Trp Glu Cys Ile Leu
                805                 810                 815
Met Gly Lys Phe Thr Thr Ala Ser Asp Val Trp Ala Phe Gly Val Thr
            820                 825                 830
Leu Trp Glu Val Leu Met Leu Cys Arg Ala Gln Pro Phe Gly Gln Leu
        835                 840                 845
Thr Asp Glu Gln Val Ile Glu Asn Ala Gly Glu Phe Phe Arg Asp Gln
    850                 855                 860
Gly Arg Gln Val Tyr Leu Ser Arg Pro Pro Ala Cys Pro Gln Gly Leu
865                 870                 875                 880
Tyr Glu Leu Met Leu Arg Cys Trp Ser Arg Glu Ser Glu Gln Arg Pro
                885                 890                 895
Pro Phe Ser Gln Leu His Arg Phe Leu Ala Glu Asp Ala Leu Asn Thr
            900                 905                 910
Val

<210> 4
<211> 2739
<212> DNA
<213> Human

<400> 4

atgggaccag aggccctgtc atctttactg ctgctgctct tggtggcaag tggagatgct    60
gacatgaagg gacattttga tcctgccaag tgccgctatg ccctgggcat gcaggaccgg   120
accatcccag acagtgacat ctctgcttcc agctcctggt cagattccac tgccgcccgc   180
cacagcaggt tggagagcag tgacggggat ggggcctggt gccccgcagg gtcggtgttt   240
cccaaggagg aggagtactt gcaggtggat ctacaacgac tgcacctggt ggctctggtg   300
ggcacccagg gacggcatgc cgggggcctg ggcaaggagt tctcccggag ctaccggctg   360
cgttactccc gggatggtcg ccgctggatg ggctggaagg accgctgggg tcaggaggtg   420
atctcaggca atgaggaccc tgagggagtg gtgctgaagg accttggccc ccccatggtt   480
gcccgactgg ttcgcttcta cccccgggct gaccgggtca tgagcgtctg tctgcgggta   540
gagctctatg gctgcctctg gagggatgga ctcctgtctt acaccgcccc tgtggggcag   600
acaatgtatt tatctgaggc cgtgtacctc aacgactcca cctatgacgg acataccgtg   660
ggcggactgc agtatggggg tctgggccag ctggcagatg gtgtggtggg gctggatgac   720
tttaggaaga gtcaggagct gcgggtctgg ccaggctatg actatgtggg atggagcaac   780
cacagcttct ccagtggcta tgtggagatg gagtttgagt ttgaccggct gagggccttc   840
caggctatgc aggtccactg taacaacatg cacacgctgg gagcccgtct gcctggcggg   900
gtggaatgtc gcttccggcg tggccctgcc atggcctggg agggggagcc catgcgccac   960
aacctagggg gcaacctggg ggaccccaga gcccgggctg tctcagtgcc ccttggcggc  1020
cgtgtggctc gctttctgca gtgccgcttc ctctttgcgg ggccctggtt actcttcagc  1080
gaaatctcct tcatctctga tgtggtgaac aattcctctc cggcactggg aggcaccttc  1140
ccgccagccc cctggtggcc gcctggccca cctcccacca acttcagcag cttggagctg  1200
gagcccagag gccagcagcc cgtggccaag gccgagggga gcccgaccgc catcctcatc  1260
ggctgcctgg tggccatcat cctgctcctg ctgctcatca ttgccctcat gctctggcgg  1320
ctgcactggc gcaggctcct cagcaaggct gaacggaggg tgttggaaga ggagctgacg  1380
gttcacctct ctgtccctgg ggacactatc ctcatcaaca accgcccagg tcctagagag  1440
ccacccccgt accaggagcc ccggcctcgt gggaatccgc cccactctgc tccctgtgtc  1500
cccaatggct ctgcgttgct gctctccaat ccagcctacc gcctccttct ggccacttac  1560
gcccgtcccc ctcgaggccc gggccccccc acacccgcct gggccaaacc caccaacacc  1620
caggcctaca gtggggacta tatggagcct gagaagccag gcgccccgct tctgccccca  1680
cctccccaga acagcgtccc ccattatgcc gaggctgaca ttgttaccct gcagggcgtc  1740
accgggggca acacctatgc tgtgcctgca ctgcccccag ggcagtcggg ggatggcccc  1800
cccagagtgg atttccctcg atctcgactc cgcttcaagg agaagcttgg cgagggccag  1860
tttggggagg tgcacctgtg tgaggtcgac agccctcaag atctggtcag tcttgatttc  1920
ccccttaatg tgcgtaaggg acaccctttg ctggtagctg tcaagatctt acggccagat  1980
gccaccaaga atgccaggaa tgatttcctg aaagaggtga agatcatgtc gaggctcaag  2040
gacccaaaca tcattcggct gctgggcgtg tgtgtgcagg acgaccccct ctgcatgatt  2100
actgactaca tggagaacgg cgacctcaac cagttcctca gtgcccacca gctggaggac  2160
aaggcagccg agggggcccc tggggacggg caggctgcgc aggggcccac catcagctac  2220
ccaatgctgc tgcatgtggc agcccagatc gcctccggca tgcgctatct ggccacactc  2280
aactttgtac atcgggacct ggccacgcgg aactgcctag ttggggaaaa tttcaccatc  2340
aaaatcgcag actttggcat gagccggaac ctctatgctg gggactatta ccgtgtgcag  2400
ggccgggcag tgctgcccat ccgctggatg gcctgggagt gcatcctcat ggggaagttc  2460
acgactgcga gtgacgtgtg ggcctttggt gtgaccctgt gggaggtgct gatgctctgt  2520
agggcccagc cctttgggca gctcaccgac gagcaggtca tcgagaacgc gggggagttc  2580
ttccggaccc agggccggca ggtgtacctg tcccggccgc ctgcctgccc gcagggccta  2640
tatgagctga tgcttcggtg ctggagccgg gagtctgagc agcgaccacc cttttcccag  2700
ctgcatcggt tcctggcaga ggatgcactc aacacggtg                          2739

<210> 5
<211> 919
<212> PRT
<213> Human

<400> 5

Met Gly Pro Glu Ala Leu Ser Ser Leu Leu Leu Leu Leu Leu Val Ala
                5                   10                  15
Ser Gly Asp Ala Asp Met Lys Gly His Phe Asp Pro Ala Lys Cys Arg
            20                  25                  30
Tyr Ala Leu Gly Met Gln Asp Arg Thr Ile Pro Asp Ser Asp Ile Ser
        35                  40                  45

Ala Ser Ser Ser Trp Ser Asp Ser Thr Ala Ala Arg His Ser Arg Leu
    50                  55                  60
Glu Ser Ser Asp Gly Asp Gly Ala Trp Cys Pro Ala Gly Ser Val Phe
65                  70                  75                  80
Pro Lys Glu Glu Glu Tyr Leu Gln Val Asp Leu Gln Arg Leu His Leu
                85                  90                  95
Val Ala Leu Val Gly Thr Gln Gly Arg His Ala Gly Gly Leu Gly Lys
            100                 105                 110
Glu Phe Ser Arg Ser Tyr Arg Leu Arg Tyr Ser Arg Asp Gly Arg Arg
        115                 120                 125
Trp Met Gly Trp Lys Asp Arg Trp Gly Gln Glu Val Ile Ser Gly Asn
    130                 135                 140
Glu Asp Pro Glu Gly Val Val Leu Lys Asp Leu Gly Pro Pro Met Val
145                 150                 155                 160
Ala Arg Leu Val Arg Phe Tyr Pro Arg Ala Asp Arg Val Met Ser Val
                165                 170                 175
Cys Leu Arg Val Glu Leu Tyr Gly Cys Leu Trp Arg Asp Gly Leu Leu
            180                 185                 190
Ser Tyr Thr Ala Pro Val Gly Gln Thr Met Tyr Leu Ser Glu Ala Val
        195                 200                 205
Tyr Leu Asn Asp Ser Thr Tyr Asp Gly His Thr Val Gly Gly Leu Gln
    210                 215                 220
Tyr Gly Gly Leu Gly Gln Leu Ala Asp Gly Val Val Gly Leu Asp Asp
225                 230                 235                 240
Phe Arg Lys Ser Gln Glu Leu Arg Val Trp Pro Gly Tyr Asp Tyr Val
                245                 250                 255
Gly Trp Ser Asn His Ser Phe Ser Ser Gly Tyr Val Glu Met Glu Phe
            260                 265                 270
Glu Phe Asp Arg Leu Arg Ala Phe Gln Ala Met Gln Val His Cys Asn
        275                 280                 285
Asn Met His Thr Leu Gly Ala Arg Leu Pro Gly Gly Val Glu Cys Arg
    290                 295                 300
Phe Arg Arg Gly Pro Ala Met Ala Trp Glu Gly Glu Pro Met Arg His
305                 310                 315                 320
Asn Leu Gly Gly Asn Leu Gly Asp Pro Arg Ala Arg Ala Val Ser Val
                325                 330                 335
Pro Leu Gly Gly Arg Val Ala Arg Phe Leu Gln Cys Arg Phe Leu Phe
            340                 345                 350
Ala Gly Pro Trp Leu Leu Phe Ser Glu Ile Ser Phe Ile Ser Asp Val
        355                 360                 365
Val Asn Asn Ser Ser Pro Ala Leu Gly Gly Thr Phe Pro Pro Ala Pro
    370                 375                 380
Trp Trp Pro Pro Gly Pro Pro Pro Thr Asn Phe Ser Ser Leu Glu Leu
385                 390                 395                 400
Glu Pro Arg Gly Gln Gln Pro Val Ala Lys Ala Glu Gly Ser Pro Thr
                405                 410                 415
Ala Ile Leu Ile Gly Cys Leu Val Ala Ile Ile Leu Leu Leu Leu Leu
            420                 425                 430
Ile Ile Ala Leu Met Leu Trp Arg Leu His Trp Arg Arg Leu Leu Ser
        435                 440                 445
Lys Ala Glu Arg Arg Val Leu Glu Glu Glu Leu Thr Val His Leu Ser
    450                 455                 460
Val Pro Gly Asp Thr Ile Leu Ile Asn Asn Arg Pro Gly Pro Arg Glu
465                 470                 475                 480
Pro Pro Pro Tyr Gln Glu Pro Arg Pro Arg Gly Asn Pro Pro His Ser
                485                 490                 495
Ala Pro Cys Val Pro Asn Gly Ser Ala Leu Leu Leu Ser Asn Pro Ala
            500                 505                 510
Tyr Arg Leu Leu Leu Ala Thr Tyr Ala Arg Pro Pro Arg Gly Pro Gly
        515                 520                 525
Pro Pro Thr Pro Ala Trp Ala Lys Pro Thr Asn Thr Gln Ala Tyr Ser
    530                 535                 540
Gly Asp Tyr Met Glu Pro Glu Lys Pro Gly Ala Pro Leu Leu Pro Pro

545                 550                 555                 560
Pro Pro Gln Asn Ser Val Pro His Tyr Ala Glu Ala Asp Ile Val Thr
                565                 570                 575
Leu Gln Gly Val Thr Gly Gly Asn Thr Tyr Ala Val Pro Ala Leu Pro
            580                 585                 590
Pro Gly Ala Val Gly Asp Gly Pro Pro Arg Val Asp Phe Pro Arg Ser
        595                 600                 605
Arg Leu Arg Phe Lys Glu Lys Leu Gly Glu Gly Gln Phe Gly Glu Val
    610                 615                 620
His Leu Cys Glu Val Asp Ser Pro Gln Asp Leu Val Ser Leu Asp Phe
625                 630                 635                 640
Pro Leu Asn Val Arg Lys Gly His Pro Leu Leu Val Ala Val Lys Ile
                645                 650                 655
Leu Arg Pro Asp Ala Thr Lys Asn Ala Ser Phe Ser Leu Phe Ser Arg
            660                 665                 670
Asn Asp Phe Leu Lys Glu Val Lys Ile Met Ser Arg Leu Lys Asp Pro
        675                 680                 685
Asn Ile Ile Arg Leu Leu Gly Val Cys Val Gln Asp Asp Pro Leu Cys
    690                 695                 700
Met Ile Thr Asp Tyr Met Glu Asn Gly Asp Leu Asn Gln Phe Leu Ser
705                 710                 715                 720
Ala His Gln Leu Glu Asp Lys Ala Ala Glu Gly Ala Pro Gly Asp Gly
                725                 730                 735
Gln Ala Ala Gln Gly Pro Thr Ile Ser Tyr Pro Met Leu Leu His Val
            740                 745                 750
Ala Ala Gln Ile Ala Ser Gly Met Arg Tyr Leu Ala Thr Leu Asn Phe
        755                 760                 765
Val His Arg Asp Leu Ala Thr Arg Asn Cys Leu Val Gly Glu Asn Phe
    770                 775                 780
Thr Ile Lys Ile Ala Asp Phe Gly Met Ser Arg Asn Leu Tyr Ala Gly
785                 790                 795                 800
Asp Tyr Tyr Arg Val Gln Gly Arg Ala Val Leu Pro Ile Arg Trp Met
                805                 810                 815
Ala Trp Glu Cys Ile Leu Met Gly Lys Phe Thr Thr Ala Ser Asp Val
            820                 825                 830
Trp Ala Phe Gly Val Thr Leu Trp Glu Val Leu Met Leu Cys Arg Ala
        835                 840                 845
Gln Pro Phe Gly Gln Leu Thr Asp Glu Gln Val Ile Glu Asn Ala Gly
    850                 855                 860
Glu Phe Phe Arg Asp Gln Gly Arg Gln Val Tyr Leu Ser Arg Pro Pro
865                 870                 875                 880
Ala Cys Pro Gln Gly Leu Tyr Glu Leu Met Leu Arg Cys Trp Ser Arg
                885                 890                 895
Glu Ser Glu Gln Arg Pro Pro Phe Ser Gln Leu His Arg Phe Leu Ala
            900                 905                 910
Glu Asp Ala Leu Asn Thr Val
        915

<210> 6
<211> 2757
<212> DNA
<213> Human

<400> 6

atgggaccag aggccctgtc atctttactg ctgctgctct tggtggcaag tggagatgct      60
gacatgaagg gacattttga tcctgccaag tgccgctatg ccctgggcat gcaggaccgg     120
accatcccag acagtgacat ctctgcttcc agctcctggt cagattccac tgccgcccgc     180
cacagcaggt tggagagcag tgacggggat ggggcctggt gccccgcagg gtcggtgttt     240
cccaaggagg aggagtactt gcaggtggat ctacaacgac tgcacctggt ggctctggtg     300
ggcacccagg gacggcatgc cggggggctg ggcaaggagt tctcccggag ctaccggctg     360
cgttactccc gggatggtcg ccgctggatg ggctggaagg accgctgggg tcaggaggtg     420

atctcaggca atgaggaccc tgagggagtg gtgctgaagg accttgggcc ccccatggtt 480
gcccgactgg ttcgcttcta cccccgggct gaccgggtca tgagcgtctg tctgcgggta 540
gagctctatg gctgcctctg gagggatgga ctcctgtctt acaccgcccc tgtggggcag 600
acaatgtatt tatctgaggc cgtgtacctc aacgactcca cctatgacgg acataccgtg 660
ggcggactgc agtatggggg tctgggccag ctggcagatg gtgtggtggg gctggatgac 720
tttaggaaga gtcaggagct gcgggtctgg ccaggctatg actatgtggg atggagcaac 780
cacagcttct ccagtggcta tgtggagatg gagtttgagt ttgaccggct gagggccttc 840
caggctatgc aggtccactg taacaacatg cacacgctgg gagcccgtct gcctggcggg 900
gtggaatgtc gcttccggcg tggccctgcc atggcctggg agggggagcc catgcgccac 960
aacctagggg gcaacctggg ggaccccaga gcccgggctg tctcagtgcc ccttggcggc 1020
cgtgtggctc gctttctgca gtgccgcttc ctctttgcgg ggccctggtt actcttcagc 1080
gaaatctcct tcatctctga tgtggtgaac aattcctctc cggcactggg aggcaccttc 1140
ccgccagccc cctggtggcc gcctggccca cctcccacca acttcagcag cttggagctg 1200
gagcccagag gccagcagcc cgtggccaag gccgagggga gcccgaccgc catcctcatc 1260
ggctgcctgg tggccatcat cctgctcctg ctgctcatca ttgccctcat gctctggcgg 1320
ctgcactggc gcaggctcct cagcaaggct gaacggaggg tgttggaaga ggagctgacg 1380
gttcacctct ctgtccctgg ggacactatc ctcatcaaca accgcccagg tcctagagag 1440
ccacccccgt accaggagcc ccggcctcgt gggaatccgc cccactccgc tccctgtgtc 1500
cccaatggct ctgcgttgct gctctccaat ccagcctacc gcctccttct ggccacttac 1560
gcccgtcccc ctcgaggccc gggccccccc acacccgcct gggccaaacc caccaacacc 1620
caggcctaca gtggggacta tatggagcct gagaagccag gcgccccgct tctgccccca 1680
cctccccaga acagcgtccc ccattatgcc gaggctgaca ttgttaccct gcagggcgtc 1740
accgggggca acacctatgc tgtgcctgca ctgcccccag ggcagtcggg gatgggcccc 1800
cccagagtgg atttccctcg atctcgactc cgcttcaagg agaagcttgg cgagggccag 1860
tttggggagg tgcacctgtg tgaggtcgac agccctcaag atctggttag tcttgatttc 1920
ccccttaatg tgcgtaaggg acaccctttg ctggtagctg tcaagatctt acggccagat 1980
gccaccaaga atgccagctt ctccttgttc tccaggaatg atttcctgaa agaggtgaag 2040
atcatgtcga ggctcaagga cccaaacatc attcggctgc tgggcgtgtg tgtgcaggac 2100
gaccccctct gcatgattac tgactacatg gagaacggcg acctcaacca gttcctcagt 2160
gcccaccagc tggaggacaa ggcagccgag ggggcccctg gggacgggca ggctgcgcag 2220
gggcccacca tcagctaccc aatgctgctg catgtggcag cccagatcgc ctccggcatg 2280
cgctatctgg ccacactcaa ctttgtacat cgggacctgg ccacgcggaa ctgcctagtt 2340
ggggaaaatt tcaccatcaa aatcgcagac tttggcatga gccggaacct ctatgctggg 2400
gactattacc gtgtgcaggg ccgggcagtg ctgcccatcc gctggatggc ctgggagtgc 2460
atcctcatgg ggaagttcac gactgcgagt gacgtgtggg cctttggtgt gaccctgtgg 2520
gaggtgctga tgctctgtag ggcccagccc tttgggcagc tcaccgacga gcaggtcatc 2580
gagaacgcgg gggagttctt ccgggaccag ggccggcagg tgtacctgtc ccggccgcct 2640
gcctgcccgc agggcctata tgagctgatg cttcggtgct ggagccggga gtctgagcag 2700
cgaccaccct tttcccagct gcatcggttc ctggcagagg atgcactcaa cacggtg 2757

<210> 7
<211> 508
<212> PRT
<213> Human

<400> 7

Met Gly Pro Glu Ala Leu Ser Ser Leu Leu Leu Leu Leu Leu Val Ala
                5                   10                  15
Ser Gly Asp Ala Asp Met Lys Gly His Phe Asp Pro Ala Lys Cys Arg
            20                  25                  30
Tyr Ala Leu Gly Met Gln Asp Arg Thr Ile Pro Asp Ser Asp Ile Ser
        35                  40                  45
Ala Ser Ser Ser Trp Ser Asp Ser Thr Ala Ala Arg His Ser Arg Leu
    50                  55                  60
Glu Ser Ser Asp Gly Asp Gly Ala Trp Cys Pro Ala Gly Ser Val Phe
65                  70                  75                  80
Pro Lys Glu Glu Glu Tyr Leu Gln Val Asp Leu Gln Arg Leu His Leu
                85                  90                  95
Val Ala Leu Val Gly Thr Gln Gly Arg His Ala Gly Gly Leu Gly Lys
            100                 105                 110
Glu Phe Ser Arg Ser Tyr Arg Leu Arg Tyr Ser Arg Asp Gly Arg Arg

115                 120                 125
Trp Met Gly Trp Lys Asp Arg Trp Gly Gln Glu Val Ile Ser Gly Asn
    130                 135                 140
Glu Asp Pro Glu Gly Val Val Leu Lys Asp Leu Gly Pro Pro Met Val
145                 150                 155                 160
Ala Arg Leu Val Arg Phe Tyr Pro Arg Ala Asp Arg Val Met Ser Val
                165                 170                 175
Cys Leu Arg Val Glu Leu Tyr Gly Cys Leu Trp Arg Asp Gly Leu Leu
            180                 185                 190
Ser Tyr Thr Ala Pro Val Gly Gln Thr Met Tyr Leu Ser Glu Ala Val
        195                 200                 205
Tyr Leu Asn Asp Ser Thr Tyr Asp Gly His Thr Val Gly Gly Leu Gln
    210                 215                 220
Tyr Gly Gly Leu Gly Gln Leu Ala Asp Gly Val Val Gly Leu Asp Asp
225                 230                 235                 240
Phe Arg Lys Ser Gln Glu Leu Arg Val Trp Pro Gly Tyr Asp Tyr Val
                245                 250                 255
Gly Trp Ser Asn His Ser Phe Ser Ser Gly Tyr Val Glu Met Glu Phe
            260                 265                 270
Glu Phe Asp Arg Leu Arg Ala Phe Gln Ala Met Gln Val His Cys Asn
        275                 280                 285
Asn Met His Thr Leu Gly Ala Arg Leu Pro Gly Gly Val Glu Cys Arg
    290                 295                 300
Phe Arg Arg Gly Pro Ala Met Ala Trp Glu Gly Glu Pro Met Arg His
305                 310                 315                 320
Asn Leu Gly Gly Asn Leu Gly Asp Pro Arg Ala Arg Ala Val Ser Val
                325                 330                 335
Pro Leu Gly Gly Arg Val Ala Arg Phe Leu Gln Cys Arg Phe Leu Phe
            340                 345                 350
Ala Gly Pro Trp Leu Leu Phe Ser Glu Ile Ser Phe Ile Ser Asp Val
        355                 360                 365
Val Asn Asn Ser Ser Pro Ala Leu Gly Gly Thr Phe Pro Pro Ala Pro
    370                 375                 380
Trp Trp Pro Pro Gly Pro Pro Pro Thr Asn Phe Ser Ser Leu Glu Leu
385                 390                 395                 400
Glu Pro Arg Gly Gln Gln Pro Val Ala Lys Ala Glu Gly Ser Pro Thr
                405                 410                 415
Ala Ile Leu Ile Gly Cys Leu Val Ala Ile Ile Leu Leu Leu Leu Leu
            420                 425                 430
Ile Ile Ala Leu Met Leu Trp Arg Leu His Trp Arg Arg Leu Leu Ser
        435                 440                 445
Lys Ala Glu Arg Arg Val Leu Glu Glu Glu Leu Thr Val His Leu Ser
    450                 455                 460
Val Pro Gly Asp Thr Ile Leu Ile Asn Asn Arg Pro Gly Pro Arg Glu
465                 470                 475                 480
Pro Pro Pro Tyr Gln Glu Pro Arg Pro Arg Gly Asn Pro Pro His Ser
                485                 490                 495
Ala Pro Cys Val Pro Asn Gly Ser Gly Ala Pro Val
            500                 505

<210> 8
<211> 1524
<212> DNA
<213> Human

<400> 8

atgggaccag aggccctgtc atctttactg ctgctgctct tggtggcaag tggagatgct     60
gacatgaagg gacattttga tcctgccaag tgccgctatg ccctgggcat gcaggaccgg    120
accatcccag acagtgacat ctctgcttcc agctcctggt cagattccac tgccgcccgc    180
cacagcaggt tggagagcag tgacggggat ggggcctggt gccccgcagg gtcggtgttt    240
cccaaggagg aggagtactt gcaggtggat ctacaacgac tgcacctggt ggctctggtg    300

ggcacccagg gacggcatgc cgggggcctg ggcaaggagt tctcccggag ctaccggctg 360
cgttactccc gggatggtcg ccgctggatg ggctggaagg accgctgggg tcaggaggtg 420
atctcaggca atgaggaccc tgagggagtg gtgctgaagg accttgggcc ccccatggtt 480
gcccgactgg ttcgcttcta cccccgggct gaccgggtca tgagcgtctg tctgcgggta 540
gagctctatg gctgcctctg gagggatgga ctcctgtctt acactgcccc tgtggggcag 600
acaatgtatt tatctgaggc cgtgtacctc aacgactcca cctatgacgg acataccgtg 660
ggcggactgc agtatggggg tctgggccag ctggcagatg gtgtggtggg gctggatgac 720
tttaggaaga gtcaggagct gcgggtctgg ccaggctatg actatgtggg atggagcaac 780
cacagcttct ccagtggcta tgtggagatg gagtttgagt ttgaccggct gagggccttc 840
caggctatgc aggtccactg taacaacatg cacacgctgg gagcccgtct gcctggcggg 900
gtggaatgtc gcttccggcg tggccctgcc atggcctggg agggggagcc catgcgccac 960
aacctagggg gcaacctggg ggaccccaga gcccgggctg tctcagtgcc ccttggcggc 1020
cgtgtggctc gctttctgca gtgccgcttc ctctttgcgg ggccctggtt actcttcagc 1080
gaaatctcct tcatctctga tgtggtgaac aattcctctc cggcactggg aggcaccttc 1140
ccgccagccc cctggtggcc gcctggccca cctcccacca acttcagcag cttggagctg 1200
gagcccagag gccagcagcc cgtggccaag gccgagggga gcccgaccgc catcctcatc 1260
ggctgcctgg tggccatcat cctgctcctg ctgctcatca ttgccctcat gctctggcgg 1320
ctgcactggc gcaggctcct cagcaaggct gaacggaggg tgttggaaga ggagctgacg 1380
gttcacctct ctgtccctgg ggacactatc ctcatcaaca accgcccagg tcctagagag 1440
ccacccccgt accaggagcc ccggcctcgt gggaatccgc cccactccgc tccctgtgtc 1500
cccaatggct ctggtgcacc tgtg 1524

<210> 9
<211> 767
<212> PRT
<213> Human

<400> 9
   Met Gly Pro Glu Ala Leu Ser Ser Leu Leu Leu Leu Leu Leu Val Ala
   5 10 15
   Ser Gly Asp Ala Asp Met Lys Gly His Phe Asp Pro Ala Lys Cys Arg
   20 25 30
   Tyr Ala Leu Gly Met Gln Asp Arg Thr Ile Pro Asp Ser Asp Ile Ser
   35 40 45
   Ala Ser Ser Ser Trp Ser Asp Ser Thr Ala Ala Arg His Ser Arg Leu
   50 55 60
   Glu Ser Ser Asp Gly Asp Gly Ala Trp Cys Pro Ala Gly Ser Val Phe
   65 70 75 80
   Pro Lys Glu Glu Glu Tyr Leu Gln Val Asp Leu Gln Arg Leu His Leu
   85 90 95
   Val Ala Leu Val Gly Thr Gln Gly Arg His Ala Gly Gly Leu Gly Lys
   100 105 110
   Glu Phe Ser Arg Ser Tyr Arg Leu Arg Tyr Ser Arg Asp Gly Arg Arg
   115 120 125
   Trp Met Gly Trp Lys Asp Arg Trp Gly Gln Glu Val Ile Ser Gly Asn
   130 135 140
   Glu Asp Pro Glu Gly Val Val Leu Lys Asp Leu Gly Pro Pro Met Val
   145 150 155 160
   Ala Arg Leu Val Arg Phe Tyr Pro Arg Ala Asp Arg Val Met Ser Val
   165 170 175
   Cys Leu Arg Val Glu Leu Tyr Gly Cys Leu Trp Arg Asp Gly Leu Leu
   180 185 190
   Ser Tyr Thr Ala Pro Val Gly Gln Thr Met Tyr Leu Ser Glu Ala Val
   195 200 205
   Tyr Leu Asn Asp Ser Thr Tyr Asp Gly His Thr Val Gly Gly Leu Gln
   210 215 220
   Tyr Gly Gly Leu Gly Gln Leu Ala Asp Gly Val Val Gly Leu Asp Asp
   225 230 235 240
   Phe Arg Lys Ser Gln Glu Leu Arg Val Trp Pro Gly Tyr Asp Tyr Val
   245 250 255
   Gly Trp Ser Asn His Ser Phe Ser Ser Gly Tyr Val Glu Met Glu Phe
   260 265 270
   Glu Phe Asp Arg Leu Arg Ala Phe Gln Ala Met Gln Val His Cys Asn
   275 280 285
   Asn Met His Thr Leu Gly Ala Arg Leu Pro Gly Gly Val Glu Cys Arg
   290 295 300
   Phe Arg Arg Gly Pro Ala Met Ala Trp Glu Gly Glu Pro Met Arg His
   305 310 315 320
   Asn Leu Gly Gly Asn Leu Gly Asp Pro Arg Ala Arg Ala Val Ser Val
   325 330 335
   Pro Leu Gly Gly Arg Val Ala Arg Phe Leu Gln Cys Arg Phe Leu Phe
   340 345 350
   Ala Gly Pro Trp Leu Leu Phe Ser Glu Ile Ser Phe Ile Ser Asp Val
   355 360 365
   Val Asn Asn Ser Ser Pro Ala Leu Gly Gly Thr Phe Pro Pro Ala Pro
   370 375 380
   Trp Trp Pro Pro Gly Pro Pro Pro Thr Asn Phe Ser Ser Leu Glu Leu
   385 390 395 400
   Glu Pro Arg Gly Gln Gln Pro Val Ala Lys Ala Glu Gly Ser Pro Thr
   405 410 415
   Ala Ile Leu Ile Gly Cys Leu Val Ala Ile Ile Leu Leu Leu Leu Leu
   420 425 430
   Ile Ile Ala Leu Met Leu Trp Arg Leu His Trp Arg Arg Leu Leu Ser
   435 440 445
   Lys Val Leu Glu Ser His Pro Arg Thr Arg Ser Pro Gly Leu Val Gly
   450 455 460
   Ile Arg Pro Thr Pro Leu Pro Val Ser Pro Met Ala Leu Val His Leu
   465 470 475 480
   Cys Glu Val Asp Ser Pro Gln Asp Leu Val Ser Leu Asp Phe Pro Leu
   485 490 495
   Asn Val Arg Lys Gly His Pro Leu Leu Val Ala Val Lys Ile Leu Arg
   500 505 510
   Pro Asp Ala Thr Lys Asn Ala Arg Asn Asp Phe Leu Lys Glu Val Lys
   515 520 525
   Ile Met Ser Arg Leu Lys Asp Pro Asn Ile Ile Arg Leu Leu Gly Val
   530 535 540
   Cys Val Gln Asp Asp Pro Leu Cys Met Ile Thr Asp Tyr Met Glu Asn
   545 550 555 560
   Gly Asp Leu Asn Gln Phe Leu Ser Ala His Gln Leu Glu Asp Lys Ala
   565 570 575
   Ala Glu Gly Ala Pro Gly Asp Gly Gln Ala Ala Gln Gly Pro Thr Ile
   580 585 590
   Ser Tyr Pro Met Leu Leu His Val Ala Ala Gln Ile Ala Ser Gly Met
   595 600 605
   Arg Tyr Leu Ala Thr Leu Asn Phe Val His Arg Asp Leu Ala Thr Arg
   610 615 620
   Asn Cys Leu Val Gly Glu Asn Phe Thr Ile Lys Ile Ala Asp Phe Gly
   625 630 635 640
   Met Ser Arg Asn Leu Tyr Ala Gly Asp Tyr Tyr Arg Val Gln Gly Arg
   645 650 655
   Ala Val Leu Pro Ile Arg Trp Met Ala Trp Glu Cys Ile Leu Met Gly
   660 665 670
   Lys Phe Thr Thr Ala Ser Asp Val Trp Ala Phe Gly Val Thr Leu Trp
   675 680 685
   Glu Val Leu Met Leu Cys Arg Ala Gln Pro Phe Gly Gln Leu Thr Asp
   690 695 700
   Glu Gln Val Ile Glu Asn Ala Gly Glu Phe Phe Arg Asp Gln Gly Arg
   705 710 715 720
   Gln Val Tyr Leu Ser Arg Pro Pro Ala Cys Pro Gln Gly Leu Tyr Glu
   725 730 735
   Leu Met Leu Arg Cys Trp Ser Arg Glu Ser Glu Gln Arg Pro Pro Phe
   740 745 750
   Ser Gln Leu His Arg Phe Leu Ala Glu Asp Ala Leu Asn Thr Val
   755 760 765

<210> 10
<211> 2301
<212> DNA
<213> Human

<400> 10

atgggaccag aggccctgtc atctttactg ctgctgctct tggtggcaag tggagatgct 60
gacatgaagg gacattttga tcctgccaag tgccgctatg ccctgggcat gcaggaccgg 120
accatcccag acagtgacat ctctgcttcc agctcctggt cagattccac tgccgcccgc 180
cacagcaggt tggagagcag tgacggggat ggggcctggt gccccgcagg gtcggtgttt 240
cccaaggagg aggagtactt gcaggtggat ctacaacgac tgcacctggt ggctctggtg 300
ggcacccagg gacggcatgc cgggggcctg ggcaaggagt tctcccggag ctaccggctg 360
cgttactccc gggatggtcg ccgctggatg ggctggaagg accgctgggg tcaggaggtg 420
atctcaggca atgaggaccc tgagggagtg gtgctgaagg accttgggcc ccccatggtt 480
gcccgactgg ttcgcttcta cccccgggct gaccgggtca tgagcgtctg tctgcgggta 540
gagctctatg gctgcctctg gagggatgga ctcctgtctt acaccgcccc tgtggggcag 600
acaatgtatt tatctgaggc cgtgtacctc aacgactcca cctatgacgg acataccgtg 660
ggcggactgc agtatggggg tctgggccag ctggcagatg gtgtggtggg gctggatgac 720
tttaggaaga gtcaggagct gcgggtctgg ccaggctatg actatgtggg atggagcaac 780
cacagcttct ccagtggcta tgtggagatg gagtttgagt ttgaccggct gagggccttc 840
caggctatgc aggtccactg taacaacatg cacacgctgg gagcccgtct gcctggcggg 900
gtggaatgtc gcttccggcg tggccctgcc atggcctggg agggggagcc catgcgccac 960
aacctagggg gcaacctggg ggaccccaga gcccgggctg tctcagtgcc ccttggcggc 1020
cgtgtggctc gctttctgca gtgccgcttc ctctttgcgg ggccctggtt actcttcagc 1080
gaaatctcct tcatctctga tgtggtgaac aattcctctc cggcactggg aggcaccttc 1140
ccgccagccc cctggtggcc gcctggccca cctcccacca acttcagcag cttggagctg 1200
gagcccagag gccagcagcc cgtggccaag gccgagggga gcccgaccgc catcctcatc 1260
ggctgcctgg tggccatcat cctgctcctg ctgctcatca ttgccctcat gctctggcgg 1320
ctgcactggc gcaggctcct cagcaaggtc ctagagagcc acccccgtac caggagcccc 1380
ggcctcgtgg gaatccgccc cactccgctc cctgtgtccc caatggctct ggtgcacctg 1440
tgtgaggtcg acagccctca agatctggtt agtcttgatt tcccccttaa tgtgcgtaag 1500
ggacaccctt tgctggtagc tgtcaagatc ttacggccag atgccaccaa gaatgccagg 1560
aatgatttcc tgaaagaggt gaagatcatg tcgaggctca aggacccaaa catcattcgg 1620
ctgctgggcg tgtgtgtgca ggacgacccc ctctgcatga ttactgacta catggagaac 1680
ggcgacctca accagttcct cagtgcccac cagctggagg acaaggcagc cgagggggcc 1740
cctggggacg ggcaggctgc gcaggggccc accatcagct acccaatgct gctgcatgtg 1800
gcagcccaga tcgcctccgg catgcgctat ctggccacac tcaactttgt acatcgggac 1860
ctggccacgc ggaactgcct agttggggaa aatttcacca tcaaaatcgc agactttggc 1920
atgagccgga acctctatgc tggggactat taccgtgtgc agggccgggc agtgctgccc 1980
atccgctgga tggcctggga gtgcatcctc atggggaagt tcacgactgc gagtgacgtg 2040
tgggcctttg gtgtgaccct gtgggaggtg ctgatgctct gtagggccca gccctttggg 2100
cagctcaccg acgagcaggt catcgagaac gcgggggagt tcttccggga ccagggccgg 2160
caggtgtacc tgtcccggcc gcctgcctgc ccgcagggcc tatatgagct gatgcttcgg 2220
tgctggagcc gggagtctga gcagcgacca cccttttccc agctgcatcg gttcctggca 2280
gaggatgcac tcaacacggt g 2301


<210> 11
<211> 29
<212> DNA
<213> Artificial Sequence

<220>
<223> nucleotide sequence of primer 1

<400> 11
aattggatcc atgggaccag aggccctgt 29


<210> 12

<211> 30
<212> DNA
<213> Artificial Sequence

<220>
<223> nucleotide sequence of primer 2

<400> 12

aatgaattct cacaccgtgt tgagtgcatc                                        30


<210> 13
<211> 32
<212> DNA
<213> Artificial Sequence

<220>
<223> nucleotide sequence of primer 3

<400> 13

ccgctcgagc cttcctggca gaggatgcac tc                                     32


<210> 14
<211> 28
<212> DNA
<213> Artificial Sequence

<220>
<223> nucleotide sequence of primer 4

<400> 14

tggctggcaa ctagaaggca cagtcgag                                          28


<210> 15
<211> 536
<212> PRT
<213> Human

<400> 15
   Met Gly Pro Glu Ala Leu Ser Ser Leu Leu Leu Leu Leu Leu Val Ala
                   5                   10                  15
   Ser Gly Asp Ala Asp Met Lys Gly His Phe Asp Pro Ala Lys Cys Arg
               20                  25                  30
   Tyr Ala Leu Gly Met Gln Asp Arg Thr Ile Pro Asp Ser Asp Ile Ser
           35                  40                  45
   Ala Ser Ser Ser Trp Ser Asp Ser Thr Ala Ala Arg His Ser Arg Leu
       50                  55                  60
   Glu Ser Ser Asp Gly Asp Gly Ala Trp Cys Pro Ala Gly Ser Val Phe
   65                  70                  75                  80
   Pro Lys Glu Glu Glu Tyr Leu Gln Val Asp Leu Gln Arg Leu His Leu
                   85                  90                  95
   Val Ala Leu Val Gly Thr Gln Gly Arg His Ala Gly Gly Leu Gly Lys
               100                 105                 110
   Glu Phe Ser Arg Ser Tyr Arg Leu Arg Tyr Ser Arg Asp Gly Arg Arg
           115                 120                 125
   Trp Met Gly Trp Lys Asp Arg Trp Gly Gln Glu Val Ile Ser Gly Asn
       130                 135                 140
   Glu Asp Pro Glu Gly Val Val Leu Lys Asp Leu Gly Pro Pro Met Val
   145                 150                 155                 160
   Ala Arg Leu Val Arg Phe Tyr Pro Arg Ala Asp Arg Val Met Ser Val
                   165                 170                 175
   Cys Leu Arg Val Glu Leu Tyr Gly Cys Leu Trp Arg Asp Gly Leu Leu
               180                 185                 190
   Ser Tyr Thr Ala Pro Val Gly Gln Thr Met Tyr Leu Ser Glu Ala Val
           195                 200                 205
   Tyr Leu Asn Asp Ser Thr Tyr Asp Gly His Thr Val Gly Gly Leu Gln
       210                 215                 220
   Tyr Gly Gly Leu Gly Gln Leu Ala Asp Gly Val Val Gly Leu Asp Asp
   225                 230                 235                 240
   Phe Arg Lys Ser Gln Glu Leu Arg Val Trp Pro Gly Tyr Asp Tyr Val
                   245                 250                 255
   Gly Trp Ser Asn His Ser Phe Ser Ser Gly Tyr Val Glu Met Glu Phe
               260                 265                 270
   Glu Phe Asp Arg Leu Arg Ala Phe Gln Ala Met Gln Val His Cys Asn
           275                 280                 285
   Asn Met His Thr Leu Gly Ala Arg Leu Pro Gly Gly Val Glu Cys Arg
       290                 295                 300
   Phe Arg Arg Gly Pro Ala Met Ala Trp Glu Gly Glu Pro Met Arg His
   305                 310                 315                 320
   Asn Leu Gly Gly Asn Leu Gly Asp Pro Arg Ala Arg Ala Val Ser Val
                   325                 330                 335
   Pro Leu Gly Gly Arg Val Ala Arg Phe Leu Gln Cys Arg Phe Leu Phe
               340                 345                 350
   Ala Gly Pro Trp Leu Leu Phe Ser Glu Ile Ser Phe Ile Ser Asp Val
           355                 360                 365
   Val Asn Asn Ser Ser Pro Ala Leu Gly Gly Thr Phe Pro Pro Ala Pro
       370                 375                 380
   Trp Trp Pro Pro Gly Pro Pro Pro Thr Asn Phe Ser Ser Leu Glu Leu
   385                 390                 395                 400
   Glu Pro Arg Gly Gln Gln Pro Val Ala Lys Ala Glu Gly Ser Pro Thr
                   405                 410                 415
   Ala Ile Leu Ile Gly Cys Leu Val Ala Ile Ile Leu Leu Leu Leu Leu
               420                 425                 430
   Ile Ile Ala Leu Met Leu Trp Arg Leu His Trp Arg Arg Leu Leu Ser
           435                 440                 445
   Lys Ala Glu Arg Arg Val Leu Glu Glu Glu Leu Thr Val His Leu Ser
       450                 455                 460
   Val Pro Gly Asp Thr Ile Leu Ile Asn Asn Arg Pro Gly Pro Arg Glu
   465                 470                 475                 480
   Pro Pro Pro Tyr Gln Glu Pro Arg Pro Arg Gly Asn Pro Pro His Ser
                   485                 490                 495
   Ala Pro Cys Val Pro Asn Gly Ser Ala Leu Leu Leu Ser Asn Pro Ala
               500                 505                 510
   Tyr Arg Leu Leu Leu Ala Thr Tyr Ala Arg Pro Pro Arg Ala Phe Leu
           515                 520                 525
   Ala Glu Asp Ala Leu Asn Thr Val
       530                 535

<210> 16
<211> 1608
<212> DNA
<213> Human

<400> 16

atgggaccag aggccctgtc atctttactg ctgctgctct tggtggcaag tggagatgct      60
gacatgaagg gacattttga tcctgccaag tgccgctatg ccctgggcat gcaggaccgg     120
accatcccag acagtgacat ctctgcttcc agctcctggt cagattccac tgccgcccgc     180
cacagcaggt tggagagcag tgacggggat ggggcctggt gccccgcagg gtcggtgttt     240

cccaaggagg aggagtactt gcaggtggat ctacaacgac tgcacctggt ggctctggtg 300
ggcacccagg gacggcatgc cgggggcctg ggcaaggagt tctcccggag ctaccggctg 360
cgttactccc gggatggtcg ccgctggatg ggctggaagg accgctgggg tcaggaggtg 420
atctcaggca atgaggaccc tgagggagtg gtgctgaagg accttgggcc ccccatggtt 480
gcccgactgg ttcgcttcta cccccgggct gaccgggtca tgagcgtctg tctgcgggta 540
gagctctatg gctgcctctg gagggatgga ctcctgtctt acaccgcccc tgtggggcag 600
acaatgtatt tatctgaggc cgtgtacctc aacgactcca cctatgacgg acataccgtg 660
ggcggactgc agtatggggg tctgggccag ctggcagatg gtgtggtggg gctggatgac 720
tttaggaaga gtcaggagct gcgggtctgg ccaggctatg actatgtggg atggagcaac 780
cacagcttct ccagtggcta tgtggagatg gagtttgagt ttgaccggct gagggccttc 840
caggctatgc aggtccactg taacaacatg cacacgctgg gagcccgtct gcctggcggg 900
gtggaatgtc gcttccggcg tggccctgcc atggcctggg agggggagcc catgcgccac 960
aacctagggg gcaacctggg ggaccccaga gcccgggctg tctcagtgcc ccttggcggc 1020
cgtgtggctc gctttctgca gtgccgcttc ctctttgcgg ggccctggtt actcttcagc 1080
gaaatctcct tcatctctga tgtggtgaac aattcctctc cggcactggg aggcaccttc 1140
ccgccagccc cctggtggcc gcctggccca cctcccacca acttcagcag cttggagctg 1200
gagcccagag gccagcagcc cgtggccaag gccgagggga gcccgaccgc catcctcatc 1260
ggctgcctgg tggccatcat cctgctcctg ctgctcatca ttgccctcat gctctggcgg 1320
ctgcactggc gcaggctcct cagcaaggct gaacggaggg tgttggaaga ggagctgacg 1380
gttcacctct ctgtccctgg ggacactatc ctcatcaaca accgcccagg tcctagagag 1440
ccacccccgt accaggagcc ccggcctcgt gggaatccgc cccactctgc tccctgtgtc 1500
cccaatggct ctgcgttgct gctctccaat ccagcctacc gcctccttct ggccacttac 1560
gcccgtcccc ctcgagcctt cctggcagag gatgcactca acacggtg 1608

<210> 17
<211> 22
<212> DNA
<213> Artificial Sequence

<220>
<223> nucleotide sequence of primer 5

<400> 17

agagtggatt tccctcgatc tc 22

<210> 18
<211> 20
<212> DNA
<213> Artificial Sequence

<220>
<223> nucleotide sequence of primer 6

<400> 18

agggtgtccc ttacgcacat 20

<210> 19
<211> 24
<212> DNA
<213> Artificial Sequence

<220>
<223> nucleotide sequence of TaqMan probe 1

<400> 19

tgcacctgtg tgaggtcgac agcc 24

<210> 20
<211> 16
<212> DNA
<213> Artificial Sequence

<220>
<223> nucleotide sequence of primer 7

<400> 20

ccggcctcgt gggaat                                                        16


<210> 21
<211> 18
<212> DNA
<213> Artificial Sequence

<220>
<223> nucleotide sequence of primer 8

<400> 21

cggtaggctg gattggag                                                      18


<210> 22
<211> 22
<212> DNA
<213> Artificial Sequence

<220>
<223> nucleotide sequence of TaqMan probe 2

<400> 22

cccaatggct ctgcgttgct gc                                                 22


<210> 23
<211> 27
<212> DNA
<213> Artificial Sequence

<220>
<223> nucleotide sequence of primer 9

<400> 23

atgaattccg ggctcccctc ggccttg                                            27


<210> 24
<211> 648
<212> PRT
<213> Human

<400> 24

Met Gly Pro Glu Ala Leu Ser Ser Leu Leu Leu Leu Leu Leu Val Ala
                5                   10                  15
Ser Gly Asp Ala Asp Met Lys Gly His Phe Asp Pro Ala Lys Cys Arg

                20                  25                  30
Tyr Ala Leu Gly Met Gln Asp Arg Thr Ile Pro Asp Ser Asp Ile Ser
        35                  40                  45
Ala Ser Ser Ser Trp Ser Asp Ser Thr Ala Ala Arg His Ser Arg Leu
    50                  55                  60
Glu Ser Ser Asp Gly Asp Gly Ala Trp Cys Pro Ala Gly Ser Val Phe
65                  70                  75                  80
Pro Lys Glu Glu Glu Tyr Leu Gln Val Asp Leu Gln Arg Leu His Leu
                85                  90                  95
Val Ala Leu Val Gly Thr Gln Gly Arg His Ala Gly Gly Leu Gly Lys
            100                 105                 110
Glu Phe Ser Arg Ser Tyr Arg Leu Arg Tyr Ser Arg Asp Gly Arg Arg
        115                 120                 125
Trp Met Gly Trp Lys Asp Arg Trp Gly Gln Glu Val Ile Ser Gly Asn
    130                 135                 140
Glu Asp Pro Glu Gly Val Val Leu Lys Asp Leu Gly Pro Pro Met Val
145                 150                 155                 160
Ala Arg Leu Val Arg Phe Tyr Pro Arg Ala Asp Arg Val Met Ser Val
                165                 170                 175
Cys Leu Arg Val Glu Leu Tyr Gly Cys Leu Trp Arg Asp Gly Leu Leu
            180                 185                 190
Ser Tyr Thr Ala Pro Val Gly Gln Thr Met Tyr Leu Ser Glu Ala Val
        195                 200                 205
Tyr Leu Asn Asp Ser Thr Tyr Asp Gly His Thr Val Gly Gly Leu Gln
    210                 215                 220
Tyr Gly Gly Leu Gly Gln Leu Ala Asp Gly Val Val Gly Leu Asp Asp
225                 230                 235                 240
Phe Arg Lys Ser Gln Glu Leu Arg Val Trp Pro Gly Tyr Asp Tyr Val
                245                 250                 255
Gly Trp Ser Asn His Ser Phe Ser Ser Gly Tyr Val Glu Met Glu Phe
            260                 265                 270
Glu Phe Asp Arg Leu Arg Ala Phe Gln Ala Met Gln Val His Cys Asn
        275                 280                 285
Asn Met His Thr Leu Gly Ala Arg Leu Pro Gly Gly Val Glu Cys Arg
    290                 295                 300
Phe Arg Arg Gly Pro Ala Met Ala Trp Glu Gly Glu Pro Met Arg His
305                 310                 315                 320
Asn Leu Gly Gly Asn Leu Gly Asp Pro Arg Ala Arg Ala Val Ser Val
                325                 330                 335
Pro Leu Gly Gly Arg Val Ala Arg Phe Leu Gln Cys Arg Phe Leu Phe
            340                 345                 350
Ala Gly Pro Trp Leu Leu Phe Ser Glu Ile Ser Phe Ile Ser Asp Val
        355                 360                 365
Val Asn Asn Ser Ser Pro Ala Leu Gly Gly Thr Phe Pro Pro Ala Pro
    370                 375                 380
Trp Trp Pro Pro Gly Pro Pro Pro Thr Asn Phe Ser Ser Leu Glu Leu
385                 390                 395                 400
Glu Pro Arg Gly Gln Gln Pro Val Ala Lys Ala Glu Gly Ser Pro Glu
                405                 410                 415
Phe Pro Lys Ser Cys Asp Lys Thr His Thr Cys Pro Pro Cys Pro Ala
            420                 425                 430
Pro Glu Leu Leu Gly Gly Pro Ser Val Phe Leu Phe Pro Pro Lys Pro
        435                 440                 445
Lys Asp Thr Leu Met Ile Ser Arg Thr Pro Glu Val Thr Cys Val Val
    450                 455                 460
Val Asp Val Ser His Glu Asp Pro Glu Val Lys Phe Asn Trp Tyr Val
465                 470                 475                 480
Asp Gly Val Glu Val His Asn Ala Lys Thr Lys Pro Arg Glu Glu Gln
                485                 490                 495
Tyr Asn Ser Thr Tyr Arg Val Val Ser Val Leu Thr Val Leu His Gln
            500                 505                 510
Asp Trp Leu Asn Gly Lys Glu Tyr Lys Cys Lys Val Ser Asn Lys Ala
        515                 520                 525

Leu Pro Ala Pro Ile Glu Lys Thr Ile Ser Lys Ala Lys Gly Gln Pro
    530                 535                 540
Arg Glu Pro Gln Val Tyr Thr Leu Pro Pro Ser Arg Asp Glu Leu Thr
545                 550                 555                 560
Lys Asn Gln Val Ser Leu Thr Cys Leu Val Lys Gly Phe Tyr Pro Ser
                565                 570                 575
Asp Ile Ala Val Glu Trp Glu Ser Asn Gly Gln Pro Glu Asn Asn Tyr
            580                 585                 590
Lys Thr Thr Pro Pro Val Leu Asp Ser Asp Gly Ser Phe Phe Leu Tyr
        595                 600                 605
Ser Lys Leu Thr Val Asp Lys Ser Arg Trp Gln Gln Gly Asn Val Phe
    610                 615                 620
Ser Cys Ser Val Met His Glu Ala Leu His Asn His Tyr Thr Gln Lys
625                 630                 635                 640
Ser Leu Ser Leu Ser Pro Gly Lys
                645

<210> 25
<211> 1944
<212> DNA
<213> Human

<400> 25

atgggaccag aggccctgtc atctttactg ctgctgctct tggtggcaag tggagatgct      60
gacatgaagg gacattttga tcctgccaag tgccgctatg ccctgggcat gcaggaccgg     120
accatcccag acagtgacat ctctgcttcc agctcctggt cagattccac tgccgcccgc     180
cacagcaggt tggagagcag tgacggggat ggggcctggt gccccgcagg gtcggtgttt     240
cccaaggagg aggagtactt gcaggtggat ctacaacgac tgcacctggt ggctctggtg     300
ggcacccagg gacggcatgc cgggggcctg ggcaaggagt tctcccggag ctaccggctg     360
cgttactccc gggatggtcg ccgctggatg ggctggaagg accgctgggg tcaggaggtg     420
atctcaggca atgaggaccc tgagggagtg gtgctgaagg accttgggcc ccccatggtt     480
gcccgactgg ttcgcttcta cccccgggct gaccgggtca tgagcgtctg tctgcgggta     540
gagctctatg gctgcctctg gagggatgga ctcctgtctt acaccgcccc tgtggggcag     600
acaatgtatt tatctgaggc cgtgtacctc aacgactcca cctatgacgg acataccgtg     660
ggcggactgc agtatggggg tctgggccag ctggcagatg gtgtggtggg gctggatgac     720
tttaggaaga gtcaggagct gcgggtctgg ccaggctatg actatgtggg atggagcaac     780
cacagcttct ccagtggcta tgtggagatg gagtttgagt ttgaccggct gagggccttc     840
caggctatgc aggtccactg taacaacatg cacacgctgg gagcccgtct gcctggcggg     900
gtggaatgtc gcttccggcg tggccctgcc atggcctggg agggggagcc catgcgccac     960
aacctagggg gcaacctggg ggaccccaga gcccgggctg tctcagtgcc ccttggcggc    1020
cgtgtggctc gctttctgca gtgccgcttc ctctttgcgg ggccctggtt actcttcagc    1080
gaaatctcct tcatctctga tgtggtgaac aattcctctc cggcactggg aggcaccttc    1140
ccgccagccc cctggtggcc gcctggccca cctcccacca acttcagcag cttggagctg    1200
gagcccagag gccagcagcc cgtggccaag gccgagggga gcccggaatt ccccaaatct    1260
tgtgacaaaa ctcacacatg cccaccgtgc ccagcacctg aactcctggg gggaccgtca    1320
gtcttcctct tccccccaaa acccaaggac accctcatga tctcccggac ccctgaggtc    1380
acatgcgtgg tggtggacgt gagccacgaa gaccctgagg tcaagttcaa ctggtacgtg    1440
gacggcgtgg aggtgcataa tgccaagaca aagccgcggg aggagcagta caacagcacg    1500
taccgtgtgg tcagcgtcct caccgtcctg caccaggact ggctgaatgg caaggagtac    1560
aagtgcaagg tctccaacaa agccctccca gcccccatcg agaaaaccat ctccaaagcc    1620
aaagggcagc cccgagaacc acaggtgtac accctgcccc catcccggga tgagctgacc    1680
aagaaccagg tcagcctgac ctgcctggtc aaaggcttct atcccagcga catcgccgtg    1740
gagtgggaga gcaatgggca gccggagaac aactacaaga ccacgcctcc cgtgctggac    1800
tccgacggct ccttcttcct ctacagcaag ctcaccgtgg acaagagcag gtggcagcag    1860
gggaacgtct tctcatgctc cgtgatgcat gaggctctgc acaaccacta cacgcagaag    1920
agcctctccc tgtctccggg taaa                                           1944

<210> 26
<211> 436
<212> PRT

<213> Human

<400> 26

Met Gly Pro Glu Ala Leu Ser Ser Leu Leu Leu Leu Leu Leu Val Ala
                5                   10                  15
Ser Gly Asp Ala Asp Met Lys Gly His Phe Asp Pro Ala Lys Cys Arg
            20                  25                  30
Tyr Ala Leu Gly Met Gln Asp Arg Thr Ile Pro Asp Ser Asp Ile Ser
        35                  40                  45
Ala Ser Ser Ser Trp Ser Asp Ser Thr Ala Ala Arg His Ser Arg Leu
    50                  55                  60
Glu Ser Ser Asp Gly Asp Gly Ala Trp Cys Pro Ala Gly Ser Val Phe
65                  70                  75                  80
Pro Lys Glu Glu Glu Tyr Leu Gln Val Asp Leu Gln Arg Leu His Leu
                85                  90                  95
Val Ala Leu Val Gly Thr Gln Gly Arg His Ala Gly Gly Leu Gly Lys
            100                 105                 110
Glu Phe Ser Arg Ser Tyr Arg Leu Arg Tyr Ser Arg Asp Gly Arg Arg
        115                 120                 125
Trp Met Gly Trp Lys Asp Arg Trp Gly Gln Glu Val Ile Ser Gly Asn
    130                 135                 140
Glu Asp Pro Glu Gly Val Val Leu Lys Asp Leu Gly Pro Pro Met Val
145                 150                 155                 160
Ala Arg Leu Val Arg Phe Tyr Pro Arg Ala Asp Arg Val Met Ser Val
                165                 170                 175
Cys Leu Arg Val Glu Leu Tyr Gly Cys Leu Trp Arg Asp Gly Leu Leu
            180                 185                 190
Ser Tyr Thr Ala Pro Val Gly Gln Thr Met Tyr Leu Ser Glu Ala Val
        195                 200                 205
Tyr Leu Asn Asp Ser Thr Tyr Asp Gly His Thr Val Gly Gly Leu Gln
    210                 215                 220
Tyr Gly Gly Leu Gly Gln Leu Ala Asp Gly Val Val Gly Leu Asp Asp
225                 230                 235                 240
Phe Arg Lys Ser Gln Glu Leu Arg Val Trp Pro Gly Tyr Asp Tyr Val
                245                 250                 255
Gly Trp Ser Asn His Ser Phe Ser Ser Gly Tyr Val Glu Met Glu Phe
            260                 265                 270
Glu Phe Asp Arg Leu Arg Ala Phe Gln Ala Met Gln Val His Cys Asn
        275                 280                 285
Asn Met His Thr Leu Gly Ala Arg Leu Pro Gly Gly Val Glu Cys Arg
    290                 295                 300
Phe Arg Arg Gly Pro Ala Met Ala Trp Glu Gly Glu Pro Met Arg His
305                 310                 315                 320
Asn Leu Gly Gly Asn Leu Gly Asp Pro Arg Ala Arg Ala Val Ser Val
                325                 330                 335
Pro Leu Gly Gly Arg Val Ala Arg Phe Leu Gln Cys Arg Phe Leu Phe
            340                 345                 350
Ala Gly Pro Trp Leu Leu Phe Ser Glu Ile Ser Phe Ile Ser Asp Val
        355                 360                 365
Val Asn Asn Ser Ser Pro Ala Leu Gly Gly Thr Phe Pro Pro Ala Pro
    370                 375                 380
Trp Trp Pro Pro Gly Pro Pro Pro Thr Asn Phe Ser Ser Leu Glu Leu
385                 390                 395                 400
Glu Pro Arg Gly Gln Gln Pro Val Ala Lys Ala Glu Gly Ser Pro Glu
                405                 410                 415
Phe Asp Ile Lys Leu Ile Asp Thr Val Asp Leu Glu Asp Tyr Lys Asp
            420                 425                 430
Asp Asp Asp Lys
        435

<210> 27

<211> 1308
<212> DNA
<213> Human

<400> 27

atgggaccag aggccctgtc atctttactg ctgctgctct tggtggcaag tggagatgct     60
gacatgaagg gacattttga tcctgccaag tgccgctatg ccctgggcat gcaggaccgg    120
accatcccag acagtgacat ctctgcttcc agctcctggt cagattccac tgccgcccgc    180
cacagcaggt tggagagcag tgacggggat ggggcctggt gccccgcagg gtcggtgttt    240
cccaaggagg aggagtactt gcaggtggat ctacaacgac tgcacctggt ggctctggtg    300
ggcacccagg gacggcatgc cgggggcctg ggcaaggagt tctcccggag ctaccggctg    360
cgttactccc gggatggtcg ccgctggatg ggctggaagg accgctgggg tcaggaggtg    420
atctcaggca atgaggaccc tgagggagtg gtgctgaagg accttgggcc ccccatggtt    480
gcccgactgg ttcgcttcta cccccgggct gaccgggtca tgagcgtctg tctgcgggta    540
gagctctatg gctgcctctg gagggatgga ctcctgtctt acaccgcccc tgtggggcag    600
acaatgtatt tatctgaggc cgtgtacctc aacgactcca cctatgacgg acataccgtg    660
ggcggactgc agtatggggg tctgggccag ctggcagatg gtgtggtggg gctggatgac    720
tttaggaaga gtcaggagct gcgggtctgg ccaggctatg actatgtggg atggagcaac    780
cacagcttct ccagtggcta tgtggagatg gagtttgagt ttgaccggct gagggccttc    840
caggctatgc aggtccactg taacaacatg cacacgctgg gagcccgtct gcctggcggg    900
gtggaatgtc gcttccggcg tggccctgcc atggcctggg agggggagcc catgcgccac    960
aacctagggg gcaacctggg ggaccccaga gcccgggctg tctcagtgcc ccttggcggc   1020
cgtgtggctc gctttctgca gtgccgcttc ctctttgcgg ggccctggtt actcttcagc   1080
gaaatctcct tcatctctga tgtggtgaac aattcctctc cggcactggg aggcaccttc   1140
ccgccagccc cctggtggcc gcctggccca cctcccacca acttcagcag cttggagctg   1200
gagcccagag gccagcagcc cgtggccaag gccgagggga gcccggaatt cgatatcaag   1260
cttatcgata ccgtcgacct cgaggattac aaggatgacg acgataag                1308


<210> 28
<211> 30
<212> DNA
<213> Artificial Sequence

<220>
<223> nucleotide sequence of primer 10

<400> 28

aattgaattc cccaaatctt gtgacaaaac                                      30


<210> 29
<211> 29
<212> DNA
<213> Artificial Sequence

<220>
<223> nucleotide sequence of primer 11

<400> 29
aattctcgag tcatttaccc ggagacagg                                       29